# EUROPEAN PATENT SPECIFICATION

(11) **EP 2 004 604 B1**
(45) Date of publication and mention of the grant of the patent: **01.12.2010**
(21) Application number: 07724056.2
(22) Date of filing: 05.04.2007
(51) Int. Cl.: C07D 211/22, C07D 211/26, C07D 401/12, A61K 31/451, A61K 31/4523, A61P 3/00, C07D 401/10

(54) **SUBSTITUTED PHENYLPIPERIDINE DERIVATIVES AS MELANOCORTIN-4 RECEPTOR MODULATORS**
SUBSTITUIERTE PHENYLPIPERIDINDERIVATE ALS MELANOCORTIN-4-REZEPTORMODULATOREN
DÉRIVÉS DE PHÉNYLPIPÉRIDINE SUBSTITUÉS EN TANT QUE MODULATEURS DU RÉCEPTEUR DE LA MÉLANOCORTINE-4

(30) Priority: 07.04.2006 EP 06007416; 07.04.2006 US 790493 P
(43) Date of publication of application: 24.12.2008
(73) Proprietor: Santhera Pharmaceuticals (Schweiz) AG, 4410 Liestal (CH)
(72) Inventor: SOEBERDT, Michael, 79618 Rheinfelden (DE); DEPPE, Holger, CH-4052 Basel (CH); WEYERMANN, Philipp, CH-4450 Sissach (CH); BULAT, Stephan, 79541 Lörrach (DE); VON SPRECHER, Andreas, 4104 Oberwil (CH); FEURER, Achim, 79424 Auggen (DE); LESCOP, Cyrille, 68680 Kembs (FR); HENNEBÖHLE, Marco, 79618 Rheinfelden (DE); NORDHOFF, Sonja, 4144 Arlesheim (CH)
(74) Representative: Grünecker, Kinkeldey, Stockmair & Schwanhäusser Anwaltssozietät
(86) International application number: PCT/EP2007/003115
(87) International publication number: WO 2007/115798

(56) References cited:
- WO-A-2004/024720
- WO-A-2005/047253

## Description

### Field of the Invention

The present invention relates to substituted phenylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where, the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), a-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in human.
The MC-2R is expressed in the adrenal gland representing the ACTH receptor. The MC-2R is not a receptor for α -MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1 R, MC-3,R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4-[4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Modulators of the melanocortin receptor are already known from the literature. WO 2004/024720 A1 describes piperazine urea derivatives which are selective agonists of the human melanocortin-4 receptor and as such they are claimed to be useful in the treatment of prevention of obesity-related disorders.

WO 2005/047253 A1 describes 4,4-disubstituted piperidine derivatives which are postulated to function as melanocortin receptor agonists.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of worsen with an estrogen deficiency or for the treatment of pain.

In view of the unresolved deficiencies in treatment of various diseases and disorders as discussed above, it is an object of the present invention to provide novel substituted phenylpiperidine derivatives with improved ability to cross the blood brain barrier, which are useful as melanocortin-4 receptor modulators to treat-cancer cachexia, muscle wasting, anorexia, anxiety, depression, obesity, diabetes, sexual dysfunction and other diseases with MC-4R involvement.

### Summary of the Invention

The present invention relates to substituted phenylpiperidine derivatives of structural formula (I) 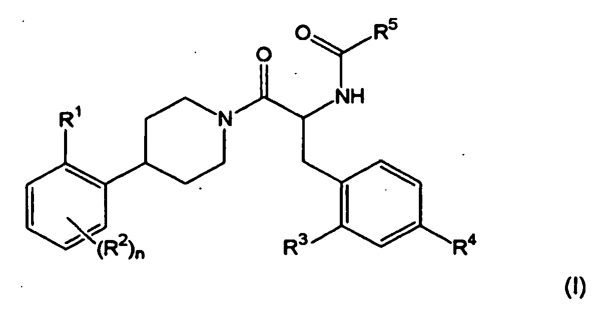 wherein R¹, R², R³, R⁴, R⁵ and n are defined as described below.

The phenylpiperidine derivatives of structural formula (I) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted phenylpiperidine derivatives useful as melanocortin receptor modulators, in particular, selective MC-4R agonists and MC-4R antagonists.

The compounds of the present invention are represented by structural formula (I) 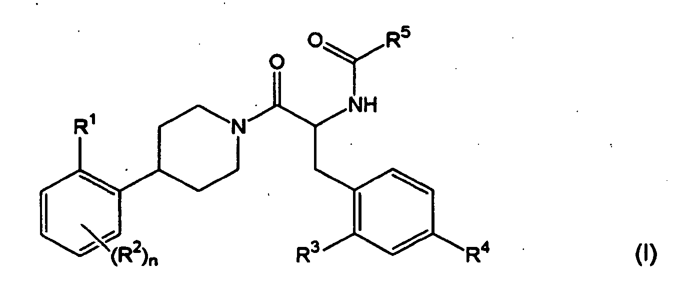 and the enantiomers, diastereomers, tautomers, solvates and pharmaceutically acceptable salts thereof,
wherein
- R¹: is -(C(R⁶)₂)ₗ-T, or
-O-(C(R⁶)₂)ₘ-T;
- R⁶: is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T: is NR⁷R⁸,
morpholine, 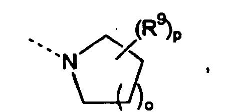 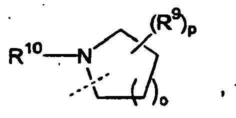 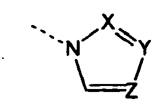 or 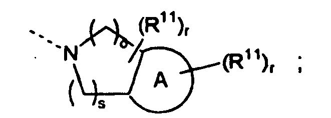
- R⁷ and R⁸: are independently from each other selected from
H,
C₁₋₆-alkyl,
C₂₋₆-alkenyl
C₂₋₆-alkinyl, and
C₁₋₆-alkylene-O-C₁₋₆-alkyl,
wherein each alkyl, alkenyl and alkinyl is optionally substituted by one or more halogen atoms, CN or OH;
- R⁹: is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
- R¹⁰: is H, or
C₁-C₆-alkyl;
- R¹¹: is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆alkylene-O-C₁₋₆-alkyl optionally substituted with 1 to 3 substituents selected from halogen, CN and OH,
-NH₂,
-NH(C₁-₆-alkyl), and
-N(C₁₋₆-alkyl)₂;
- X: is CH or N;
- Y: is CH or N;
- Z: is CH or N;
- A: is a 3-7-membered saturated, unsaturated or aromatic ring containing 0-2 nitrogen atoms;
- R²: is independently selected from
F,
Cl,
CH₃, and
CF₃;
- R³: is H,
Cl,
F, or
CH₃;
- R⁴ is: Cl; or F;
- R⁵ is: 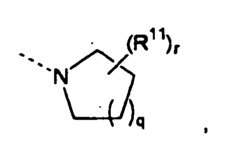 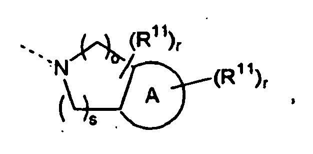 morpholine, optionally substituted by 1 to 3, same or different substituents R¹⁴, or NR¹²R¹³;
- R¹² and R¹³: are independently from each other selected from
C₁₋₆-alkyl,
C₂₋₆-alkenyl,
C₂₋₆-alkinyl, and
C₂₋₆-alkylene-O-C₁₋₆-alkyl, or
C₂₋₆-alkylene-N(C₁₋₆-alkyl)₂;
- R¹⁴: is C₁₋₆-alkyl.
C₁₋₆-alkylene-O-C₁₋₆-alkyl,
C₁₋₆-alkylene-OH, or
C₁₋₆-alkylene-NH₂,
C₁₋₆-alkylene-NH(C₁₋₆alkyl)₂, or
C₁₋₆-alkylene-N(C₁₋₆-alkyl)₂;
- l: is 1, 2, 3, or 4;
- m: is 0, 1, 2, 3, or 4;
- n: is 0, 1, 2, 3, or 4;
- o: is 0, 1, or 2;
- p: is 0, 1, 2, 3, or 4;
- q: is 0, 1, 2, or 3;
- r: is 0, 1, 2, 3, or 4 and
- s: is 1, or 2.

Preferably, the compounds according to formula (I) adopt the structural conformation of the following stereoisomer formula (I'):

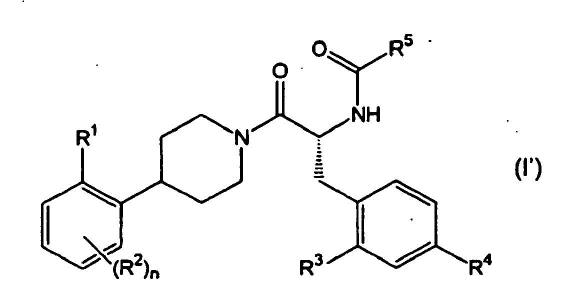

In a preferred embodiment, R² represents Cl or F. Preferably, the phenyl ring directly connected with the piperidine ring is monosubstituted by a chlorine or fluorine atom in the meta or para-position.

It is further preferred that R³ represents H, Cl, or CH₃, more preferably Cl. In an alternative embodiment, R³ preferably represents F.

Preferably, R⁴ represents Cl.

In a preferred embodiment, the variant R¹ represents -(CH₂)ₗ-T or-O-(CH₂)ₘ-T.

In a further preferred embodiment at least one of R⁷ and R⁸ is selected from C₁₋₆-alkyl, C₂₋₆-alkenyl, C₂₋₆-alkinyl and C₂₋₆alkylene-O-C₁₋₆-alkyl, more preferably from C₂₋₆-alkenyl, C₂₋₆-alkinyl and C₂₋₆-alkylene-O-C₁₋₆-alkyl.

It is preferred that R⁹ is independently selected from halogen, CN, OH, C₁₋₆-alkyl optionally substituted with 1 to 3 substituents selected from halogen, CN and OH, and O-C₁₋₆-alkyl optionally substituted with 1 to 3 substituents selected from halogen, CN and OH.

The variant I is preferably selected from 2 or 3.

The variant m is preferably selected from 2, 3 or 4, more preferably from 2 or 3.

As regards compounds of formula (I), T is preferably selected from the group consisting of the following radicals:

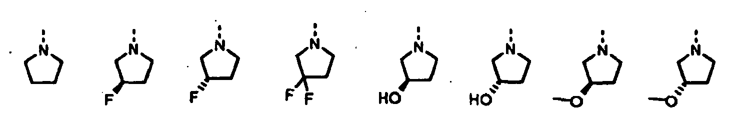

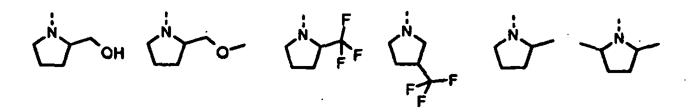

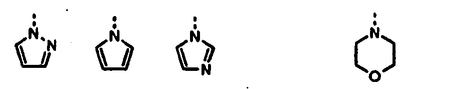

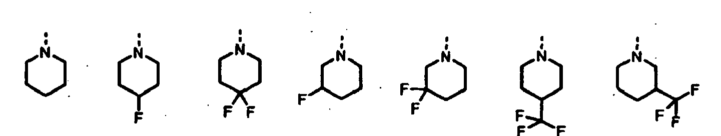

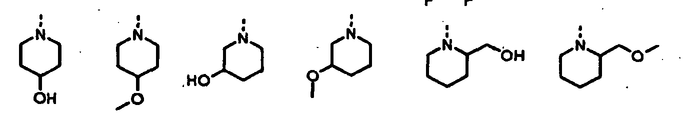

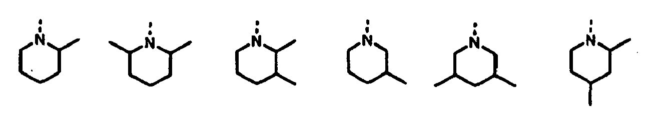

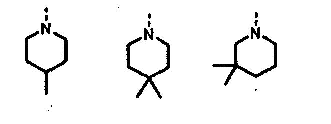

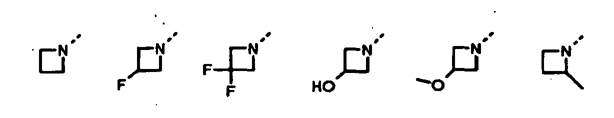

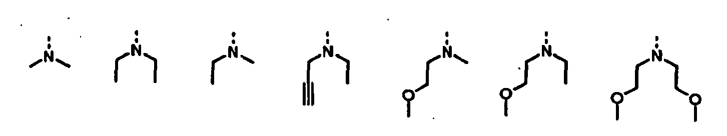

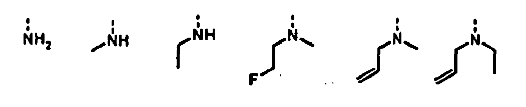

In a further preferred embodiment, R⁵ is preferably selected from the group consisting of

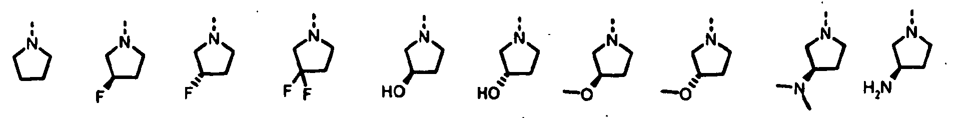

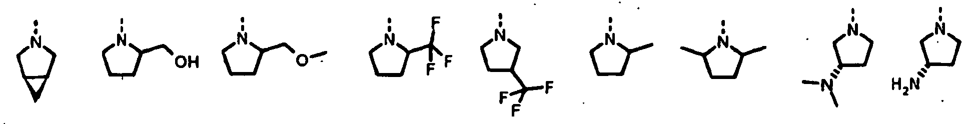

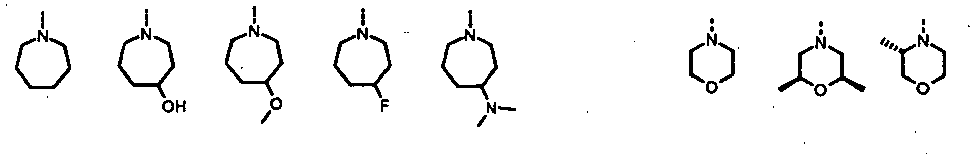

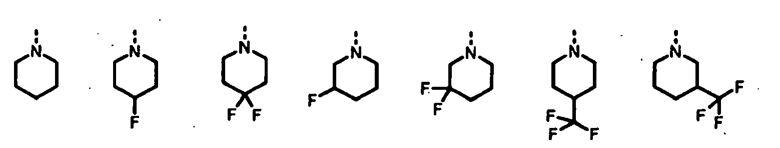

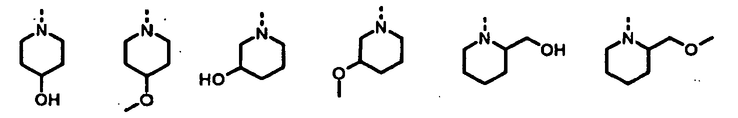

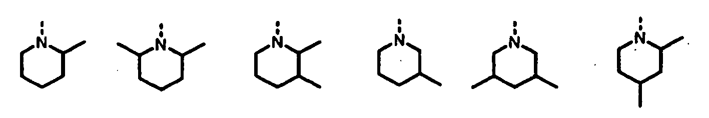

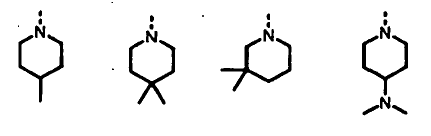

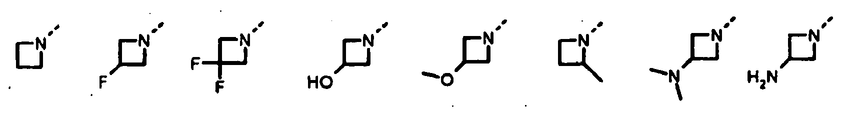

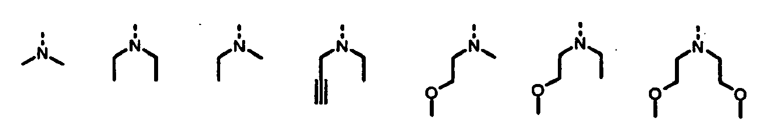

Compounds of the formula (I) in which some or all of the above-mentioned groups have the preferred or more preferred meanings are also an object of the present invention.

In the above and the following, the employed terms have the meaning as described below:
Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple - bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated, unsaturated or aromatic heterocycles are further encompassed by the above term. Examples thereof include azetidine, pyrrolidine, piperidine, azepane, piperazine, pyridine, pyrimidine, pyrazine, pyrrole, imidazole, and pyrazole.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

The compounds of structural formula (I) are particularly useful as antagonists of MC-4R. Thus, they are preferably used for the preparation of a medicament for the treatment and/or prevention of cancer cachexia, muscle wasting, anorexia, anxiety and depression.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutical acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor modulators and as such are useful in the treatment, control or prevention of diseases, disorders or conditions responsive to the inactivation of one or more of the melanocortin receptors including, but not limited to, MC-1 R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritics, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

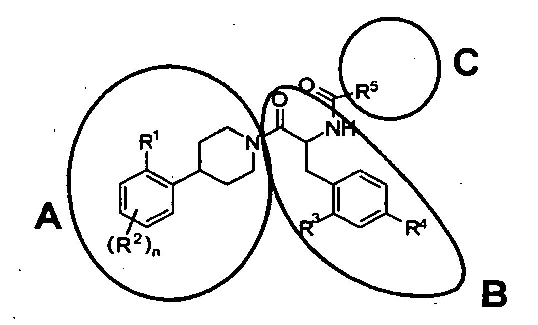

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of projective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The B and C moieties of a compound of formula (I) are linked together via a urea function. The skilled artist can, therefore, readily envision numerous routes and methods of connecting the two moieties using different well known methods.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed wherein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- AcOH: acetic acid
- Boc: tert-butoxycarbonyl
- Boc₂O: di-tert-butyl dicarbonate
- Bz₂O₂: dibenzoylperoxide
- DAST: (diethylarnino)sulfur trifluoride
- DCM: dichloromethane
- DEAD: diethyl azodicarboxylate
- DIBAL-H: diisobutylaluminumhydride
- DIAD: diisopropyl azodicarboxylate
- DIEA: ethyl-diisopropylamine
- DMA: N,N-dimethylacetarnide
- DMAP: 4-dimethylaminopyridine
- DMF: N,N-dimethylformamide
- DMS: dimethylsulfide
- DMSO: dimethylsulfoxide
- dppf: 1,1'-bis(diphenylphosphino)-ferrocen
- EDCl: 1-(3-dimethylaminopropyl)-3-ethylcarbodiimide hydrochloride
- Et₂O: diethyl ether
- EtOAc: ethyl acetate
- EtOH: ethanol
- Fmoc: 9-fluorenylmethyloxycarbonyl
- Fmoc-OSu: 9-fluorenylmethyloxycarbonyl-N-hydroxysuccinimide
- HOAt: 1-hydoxy-7-azabenzotriazole
- HOBt: 1-hydroxybenzotriazole hydrate
- h: hour(s)
- MeCN: acetonitrile
- MeOH: methanol
- NBS: N-bromosuccinimide
- NMM: N-methylmorpholine
- PG: protecting group
- PPh₃: triphenylphosphine
- TEBAC: benzyltriethylammonium chloride
- TFA: trifluoroacetic acid
- THF: tetrahydrofurane
- TMSCI: trimethylsilylchloride

The following amino acid derivatives were custom synthesized by PepTech Corporation, 20 Mall Road, Suite 460, Burlington, MA 01803 USA: D-2-chloro-4-fluorophenylalanine methyl ester hydrochloride, D-4-chloro-2-fluorophenylalanine methyl ester hydrochloride, and D-2,4-difluoro-phenylalanine methyl ester hydrochloride.

Cis-3-aza-bicyclo[3.1.0]hexane hydrochloride was prepared as described in US4,183,857.

### Reaction scheme 1:

### Synthesis of A Moieties with Alkylether Spacer (R¹=-O(C(R⁶)₂)ₘ-T)

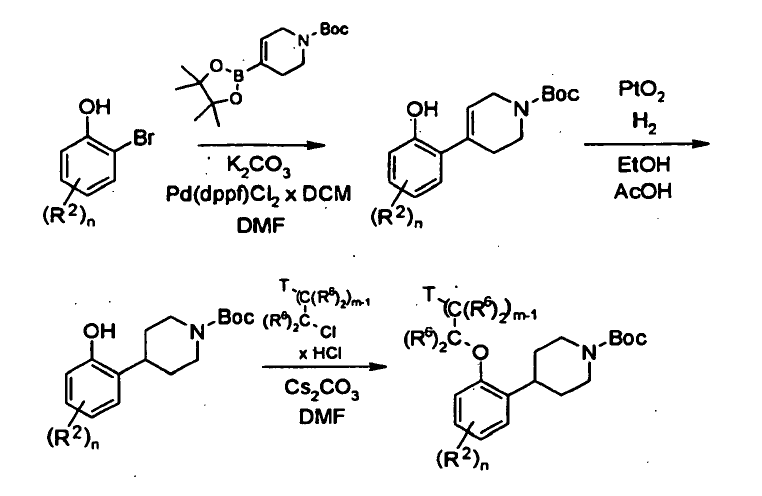

As shown in Reaction Scheme 1, optionally substituted 2-bromo-phenol and 4-(4,4,5,5-tetramethyl-[1,3,2]dioxaborolan-2-yl)-3,6-dihydro-2H-pyridine-1-carboxylic acid tert-butyl ester (Tetrahedron Lett. 2000, 41, 3705-3708) are reacted in a Suzuki coupling in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature. The resulting tetrahydropyridine can be hydrogenated in the presence of a catalyst, such as PtO₂ or Pd/C, to yield the protected piperidine. The piperidine is further reacted with an alkylchloride or alkylbromide bearing the capping group T in the presence of a base such as Cs₂CO₃ or NaH in an appropriate solvent such as DMF to give the Boc-protected A moiety.

### Reaction scheme 2:

### Alternative Synthesis of A Moieties with Alkylether Spacer (R¹ = -O(C(R⁶)₂)ₘ-T)

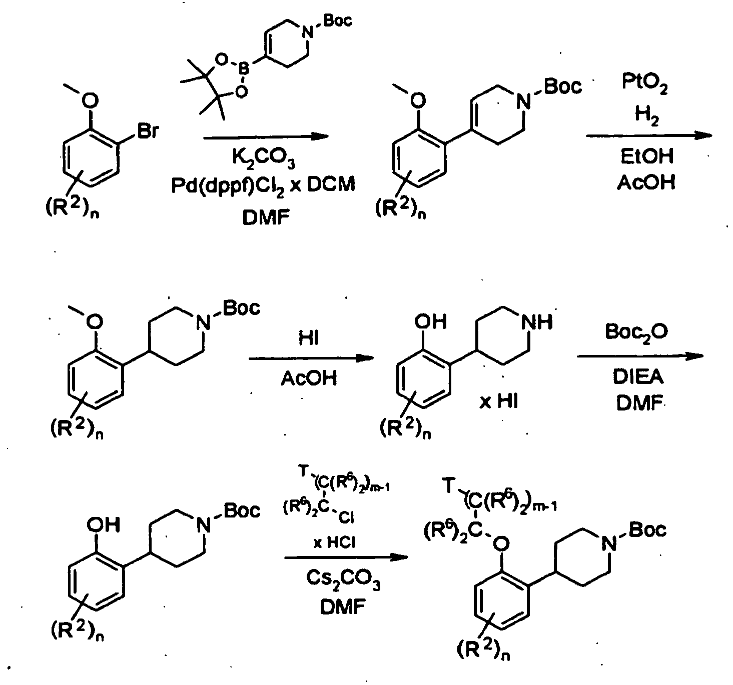

The synthesis of A Moieties bearing an alkylether spacer (R¹ = -O(C(R⁶)₂)ₘ-T) can alteratively be performed starting from optionally substituted 2-bromoanisole (see Reaction scheme 2). A Suzuki coupling with 4-(4,4,5,5-tetramethyl-[1,3,2]dioxaborolan-2-yl)-3,6-dihydro-2H-pyridine-1-cartioxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature leads to the corresponding tetrahydropyridine. The resulting tetrahydropyridine can be hydrogenated in the presence of a catalyst, such as PtO₂ or Pd/C, to yield the protected piperidine. The methylether can be cleaved with a reagent such as aqueous hydroiodic acid in acetic acid or trimethylsilyl iodide in chloroform, at a suitable temperature to get access to the corresponding phenol as hydroiodide. The Boc-protecting group, which is lost during this process, can subsequently be reintroduced by using a reagent such as Boc₂O in the presence of a base such as DIEA in an appropriate solvent such as DCM or DMF. The Boc-protected piperidine is further reacted with an alkylchloride or alkylbromide bearing the capping group T in the presence of a base such as Cs₂CO₃ or NaH in an appropriate solvent such as DMF to give the Boc-protected A moiety.

### Reaction scheme 3:

### Synthesis of A Moieties with Alkylether Spacer (R¹ =-O(C(R⁶)₂)ₘ-T) Using Mitsunobu Conditions

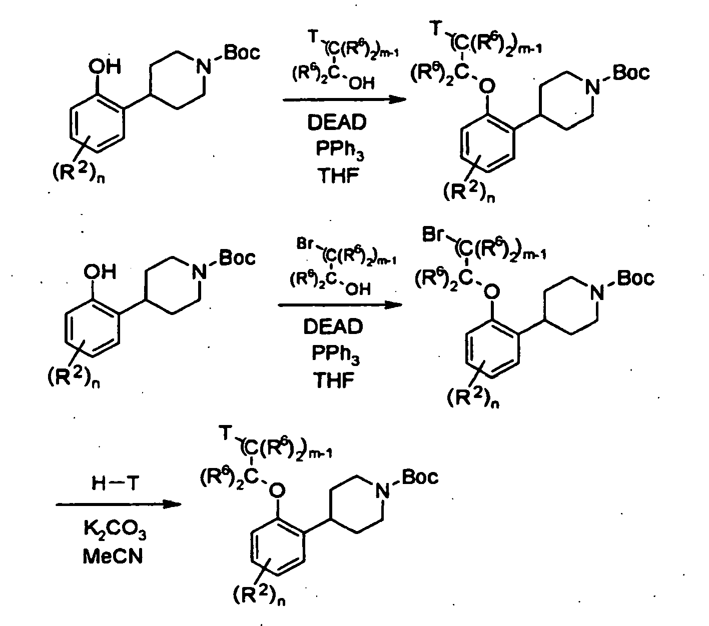

As shown in Reaction scheme 3, the intermediate product from Reaction schemes 1 and 2, optionally substituted 1-Boc-4-(2-hydroxy-phenyl)-piperidine, can also be alkylated with an ω-T-capped alkylalcohol in the presence of a reagent such as DEAD or DIAD, and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding phenolether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

### Reaction scheme 4:

### Synthesis of A Moieties with Alkylene Spacer (R¹ = -(C(R⁶)₂)ₗ-T, I = 3)

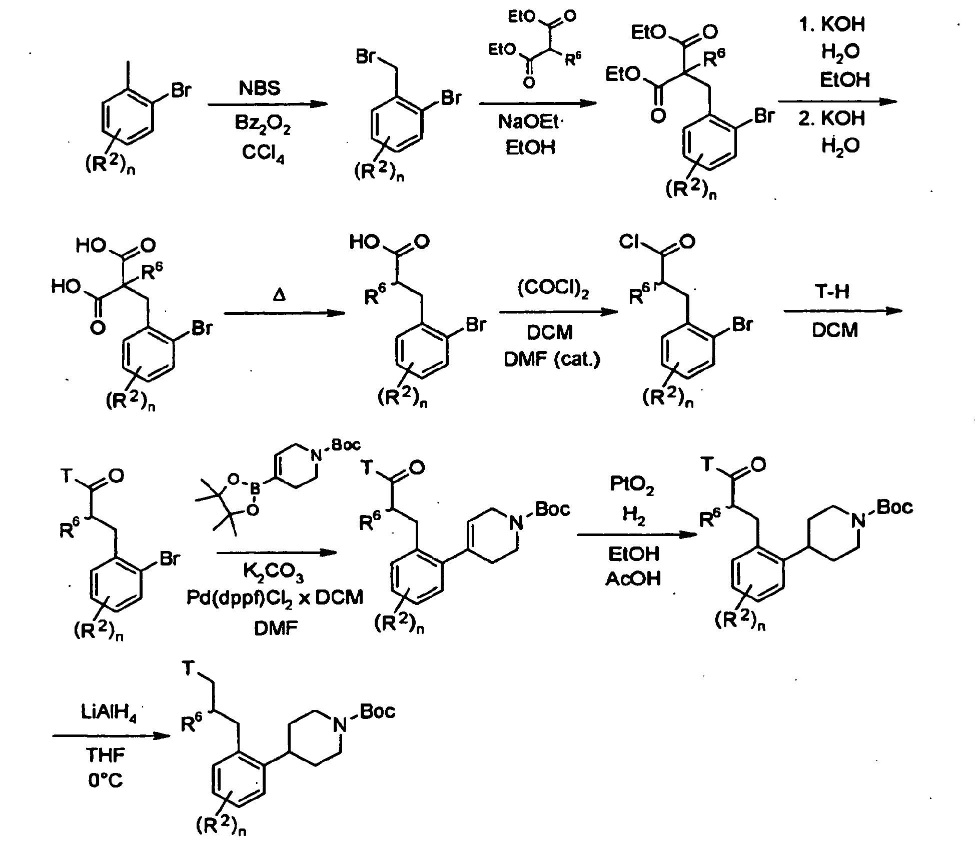

The first route for the synthesis of A moieties bearing an alkylene spacer (R¹ = -(C(R⁶)₂)ₗ-T) is depicted in Reaction scheme 4. Optionally substituted 2-bromotoluene is brominated with NBS in the presence of a radical starter such as Bz₂O₂ in an appropriate solvent such as CCl₄ at a suitable temperature to yield the corresponding benzylbromide. The benzylbromide is reacted with optionally substituted diethyl malonate in the presence of a base such as sodium ethoxide in a suitable solvent such as ethanol. Subsequent saponification with a base such as KOH in an appropriate solvent such as water-ethanol mixture followed by a second saponification step with a suitable base such as KOH in a solvent such as water leads to the alkylated malonic acid which is decarboxylated at an appropriate temperature. The product of this reaction, optionally substituted 3-(2-bromophenyl)propionic acid, is converted to the acid chloride using a reagent such as oxalyl chloride or thionyl chloride in an inert solvent such as DCM with a catalytic amount of DMF, and reacted with the capping group T to form the corresponding amide. Optionally substituted 3-(2-bromophenyl)propionic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(li) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A , moiety. **Reaction scheme 5:**

### Alternative Route for the Synthesis of A Moieties with Alkylene Spacer (R¹ = -(CH₂)ₗ-T, I=3)

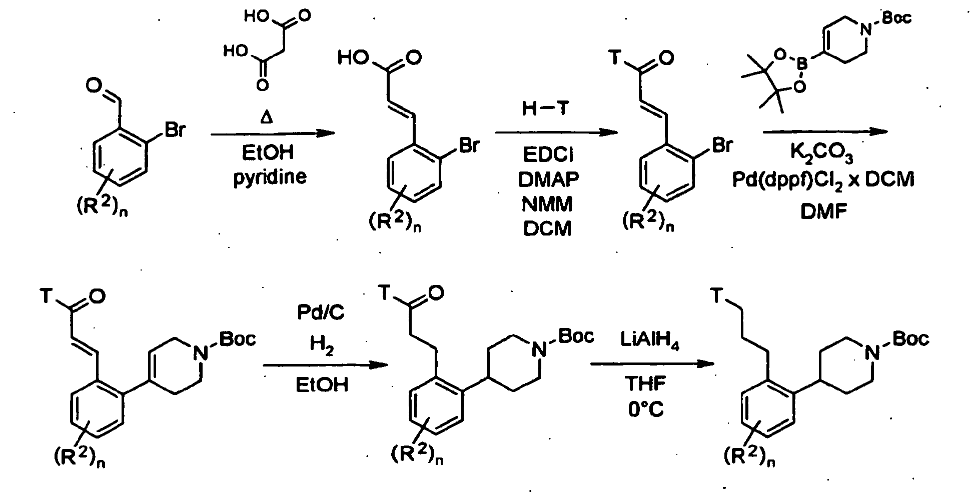

An alternative approach for the synthesis of of A moieties bearing an alkylene spacer (R¹= -(CH₂)ₗ-T) starts with optionally substituted 2-bromobenzaldehyde (see Reaction scheme 5). Reaction with malonic acid in an appropriate solvent such as ethanol, in the presence of a base such as pyridine, at a suitable temperature, leads to the corresponding 2'-bromo-cinnamic acid. Said acid is activated with a reagent such as EDCI in the presence of a catalyst such as DMAP and a base such as NMM in DCM, and reacted with the capping group T to form the corresponding amide. Optionally substituted 2'-bromo-cinnamic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-fen-ocene)palladium(II) DCM adduct, in an organic solvent such as DMF or toluene, at a suitable temperature to lead to the corresponding tetrahydropyridine. The resulting tetrahydropyridine and the cinnamic acid amide double bond can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 6:

### Alternative Route for the Synthesis of A Moieties with Alkylene Spacer (R¹ = -(C(R⁶)₂)ₗ-T, = 3)

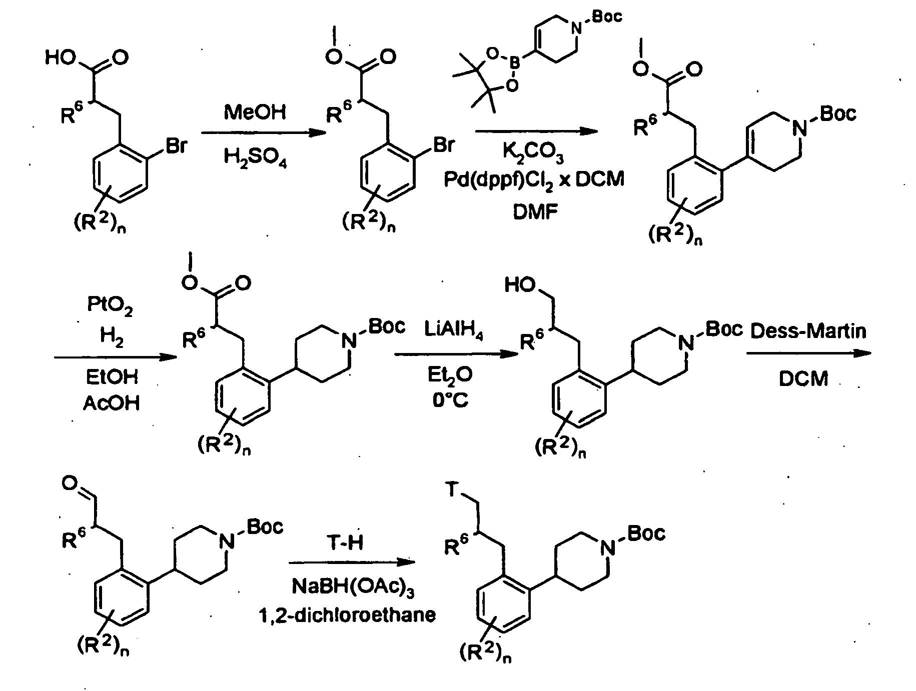

As shown in Reaction scheme 6, optionally substituted 3-(2-bromophenyl)propionic acid, is reacted with methanol in the presence of a catalyst such as sulfuric acid to form the corresponding methyl ester. Optionally substituted 3-(2-bromophenyl)propionic acid ester can be reacted with 4-(4,4,5,5-tetramethyl-[1,3,2]dioxaborolah-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain ester function can be reduced using a reagent such as LiAlH₄ or borane-THF complex in an appropriate inert solvent such as diethyl ether or THF at a suitable temperature to yield the corresponding alcohol which can subsequently be oxidized using a reagent such as Dess-Martin periodinane in an appropriate solvent such as DCM or using sulfurtrioxide-pyridine complex with a base such as triethylamine in a suitable solvent such as DCM. Optionally substituted 3-(2-bromophenyl)propionyl aldehyde is reacted with the capping group T in the presence of a reducing agent such as sodium triacetoxyborohydride in an appropriate solvent such as 1,2-dichloroethane to form the corresponding Boc-protected A moiety.

### Reaction scheme 7:

### Synthesis of A Moieties with Alkylene Spacer (R¹= -(C(R⁶)₂)ₗ-T, I = 3) Using a Negishi Coupling Reaction

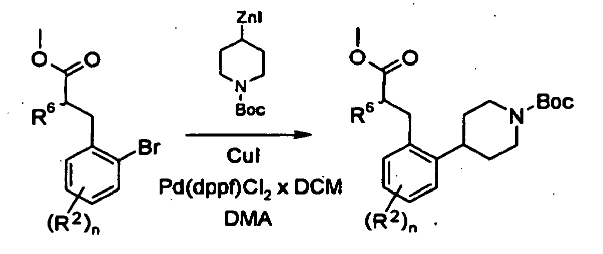

As shown in Reaction scheme 7, the intermediate product from Reaction scheme 6, optionally substituted 3-(2-bromophenyl)propionic acid ester can also be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(li) DCM adduct in an inert solvent such as DMA to yield the resulting phenylpiperidine which can be further processed as shown in Reaction scheme 6.

### Reaction scheme 8:

### Alternative Route for the Synthesis of A Moieties with Alkylene Spacer (R¹ =-(C(R⁶)₂)ₗ-T, I = 2, 3)

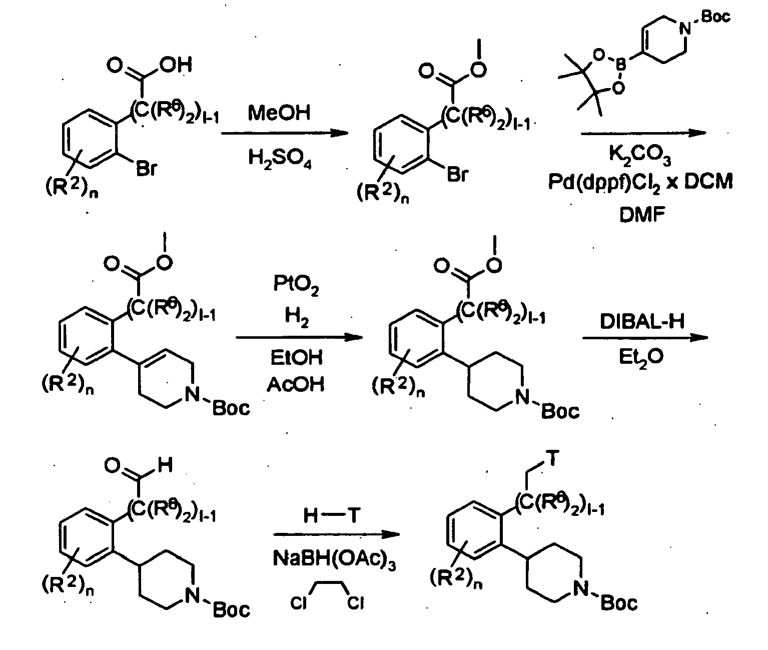

As shown in Reaction scheme 8 optionally substituted 3-(2-bromophenyl)propionic acid or 2-(2-bromophenyl)acetic acid is transformed to the corresponding methyl ester using a catalyst such as sulfuric acid. The ester can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine: The ester function can then be reduced to the corresponding aldehyde with DIBAL-H in an appropriate solvent such as Et₂O or THF at a suitable temperature. Reductive amination of the aldehyde with an amine T-H in the presence of a reducing agent such as sodium triacetoxyborohydride in an appropriate solvent such as 1,2-dichloroethane leads to the Boc-protected A moiety.

### Reaction scheme 9:

### Synthesis of A Moieties with Alkylene Spacer (R¹ = -(C(R⁶)₂)ₗ-T, I =2)

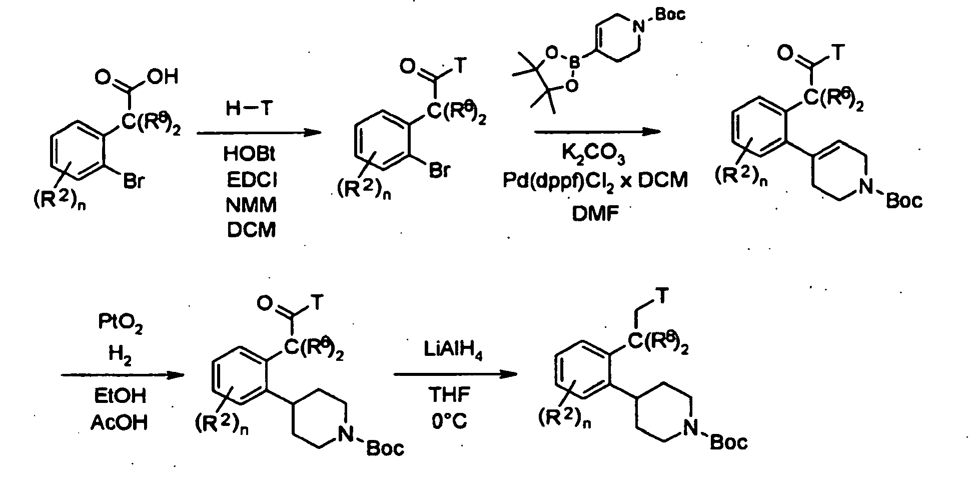

Synthesis of A Moieties with alkylene Spacer (R¹= -(C(R⁶)₂)ₗ-T, I = 2) can also be performed as described in Reaction scheme 9. Optionally substituted 2'-bromophenylacetic acid is activated with a reagent such as EDCI in the presence of a catalyst such as DMAP and a base such as NMM in DCM, and reacted with the capping group T to form the corresponding amide. Optionally substituted 2'-bromo-phenylacetic amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 10:

### Synthesis of A Moieties with Alkylene Spacer (R¹ = -(C(R⁶)₂)ₗ-T, I= 4)

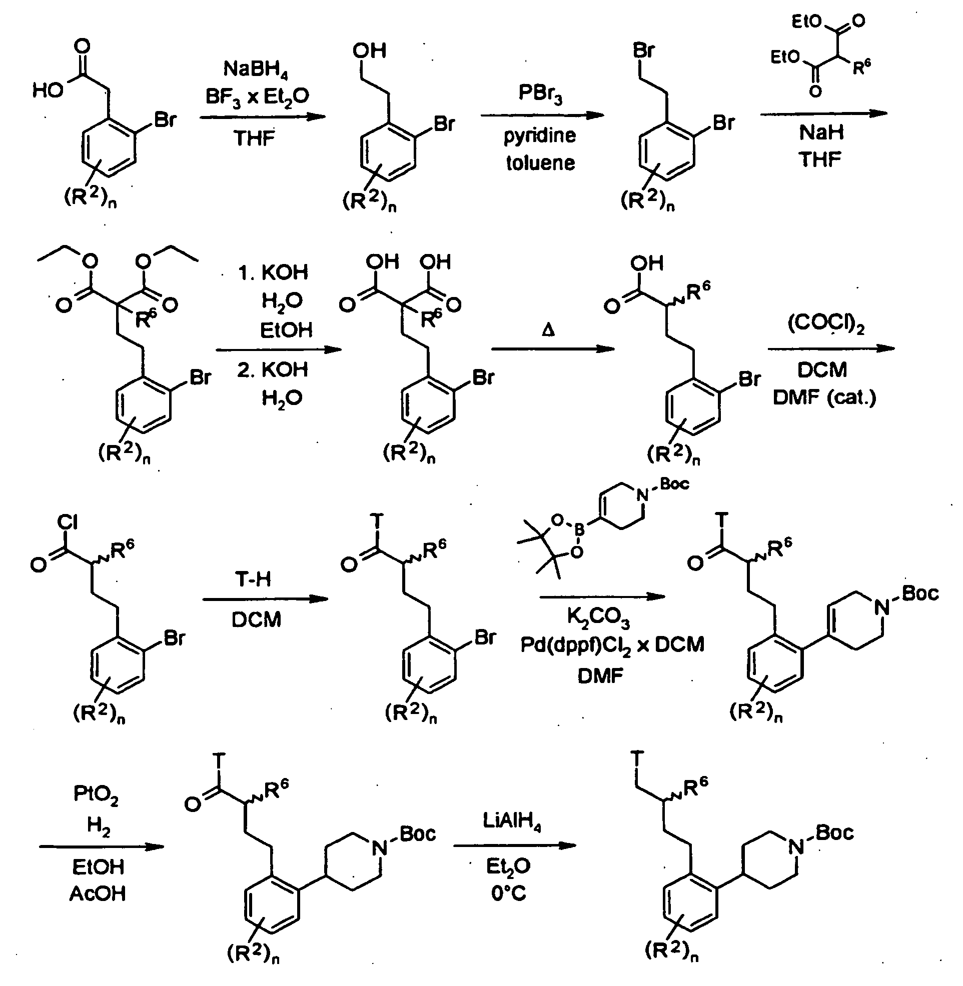

A route for the synthesis of A moieties bearing an C₄-alkylene spacer (R¹ = -(C(R⁶)₂)ₗ-T, I = 4) is depicted in Reaction scheme 10. Optionally substituted 2-bromophenylacetic acid is reduced with sodium borohydride in the presence of a reagent such like boron trifluoride diethyl etherate in an appropriate solvent such as THF at a suitable temperature to yield the corresponding phenylethylalcohol. Reaction of the alcohol with a bromination reagents such as phosphorous tribromide in the presence of a base such as pyridine in an appropriate solvent like toluene at a suitable temperature leads to the phenylethylbromide. The phenethylbromide is reacted with optionally substituted diethyl malonate in the presence of a base such as sodium hydride in a suitable solvent such as THF. Subsequent saponification with a base such as KOH in an appropriate solvent such as water-ethanol mixture followed by a second saponification step with a suitable base such as KOH in a solvent such as water leads to the alkylated malonic acid which is decarboxylated at an appropriate temperature. The product of this reaction, optionally substituted 3-(2-bromophenyl)butanoic acid, is converted to the acid chloride using a reagent such as oxalyl chloride or thionyl chloride in an inert solvent such as DCM with a catalytic amount of DMF, and reacted with the capping group T to form the corresponding amide. Optionally substituted 3-(2-bromophenyl)butanoic acid amide can be reacted with 4-(4,4,5,5-tetramethyl-[1,3,2]dioxaborolan-2.yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(lI) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

### Reaction scheme 11

### Synthesis of A Moiety Containing Cyclic Amines

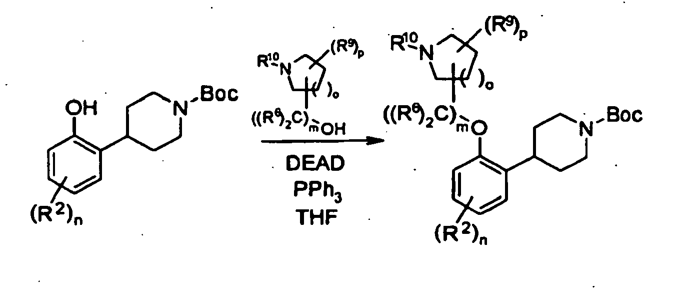

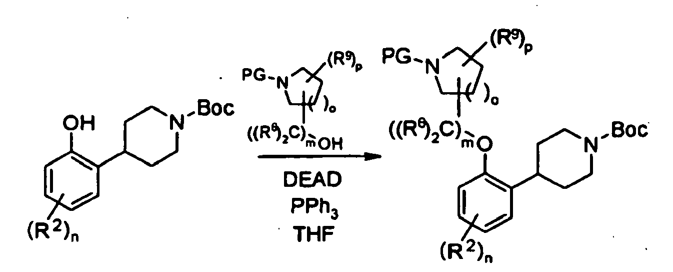

As shown in Reaction scheme 11, the intermediate product from Reaction schemes 1 and 2, optionally substituted 1-Boc-4-(2-hydroxy-phenyl)-piperidine, can also be alkylated with an alcohol which contains a cyclic tertiary amine moiety in the presence of a reagent, such as DEAD or DIAD and a phosphine such as PPh₃ in a suitable solvent such as THF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can be removed using standard methods.

### Reaction scheme 12

### A Moiety Deprotection

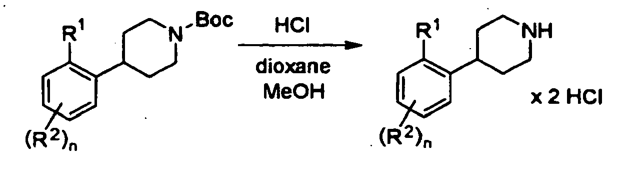

Generally, the starting material of Boc-protected phenylpiperidine (A moiety) can be deprotected in the presence of TFA/CH₂Cl₂, HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

### Reaction scheme 13:

### B-C Moiety Formation

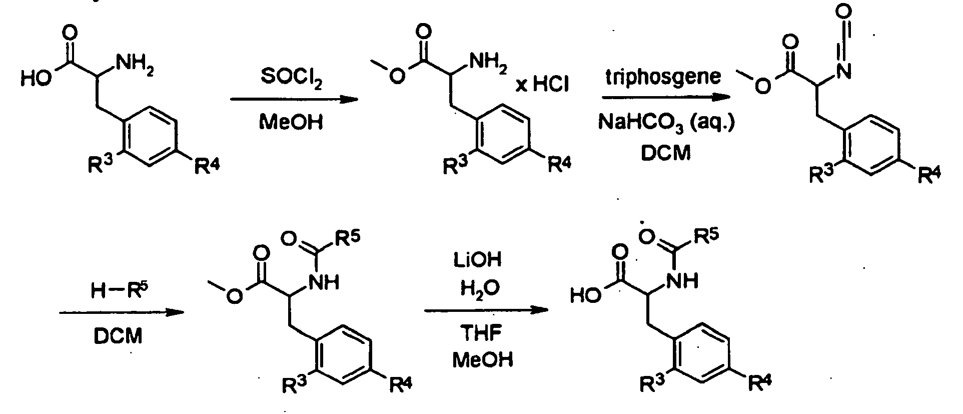

The B-C moieties can be synthesized as shown in Reaction scheme 13. Optionally substituted phenylalanine can be converted to the corresponding methyl ester hydrochloride using an activating reagent such as thionyl chloride or oxalyl chloride in methanol. Amino acid methyl ester hydrochloride can be reacted with a reagent such as triphosgene in the presence of a base such as NaHCO₃ (aq.) in a suitable solvent such as DCM to yield the isocyanate which can subsequently be reacted with an amine R⁵-H in a suitable solvent such as DCM. The ester function can be hydrolyzed with a base such as LiOH in a suitable solvent or solvent mixture such as water/THF/methanol to give access to the B-C-moiety.

### Reaction scheme 14:

### Coupling of A Moiety with B-C Moiety and Salt Formation

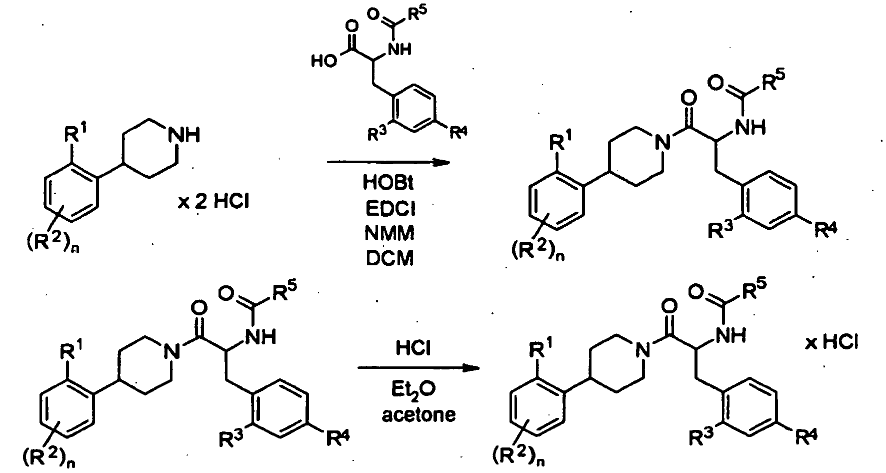

As shown in Reaction scheme 14, A moieties can be coupled with B-C moieties in the presence of EDCl/HOBt, a base such as N-methylmorpholine (NMM) and a solvent such as dichloromethane (DCM). A suitable solvent, such as DCM, DMF, THF or a mixture of the above solvents, can be used for the coupling procedure. Suitable base include triethylamine (TEA), diisopropylethylamine (DIEA), N-methylmorpholine (NMM), collidine or 2,6-lutidine. A base may not be needed when EDCl/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

The product can be transferred to a pharmaceutically acceptable salt such as a hydrochloride, using HCl in a solvent or solvent mixture such as diethyl ether/acetone.

### Reaction scheme 15:

### Urea Formation via Nitrophenylformate Intermediate

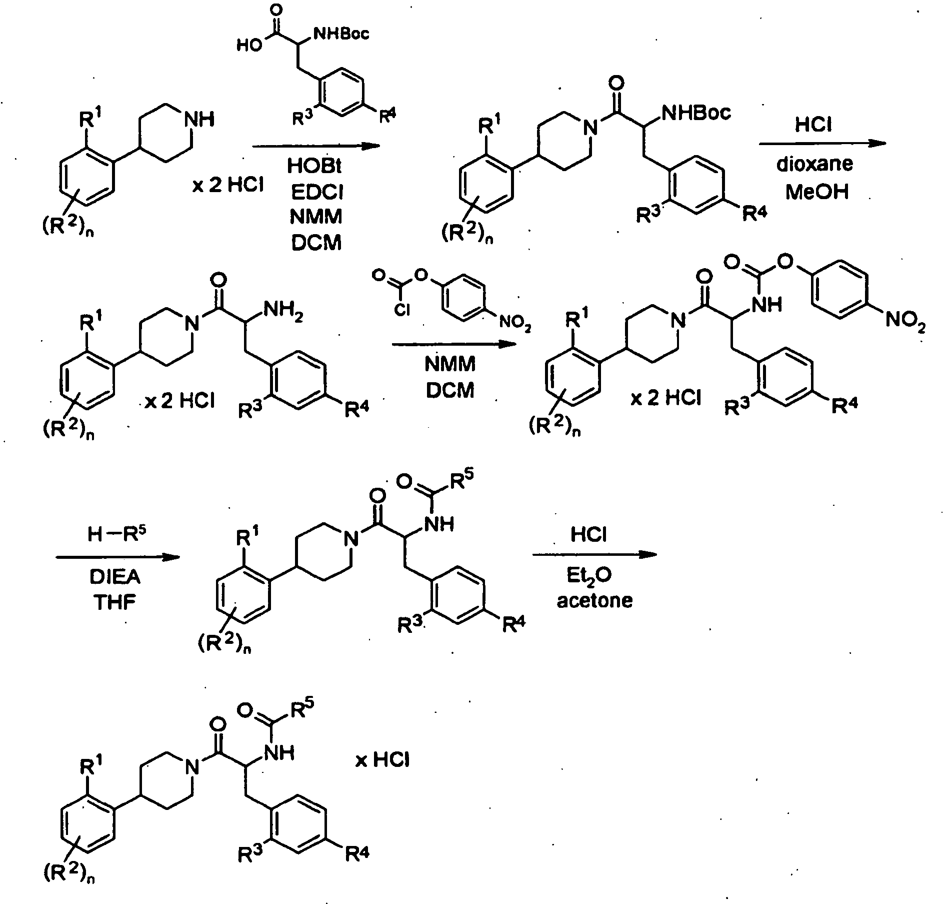

The three moieties can also be combined stepwise, as shown in Reaction scheme 15. An appropriate A moiety is coupled to a Boc-protected B moiety in the presence of EDCl/HOBt, a base such as N-methylmorpholine (NMM) and a solvent such as dichloromethane (DCM) followed by Boc deprotection with the aid of hydrogen chloride in a mixture of dioxane and methanol. The product can be reacted with 4-nitrophenyl chloroformate in the presence of a base such as NMM in an appropriate solvent such as DCM to yield the 4-nitrophenyl carbamate which subsequently can be treated with an amine H-R⁵ in the presence of a base such as DIEA in an appropriate solvent such as THF to give access to the target compound. The final product can be converted to a pharmaceutically acceptable salt as described above.

### Reaction scheme 16:

### Urea Formation Using 1-Methyl-3-(amino-1-carbonyl)-3H-imidazol-1-ium iodide as Reagent

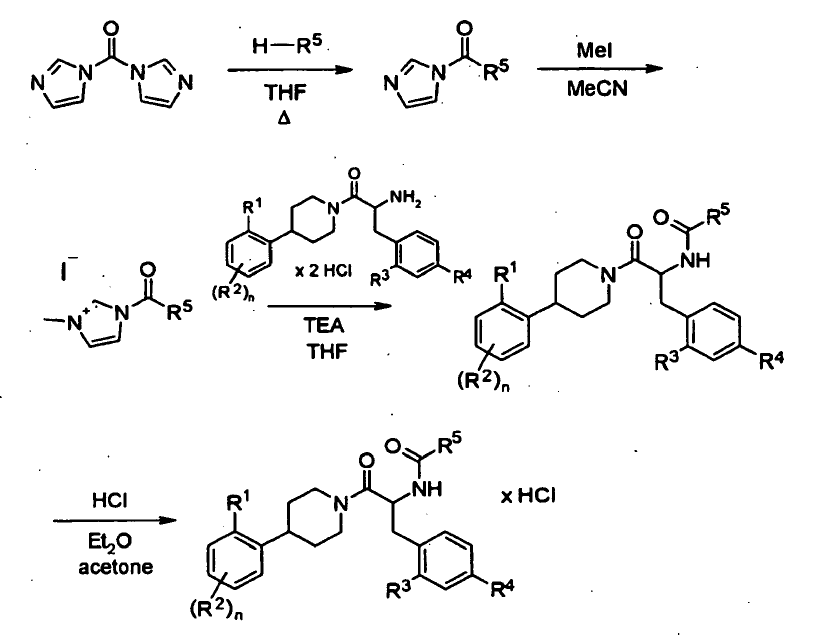

As shown in Reaction scheme 16 1,1'-carbonyldiimidazole can be reacted with an amine in an appropriate solvent such as THF at a suitable temperature. The product of this reaction is further reacted with methyl iodide in a suitable solvent such as acetonitrile to yield the 1-methyl-3-(amino-1-carbonyl)-3H-imidazol-1-ium iodide. This activated species is reacted with a deprotected A-B moiety in the presence of a base such as triethylamine in a suitable solvent such as THF to yield the final product The final product can be converted to a pharmaceutically acceptable salt as described above.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010 MS-detector in ESI+ modus with UV-detection at 214, 254 and 275 nm,
Column: Waters XTerra MS C18, 3.5 µm, 2.1 * 100 mm,
linear gradient with acetonitrile in water (0.1% HCOOH)
Flow rate of 0,4 ml/min;

| | |
|---|---|
| Mobile Phase A: | water (0.1 % HCOOH) |
| Mobile Phase B: | acetonitrile (0.1 % HCOOH) |

### Gradient A:

**linear gradient from 1% to 95% acetonitrile in water (0.1 % HCOOH)**

| | |
|---|---|
| 0.00 min | 1% B |
| 10.00 min | 95% B |
| 10.10 min | 99% B |
| 11.40 min | 99% B |
| 11.50 min | 1% B |
| 13.00 min | Pump STOP |

### Gradient B:

**linear gradient from 1% to 95% acetonitrile in water (0.1 % HCOOH)**

| | |
|---|---|
| 0.00 min | 1% B |
| 5.00 min | 95% B |
| 5.10 min | 99% B |
| 6.40 min | 99% B |
| 6.50 min | 1% B |
| 8.00 min | Pump STOP |

### Gradient C:

**linear gradient from 5% to 95% acetonitrile in water (0.1 % HCOOH)**

| | |
|---|---|
| 0.00 min | 5% B |
| 10.00 min | 95% B |
| 10.10 min | 99% B |
| 11.40 min | 99% B |
| 11.50 min | 1% B |
| 13.00 min | Pump STOP |

### Gradient D:

**linear gradient from 5% to 95% acetonitrile in water (0.1 % HCOOH)**

| | |
|---|---|
| 0.00 min | 5% B |
| 5.00 min | 95% B |
| 5.10 min | 99% B |
| 6.40 min | 99% B |
| 6.50 min | 1% B |
| 8.00 min | Pump STOP |

### Gradient E:

**linear gradient from 10% to 60% acetonitrile in water (0.1 % HCOOH)**

| | |
|---|---|
| 0.00 min | 10% B |
| 10.00 min | 60% B |
| 10.10 min | 99% B |
| 11.40 min | 99% B |
| 11.50 min | 1% B |
| 13.00 min | Pump STOP |

### Gradient F:

**linear gradient from 1% to 30% acetonitrile in water (0.1 % HCOOH)**

| | |
|---|---|
| 0.00 min | 1% B |
| 10.00 min | 30% B |
| 10.10 min | 99% B |
| 11.40 min | 99% B |
| 11.50 min | 1% B |
| 13.00 min | Pump STOP |

### Gradient G:

**linear gradient from 1 % to 70% acetonitrile in water (0.1 % HCOOH)**

| | |
|---|---|
| 0.00 min | 1% B |
| 10.00 min | 70% B |
| 10.10 min | 99% B |
| 11.40 min | 99% B |
| 11.50 min | 1% B |
| 13.00 min | Pump STOP |

### Gradient H:

**linear gradient from 1% to 60% acetonitrile in water (0.1 % HCOOH)**

| | |
|---|---|
| 0.00 min | 1% B |
| 10.00 min | 60% B |
| 10.10 min | 99% B |
| 11.40 min | 99% B |
| 11.50 min | 1% B |
| 13.00 min | Pump STOP |

The following tables describe detailed examples of the invention which can be prepared according to the Reaction schemes 1 to 16. These examples are, however, not construed to limit the scope of the invention in any manner.

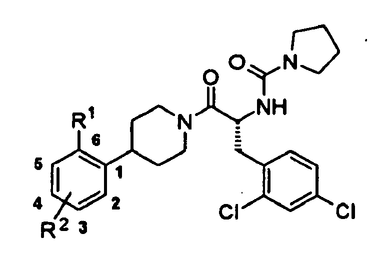

**Table 1:**

| | | | | HPLC | | MS | |
|---|---|---|---|---|---|---|---|
| No. | salt | R¹ | R² | t_{R} (min) | method | MW (calc.) free base | [M+H]⁺ (found) |
| 1 | HCl | 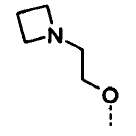 | H | 4.92 | A | 573.57 | 575 |
| 2 | HCl | 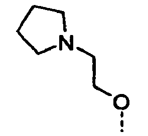 | H | 4.91 | A | 587.60 | 587 |
| 3 | HCl | 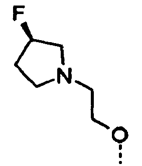 | H | 4.94 | A | 605.59 | 605 |
| 4 | HCl | 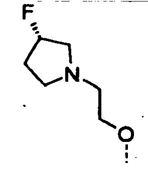 | H | 4.94 | A | 605.59 | 605 |
| 5 | HCl | 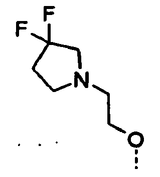 | H | 5.55 | A | 623.58 | 624 |
| 6 | HCl | 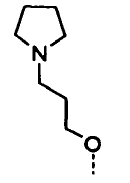 | H | 5.15 | A | 601.63 | 601 |
| 7 | HCl | 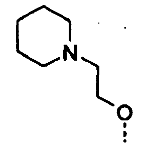 | H | 4.77 | C | 601.63 | 601 |
| 8 | HCl | 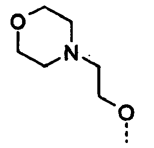 | H | 4.69 | C | 603.60 | 603 |
| 9 | HCl | 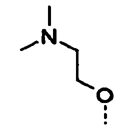 | H | 4.55 | C | 561.56 | 561 |
| 10 | HCl | 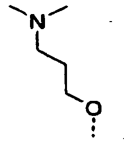 | H | 4.71 | C | 575.59 | 575 |
| 11 | HCl | 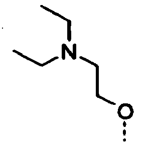 | H | 4.81 | C | 589.61 | 591 |
| 12 | - | 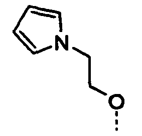 | H | 8.32 | A | 583.57 | 583 |
| 13 | HCl | 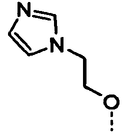 | H | 4.74 | C | 584.55 | 584 |
| 14 | HCl | 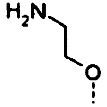 | 4-Cl | 4.73 | C | 567.94 | 567 |
| 15 | citric acid | 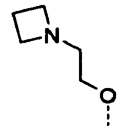 | 4-Cl | 5.04 | C | 608.02 | 609 |
| 16 | citric acid | 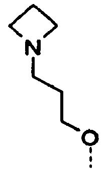 | 4-Cl | 5.21 | C | 622.03 | 621 |
| 17 | HCl | 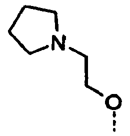 | 3-F | 4.76 | C | 605.59 | 605 |
| 18 | HCl | 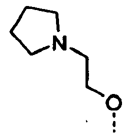 | 3-Cl | 5.18 | C | 622.04 | 621 |
| 19 | HCl | 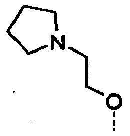 | 4-F | 4.94 | C | 605.59 | 605 |
| 20 | HCl | 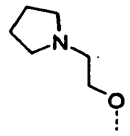 | 4-Cl | 5.23 | C | 622.04 | 621 |
| 21 | HCl | 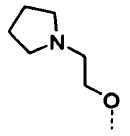 | 4-Me | 5.56 | C | 601.62 | 601 |
| 22 | HCl | 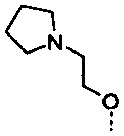 | 3-F 4-F | 5.27 | A | 623.58 | 622 |
| 23 | HCl | 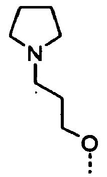 | 4-Cl | 5.31 | C | 636.07 | 635 |
| 24 | citric acid | 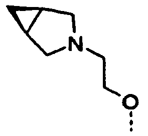 | 4-Cl | 5.41 | A | 634.04 | 633 |
| 25 | HCl | 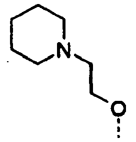 | 4-Cl | 5.40 | A | 636.07 | 635 |
| 26 | HCl | 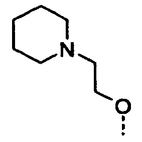 | 3-F 4-F | 5.36 | A | 637.61 | 637 |
| 27 | HCl | 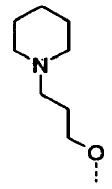 | 4-Cl | 5.49 | C | 650.10 | 649 |
| 28 | HCl | 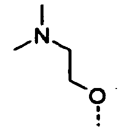 | 4-Cl | 5.23 | A | 596.00 | 595 |
| 29 | HCl | 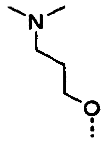 | 4-Cl | 5.24 | C | 610.03 | 609 |
| 30 | HCl | 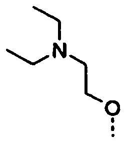 | 4-Cl | 5.33 | A | 624.05 | 623 |
| 31 | HCl | 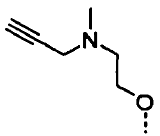 | 4-Cl | 5.19 | C | 620.02 | 619 |
| 32 | HCl | 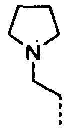 | H | 4.99 | A | 571.60 | 571 |
| 33 | HCl | 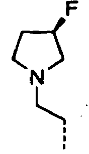 | H | 4.98 | A | 589.59 | 589 |
| 34 | HCl | 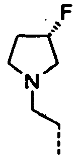 | H | 4.95 | A | 589.59 | 589 |
| 35 | HCl | 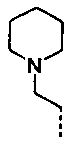 | H | 5.06 | A | 585.63 | 585 |
| 36 | HCl | 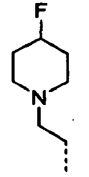 | H | 5.06 | A | 603.62 | 603 |
| 37 | HCl | 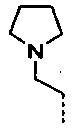 | 3-F | 5.04 | A | 589.59 | 589 |
| 38 | HCl | 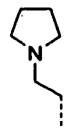 | 4-F | 5.00 | A | 589.59 | 589 |
| 39 | HCl | 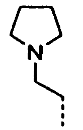 | 4-Cl | 5.26 | A | 606.04 | 605 |
| 40 | HCl | 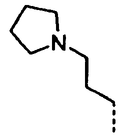 | H | 5.17 | A | 585.63 | 585 |
| 41 | HCl | 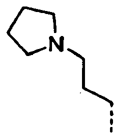 | 4-F | 5.29 | A | 603.62 | 603 |
| 42 | HCl | 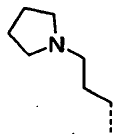 | 4-Cl | 5.39 | A | 620.07 | 619 |
| 43 | HCl | 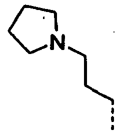 | 3-F 4-F | 3.12 | D | 621.60 | 621 |
| 44 | HCl | 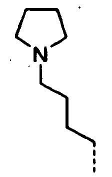 | 4-Cl | 5.58 | A | 634.10 | 633 |
| 45 | HCOOH | 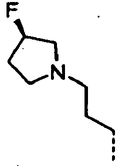 | 4-Cl | 3.28 | D | 638.06 | 637 |
| 46 | HCOOH | 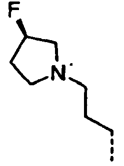 | 4-F | 3.10 | D | 621.60 | 621 |
| 47 | HCl | 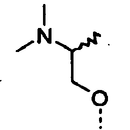 | H | 5.05 | A | 575.58 | 575 |
| 48 | - | 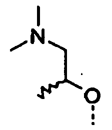 | H | 5.11 | A | 575.58 | 575 |
| 49 | HCl | 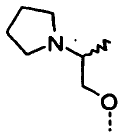 | 4-Cl | 5.34 | C | 636.06 | 635 |
| 50 | HCl | 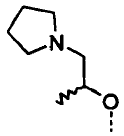 | 4-Cl | 5.35 | C | 636.06 | 635 |
| 51 | HCl | 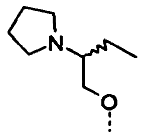 | 4-Cl | 5.33 | C | 650.09 | 649 |
| 52 | HCl | 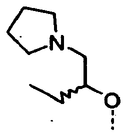 | 4-Cl | 5.34 | C | 650.09 | 649 |
| 53 | HCl | 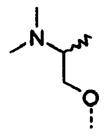 | 4-Cl | 5.02 | C | 610.02 | 609 |
| 54 | HCl | 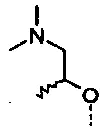 | 4-Cl | 4.99 | C | 610.02 | 609 |
| 55 | HCOOH | 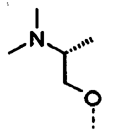 | 4-Cl | 3.26 | D | 610.02 | 609 |
| 56 | HCOOH | 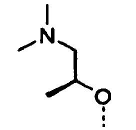 | 4-Cl | 3.07 | D | 610.02 | 609 |
| 57 | HCl | 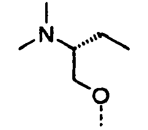 | 4-Cl | 5.30 | C | 624.05 | 623 |
| 58 | HCl | 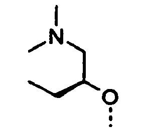 | 4-Cl | 5.36 | C | 624.05 | 623 |
| 59 | HCl | 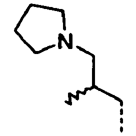 | 4-Cl | 5.53 | A | 634.10 | 633 |
| 60 | HCl | 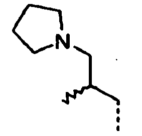 | 4-F | 5.47 | A | 617.64 | 617 |
| 61 | HCl | 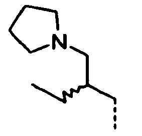 | 4-Cl | 5.63 | A | 648.12 | 647 |
| 62 | HCOOH | 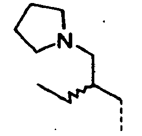 | 4-F | 3.26 | D | 631.67 | 631 |
| 63 | HCl | 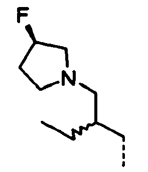 | 4-Cl | 5.69 | D | 666.11 | 666 |
| 64 | HCOOH | 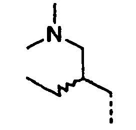 | 4-Cl | 5.50 | D | 622.08 | 621 |
| 65 | HCOOH | 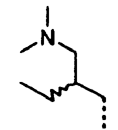 | 4-F | 3.21 | D | 605.63 | 605 |
| 66 | HCOOH | 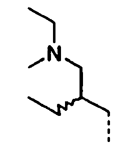 | 4-F | 3.25 | D | 619.66 | 619 |
| 67 | HCl | 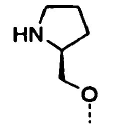 | 4-Cl | 4.99 | C | 608.01 | 607 |
| 68 | HCl | 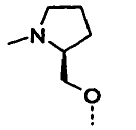 | 4-Cl | 5.01 | C | 622.03 | 621 |
| 69 | HCl | 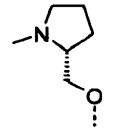 | 4-Cl | 5.24 | C | 622.03 | 621 |
| 70 | HCl | 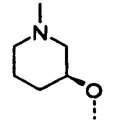 | 4-Cl | 5.27 | C | 622.03 | 621 |
| 71 | HCl | 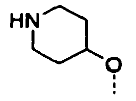 | 4-Cl | 5.01 | C | 608.01 | 607 |
| 72 | HCl | 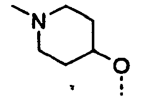 | 4-Cl | 5.01 | C | 622.03 | 621 |

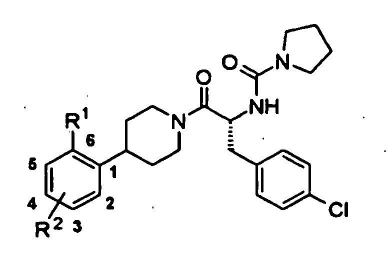

**Table 2:**

| | | | | HPLC | | MS | |
|---|---|---|---|---|---|---|---|
| No. | salt | R¹ | R² | t_{R} (min) | method | MW (calc.) free base | [M+H]⁺ (found) |
| 73 | HCl | 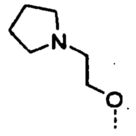 | H | 4.50 | C | 553.15 | 553 |
| 74 | HCl | 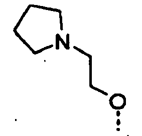 | 4-Cl | 5.07 | A | 587.59 | 587 |
| 75 | HCOOH | 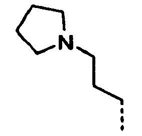 | 4-Cl | 4.51 | A | 585.62 | 585 |
| 76 | HCl | 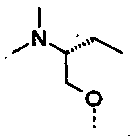 | 4-Cl | 5.04 | C | 589.60 | 589 |
| 77 | HCl | 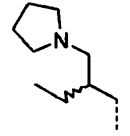 | 4-Cl | 5.40 | A | 613.68 | 613 |
| 78 | HCl | 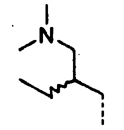 | 4-Cl | 5.20 | D | 587.64 | 587 |

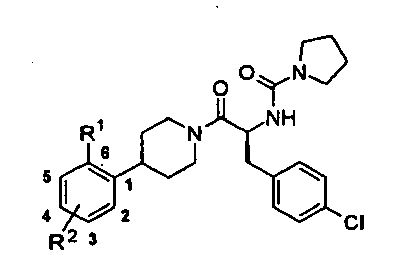

**Table 3:**

| | | | | HPLC | | MS | |
|---|---|---|---|---|---|---|---|
| No. | salt | R¹ | R⁷ | t_{R} (min) | method | MW (calc.) free base | [M+H]⁺ (found) |
| 79 | HCl | 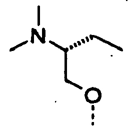 | 4-Cl | 5.00 | C | 589.60 | 589 |

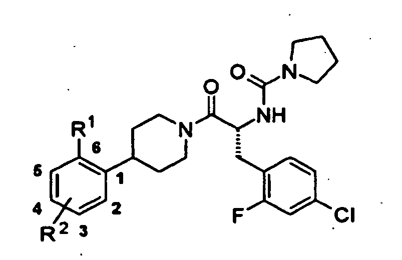

**Table 4:**

| | | | | HPLC | | MS | |
|---|---|---|---|---|---|---|---|
| No. | salt | R¹ | R² | t_{R} (min) | method | MW (calc.) free base | [M+H]⁺ (found) |
| 80 | HCl | 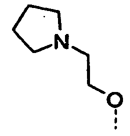 | 4-Cl | 4.93 | C | 605.59 | 605 |
| 81 | HCOOH | 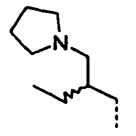 | H | 5.10 | D | 597.22 | 597 |
| 82 | HCl | 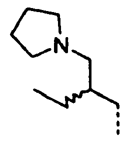 | 4-Cl | 5.36 | D | 631.67 | 631 |

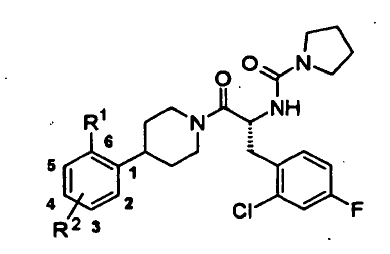

**Table 5:**

| | | | | HPLC | | MS | |
|---|---|---|---|---|---|---|---|
| No. | salt | R¹ | R² | t_{R} (min) | method | MW (calc.) free base | [M+H]⁺ (found) |
| 83 | HCOOH | 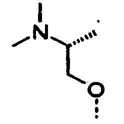 | 4-Cl | 3.07 | D | 593.57 | 593 |
| 84 | HCOOH | 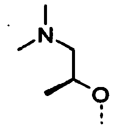 | 4-Cl | 3.08 | D | 593.57 | 593 |

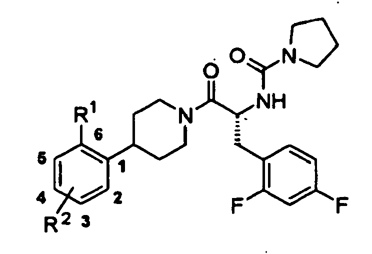

**Table 6:**

| | | | | HPLC | | MS | |
|---|---|---|---|---|---|---|---|
| No. | salt | R¹ | R² | t_{R} (min) | method | MW (calc.) free base | [M+H]⁺ (found) |
| 85 | HCl | 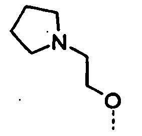 | 4-Cl | 5.46 | C | 589.13 | 589 |
| 86 | citric acid | 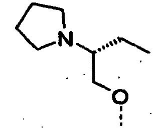 | 4-Cl | 5.15 | C | 617.18 | 617 |
| 87 | citric acid | 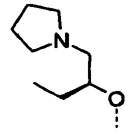 | 4-Cl | 4.96 | C | 617.18 | 617 |
| 88 | HCl | 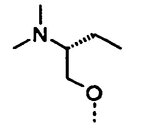 | 4-Cl | 4.70 | C | 591.14 | 591 |

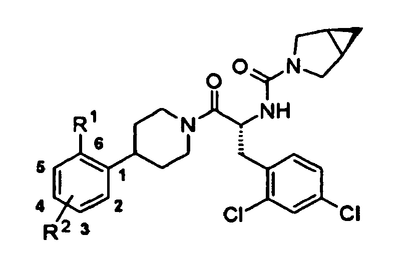

**Table 7:**

| | | | | HPLC | | MS | |
|---|---|---|---|---|---|---|---|
| No. | salt | R¹ | R² | t_{R} (min) | method | MW (calc.) free base | [M+H]⁺ (found) |
| 89 | citric acid | 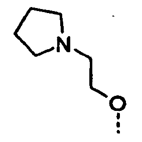 | 4-Cl | 5.99 | C | 634.04 | 633 |
| 90 | HCl | 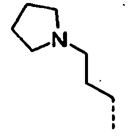 | 4-Cl | 6.29 | A | 632.08 | 631 |
| 91 | HCl | 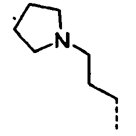 | 4-F | 6.10 | A | 615.62 | 615 |
| 92 | HCl | 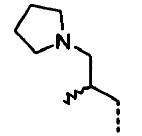 | 4-F | 6.19 | A | 629.65 | 629 |

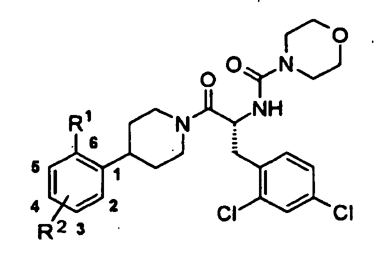

**Table 8:**

| | | | | HPLC | | MS | |
|---|---|---|---|---|---|---|---|
| No. | salt | R¹ | R² | t_{R} (min) | method | MW (calc.) free base | [M+H]⁺ (found) |
| 93 | HCl | 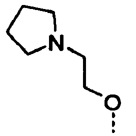 | H | 4.42 | C | 603.60 | 603 |
| 94 | HCl | 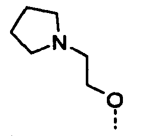 | 4-Cl | 5.84 | A | 638.03 | 637 |
| 95 | - | 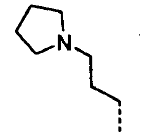 | H | 4.84 | A | 601.63 | 601 |
| 96 | HCl | 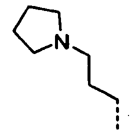 | 4-F | 5.04 | A | 619.61 | 619 |
| 97 | HCOOH | 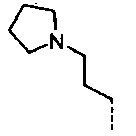 | 4-Cl | 4.68 | A | 636.07 | 635 |
| 98 | HCOOH | 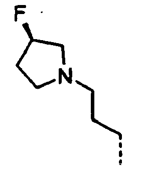 | 4-F | 5.55 | A | 637.60 | 637 |
| 99 | HCOOH | 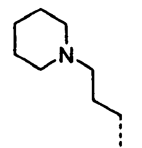 | 4-F | 5.65 | A | 633.64 | 633 |
| 100 | HCOOH | 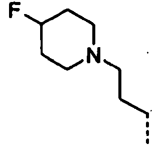 | 4-F | 5.64 | A | 651.63 | 651 |
| 101 | HCOOH | 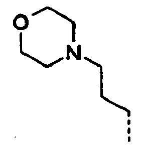 | 4-F | 5.45 | A | 635.61 | 635 |
| 102 | HCl | 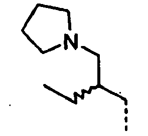 | 4-Cl | 5.42 | A | 664.12 | 663 |

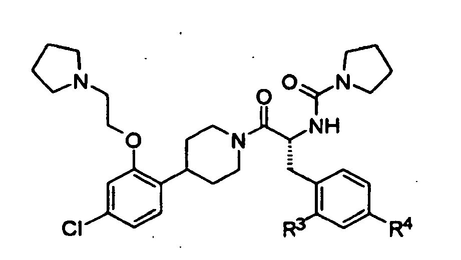

**Table 9:**

| | | | | HPLC | | MS | |
|---|---|---|---|---|---|---|---|
| No. | salt | R³ | R⁴ | t_{R} (min) | method | MW (calc.) free base | [M+H]⁺ (found) |
| 103 | HCl | H | F | 4.90 | A | 571.13 | 571 |
| 104 | HCl | Me | Cl | 3.12 | D | 601.62 | 601 |

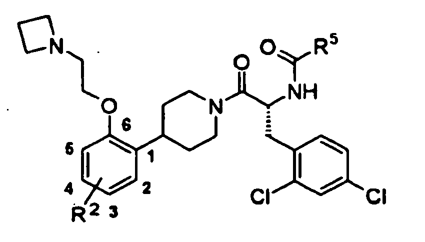

**Table 10:**

| | | | | HPLC | | MS | |
|---|---|---|---|---|---|---|---|
| No. | salt | R² | R⁵ | tR (min) | method | MW (calc.) free base | [M+H]⁺ (found) |
| 105 | HCl | H | 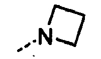 | 4.82 | A | 559.54 | 560 |
| 106 | HCl | H | 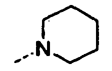 | 5.18 | A | 587.60 | 588 |

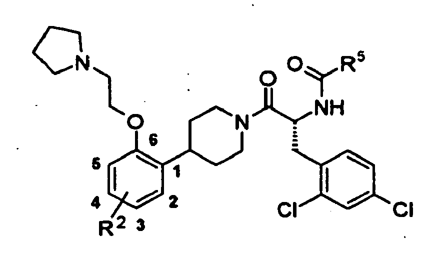

**Table 11:**

| | | | | HPLC | | MS | |
|---|---|---|---|---|---|---|---|
| No. | salt | R² | R⁵ | t_{R} (min) | method | MW (calc.) free base | [M+H]⁺ (found) |
| 107 | HCl | H | 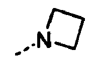 | 4.52 | C | 573.57 | 573 |
| 108 | HCl | H | 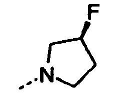 | 4.64 | C | 605.59 | 605 |
| 109 | HCl | H | 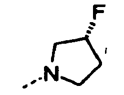 | 4.63 | C | 605.59 | 605 |
| 110 | HCl | H | 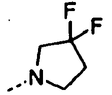 | 4.88 | C | 623.58 | 623 |
| 111 | HCl | H | 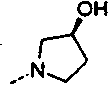 | 4.22 | C | 603.60 | 603 |
| 112 | HCl | H | 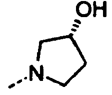 | 4.22 | C | 603.60 | 603 |
| 113 | 2xHCl | H | 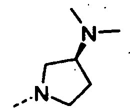 | 3.55 | C | 630.67 | 630 |
| 114 | 2xHCl | H | 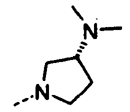 | 3.54 | C | 630.67 | 630 |
| 115 | HCl | H | 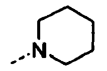 | 4.98 | C | 601.63 | 601 |
| 116 | HCl | H | 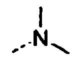 | 4.48 | C | 561.56 | 561 |
| 117 | HCl | H | 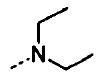 | 4.91 | C | 589.61 | 589 |
| 118 | - | 4-Cl | 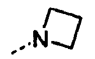 | 5.15 | A | 608.02 | 608 |
| 119 | citric acid | 4-Cl | 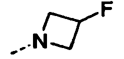 | 5.14 | A | 626.00 | 625 |
| 120 | citric acid | 4-Cl | 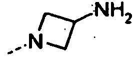 | 3.56 | C | 623.02 | 622 |
| 121 | 2 x HCOOH | 4-Cl | 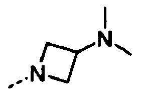 | 4.25 | C | 651.08 | 650 |
| 122 | citric acid | 4-Cl | 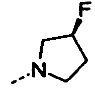 | 5.28 | A | 640.02 | 639 |
| 123 | citric acid | 4-Cl | 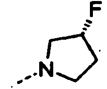 | 6.06 | A | 640.02 | 639 |
| 124 | HCl | 4-Cl | 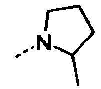 | 5.67 | C | 636.06 | 635 |
| 125 | HCl | 4-Cl | 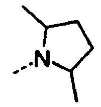 | 5.84 | C | 650.09 | 649 |
| 126 | citric acid | 4-Cl | 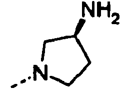 | 4.69 | C | 637.05 | 636 |
| 127 | citric acid | 4-Cl | 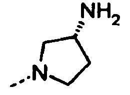 | 4.72 | C | 637.05 | 636 |
| 128 | 2 x HCOOH | 4-Cl | 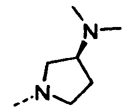 | 4.25 | A | 665.10 | 664 |
| 129 | 2 x HCOOH | 4-Cl | 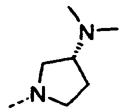 | 4.23 | A | 665.10 | 664 |
| 130 | HCl | 4-Cl | 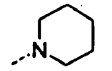 | 5.52 | A | 636.07 | 635 |
| 131 | HCl | 4-Cl | 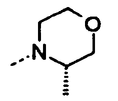 | 5.01 | C | 652.06 | 651 |
| 132 | citric acid | 4-Cl | 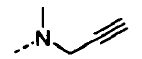 | 6.04 | A | 620.02 | 619 |
| 133 | 2 x HCl | 4-Cl | 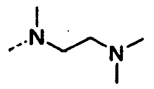 | 4.40 | A | 653.09 | 652 |

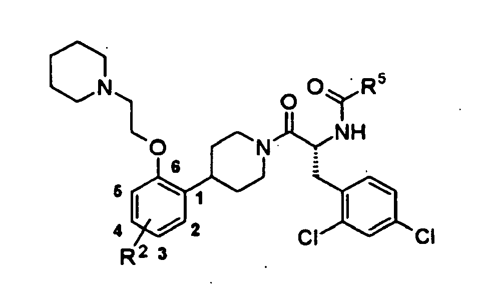

**Table 12:**

| | | | | HPLC | | MS | |
|---|---|---|---|---|---|---|---|
| No. | salt | R² | R⁵ | t_{R} (min) | method | MW (calc.) free base | [M+H]⁺ (found) |
| 134 | HCl | H | 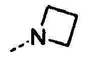 | 4.62 | C | 587.60 | 587 |
| 135 | HCl | H | 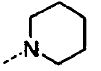 | 5.08 | C | 615.65 | 615 |

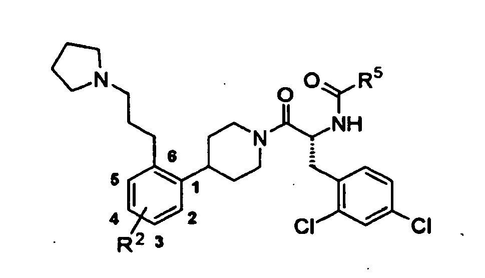

**Table 13:**

| | | | | HPLC | | MS | |
|---|---|---|---|---|---|---|---|
| No. | salt | R² | R⁵ | t_{R} (min) | method | MW (calc.) free base | [M+H]⁺ (found) |
| 136 | - | H | 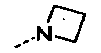 | 4.89 | A | 571.60 | 571 |
| 137 | HCl | H | 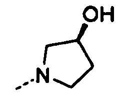 | 4.63 | A | 601.63 | 601 |
| 138 | HCl | H | 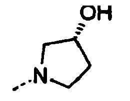 | 4.64 | A | 601.63 | 601 |
| 139 | HCl | H | 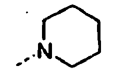 | 5.31 | A | 599.65 | 599 |
| 140 | HCl | H | 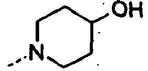 | 4.63 | A | 615.65 | 615 |
| 141 | HCl | H | 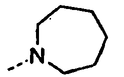 | 5.57 | A | 613.68 | 613 |
| 142 | HCOOH | 4-F | 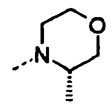 | 4.66 | A | 633.64 | 633 |
| 143 | HCOOH | 4-F | 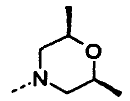 | 4.79 | A | 647.67 | 647 |
| 144 | HCOOH | 4-Cl | 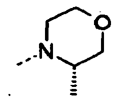 | 4.65 | A | 650.09 | 649 |
| 145 | HCOOH | 4-Cl | 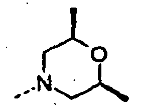 | 4.70 | A | 664.12 | 663 |

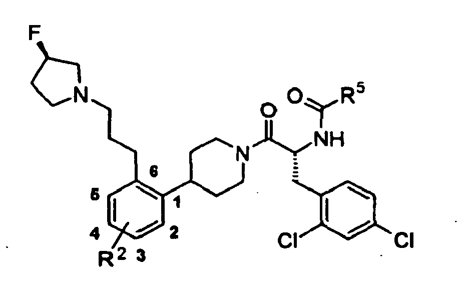

**Table 14:**

| | | | | HPLC | | MS | |
|---|---|---|---|---|---|---|---|
| No. | salt | R² | R⁵ | t_{R} (min) | method | MW (calc.) free base | [M+H]⁺ (found) |
| 146 | 2 x HCOOH | 4-F | 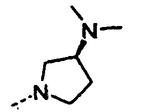 | 4.13 | A | 664.67 | 333* |
| 147 | 2 x HCOOH | 4-F | 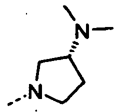 | 4.13 | A | 664.67 | 333* |
| 148 | 2 x HCOOH | 4-F | 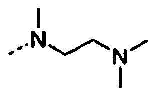 | 4.16 | A | 652.66 | 652 |

| | | | | | | | |
|---|---|---|---|---|---|---|---|
| * [M+2H]²⁺ | | | | | | | |

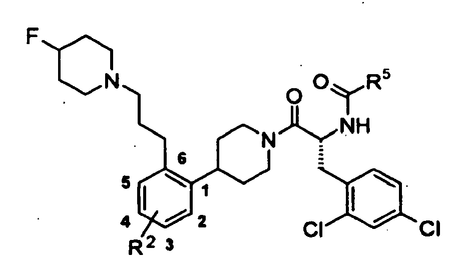

**Table 15:**

| | | | | HPLC | | MS | |
|---|---|---|---|---|---|---|---|
| No. | salt | R² | R⁵ | t_{R} (min) | method | MW (calc.) free base | [M+H]⁺ (found) |
| 149 | 2 x HCOOH | 4-F | 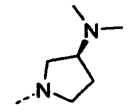 | 4.19 | A | 678.70 | 678 |
| 150 | 2 x HCOOH | 4-F | 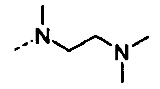 | 4.26 | A | 666.69 | 666 |

The following examples are provided to illustrate the invention and are not limiting the scope of the invention in any manner.

### Synthesis of B-C Moieties:

### B-C Moiety 1:

### Intermediate A1):

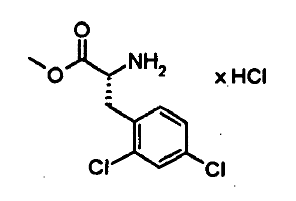

To a suspension of D-2,4-dichlorophenylalanine (10.00 g) in methanol (100 ml) was added dropwise thionylchloride (9.39 ml). During the course of the addition a clear solution was formed and the reaction started to reflux. The reaction mixture was kept under reflux for 2 h. After cooling to room temperature the mixture was evaporated to dryness at 40°C. The crude product was triturated in diethyl ether, and the insoluble compound was filtered off, washed with diethyl ether, and finally dried *in vacuo* at room temperature over P₂O₅ overnight. The product was obtained in form of colorless needles.

### Intermediate B1):

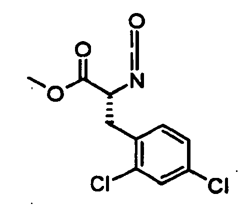

A 350 ml three-necked, flat-bottomed flask was equipped with a mechanical stirrer and charged with DCM (80 ml), saturated aqueous sodium bicarbonate solution (80 ml), and intermediate A1) (5.69 g). The biphasic mixture was cooled in an ice bath and stirred mechanically while triphosgene (1.96 g) was added in a single portion. The reaction mixture was stirred in the ice bath for 45 min and then poured into a 250 ml separatory funnel. The organic layer was collected, and the aqueous layer was extracted with three 20 ml portions of DCM. The combined organic layer was washed with water, dried over Na₂SO₄, filtered, and evaporated *in vacuo* to dryness to yield the crude product as a semisolid. The residue was purified by Kugelrohr distillation (200-240°C, 0.04-0.08 mbar). The product was obtained as clear colorless oil.

### Intermediate C1):

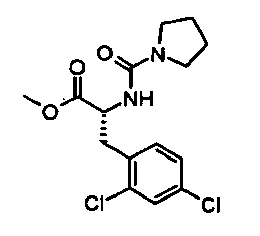

To an ice cooled solution of intermediate B1) (4.99 g) in DCM (50 ml) was added pyrrolidine (4.56 ml). After 10 minutes the ice bath was removed and stirring was continued for 4 h. The reaction mixture was evaporated *in vacuo.* The residue was redissolved in EtOAc and the organic layer was washed with 1 N HCl, water, sat. Na₂CO₃, water and brine. All the aqueous layers were extracted with EtOAc. The combined organic layer was dried over Na₂SO₄ and evaporated *in vacuo* to dryness.

### B-C Moiety 1:

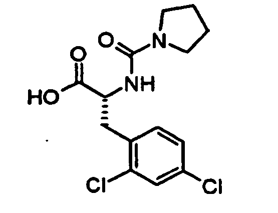

Intermediate C1) (6.28 g) was dissolved in MeOH (100 ml) and THF (30 ml) at 0°C. A solution of lithium hydroxide monohydrate (1.53 g) in water (30 ml) was added dropwise over the course of 5 min. The mixture was stirred at 0°C for 60 min and then acidified by adding 0.5 M HCl. The reaction mixture was extracted two times with EtOAc. The combined organic layer was washed two times with water and with brine, dried over Na₂SO₄ and evaporated *in vacuo.* The solid residue was triturated in Et₂O, then filtered off and washed with Et₂O. The product was obtained as a white solid.

### B-C Moiety 2:

### Intermediate A2):

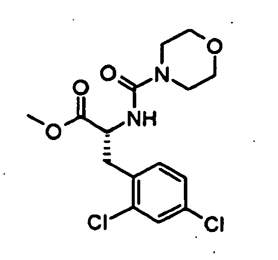

To an ice cooled solution of intermediate B1) (1.00 g) in DCM (10 ml) was added morpholine (954 µl). After 10 minutes the ice bath was removed and stirring was continued for 4 h. The reaction mixture was evaporated *in vacuo.* The residue was redissolved in EtOAc and the organic layer was washed with 1 N HCl, water, sat. Na₂CO₃, water and brine. All the aqueous layers were extracted with EtOAc. The combined organic layer was dried over Na₂SO₄ and evaporated *in vacuo* to dryness.

### B-C Moiety 2:

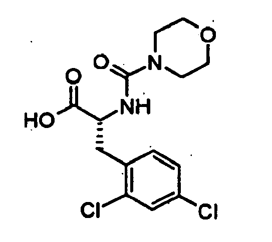

Intermediate A2) (1.26 g) was dissolved in MeOH (20 ml) and THF (6 ml) at 0°C. A solution of lithium hydroxide monohydrate (293 mg) in water (6 ml) was added dropwise over the course of 5 min. The mixture was stirred at 0°C for 60 min and then acidified by adding 0.5 M HCl. The reaction mixture was extracted two times with EtOAc. The combined organic layer was washed two times with water and with brine, dried over Na₂SO₄ and evaporated *in vacuo.* The solid residue was triturated in Et₂O, then filtered off and washed with Et₂O. The product was obtained as a white solid.

### B-C Moiety 3:

### Intermediate A3):

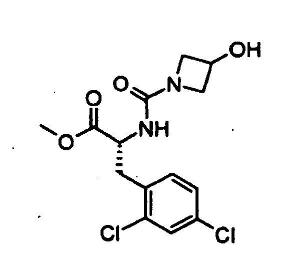

To an ice cooled solution of intermediate B1) (1.37 g) in DCM (15 ml) was added DIEA (2.61 ml) followed by 3-hydroxyazetidine hydrochloride (1.64 g). After 30 minutes the ice bath was removed and stirring was continued for 6 h. The reaction mixture was evaporated *in vacuo.* The residue was redissolved in EtOAc and the organic layer was washed with 1 N HCl (3x40 ml), sat. Na₂CO₃ (3x25 ml), water (2x25 ml) and brine (30 ml). The combined organic layer was dried over MgSO₄ and evaporated *in vacuo* to dryness. The product was purified by flash chromatography.

### Intermediate B3):

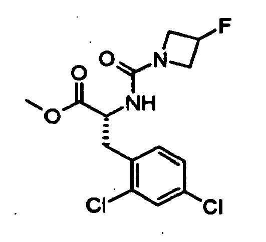

To an ice/NaCl-cooled solution of DAST (390 µl) in CH₂Cl₂ (3.0 ml) was added dropwise a solution of intermediate A3 in CH₂Cl₂ (6.0 ml). After 60 minutes the ice/NaCl bath was removed and stirring continued at room temperature for 2 h. The reaction mixture was treated with MeOH (5 ml) and evaporated *in vacuo.* The residue was redissolved in EtOAc (50 ml) and the organic layer was washed with 1 M HCl (3x20 ml), sat. Na₂CO₃ (3x15 ml), water (2x15 ml) and brine (10 ml). The organic layer was dried over MgSO₄ and evaporated *in vacuo* to dryness. The crude product was purified by flash chromatography.

### B-C Moiety 3:

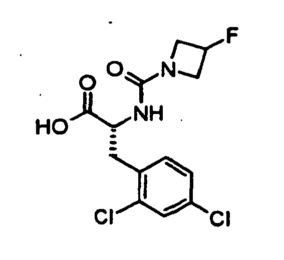

Intermediate B3) (150 mg) was dissolved in MeOH (3.00 ml) and THF (1.00 ml) at 0°C. A solution of lithium hydroxide monohydrate (35 mg) in water (1.25 ml) was added dropwise over the course of 5 min. The mixture was stirred at 0°C for 2 h and then acidified by adding 0.5 M HCl. The reaction mixture was extracted two times with EtOAc. The combined organic layer was washed two times with water and with brine, dried over MgSO₄ and evaporated *in vacuo.*

All B-C moieties used in this patent application can be prepared using this method starting from an appropriate Boc-protected amino acid and an appropriate amine. The introduction of basic C moieties was usually achieved using the 4-nitrophenylcarbamate pathway (Reaction scheme 15).

### Synthesis of Example 2:

### Intermediate 2a):

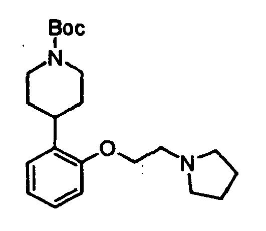

To a solution of 1-Boc-4-(2-hydroxy-phenyl)-piperidine (789 mg) in DMF (15 ml) was added 1-(2-chloroethyl)pyrrolidine hydrochloride (605 mg) and Cs₂CO₃ (3243 mg). The reaction was stirred at room temperature for 18 h. An additional amount of 1-(2-chloroethyl)pyrrolidine hydrochloride (483 mg) and Cs₂CO₃ (926 mg), was added and stirring at room temperature was continued for another 6 h. The reaction mixture was evaporated at 50°C *in vacuo* to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated *in vacuo* to dryness. The residue was finally dried under high vacuum at room temperature overnight.

### Intermediate 2b):

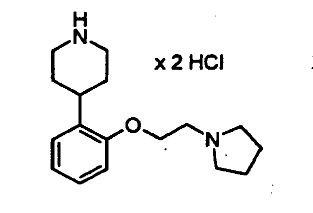

To Boc-protected intermediate 2a) (1002 mg) in methanol (5 ml) was added hydrogen chloride, 4.0 M sol. in 1,4-dioxane (25 ml) and the solution was stirred at room temperature for 2 h. The solvent was removed under reduced pressure. The residue was triturated in acetone (30 ml), filtered off, and washed with acetone (2 x 5 ml). Finally, it was dried *in vacuo* at room temperature over P₂O₅ overnight to yield a white solid.

### Example 2:

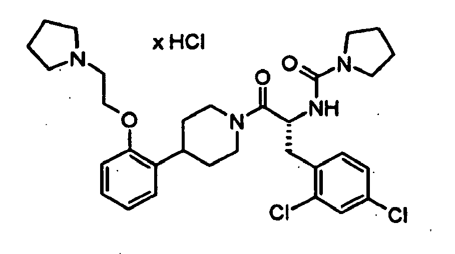

Intermediate 2b) (260 mg), B-C Moiety 1 (310 mg), and HOBt (172 mg) were dissolved in DCM (10 ml). NMM (227 µl) was added and the mixture stirred at room temperature for 30 min. EDCl (252 mg) was added, and the reaction stirred at room temperature for another 60 min. An additional amount of NMM (62 µl) was added and stirring continued at room temperature overnight. The reaction mixture was diluted with EtOAc (100 ml) and washed with sat. Na₂CO₃ (3 x 30 ml), water (2 x 20 ml) and brine (25 ml). The organic layer was dried over Na₂SO₄ and evaporated *in vacuo.* The crude product was purified by flash chromatography. The purified product was dissolved in EtOAc (3.00 ml), treated with 1 M HCl in Et₂O (633 µl), and the resulting suspension was diluted with hexane (20 ml). The precipitate was filtered off, washed with hexane (5 ml), and dried *in vacuo* at room temperature over P₂O₅ overnight. The product was obtained as a white solid.

### Synthesis of Example 3:

### Intermediate 3a):

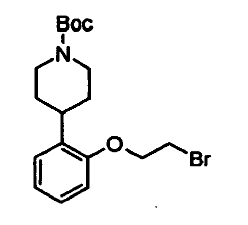

A solution of 1-Boc-4-(2-hydroxy-phenyl)-piperidine (1110 mg), 2-bromoethanol (565 µl), and triphenylphosphine (2100 mg) in THF (40 ml) under argon, was cooled in ice/H₂O. DEAD (ca. 40% in toluene, 3666 µl) was added dropwise, at a rate to keep the temperature below 5°C (ca. 25 min). After stirring for another 15 min in ice/H₂O, the cooling bath was removed and the mixture was stirred at room temperature overnight. Finally, the mixture was heated in an oil bath (45°C) for 4 h. The reaction mixture was cooled down to room temperature and then evaporated to dryness *in vacuo* at 40°C. The crude product was purified by flash chromatography to yield a slightly yellowish clear oil.

### Intermediate 3b):

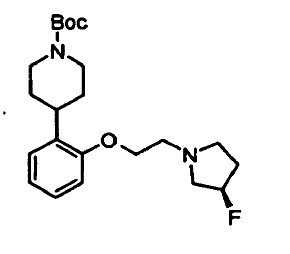

A suspension of intermediate 3a) (130 mg), (R)-2-fluoropyrrolidine hydrochloride (90 mg) and potassium carbonate (234 mg) in MeCN (5 ml) in a tightly capped flask was heated at 45°C in an oil-bath for 48 h. The reaction mixture was diluted with EtOAc (25 ml), filtered, and the filtrate was evaporated *in vacuo.* The product was purified by flash chromatography.

### Intermediate 3c):

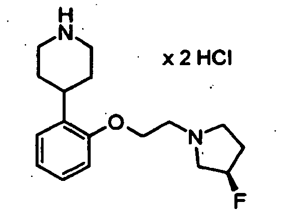

To Boc-protected intermediate 3b) (105 mg) in methanol (1 ml) was added hydrogen chloride, 4.0 M sol. in 1,4-dioxane (5 ml) and the solution was stirred at room temperature for 2 h. The solvent was removed under reduced pressure. The residue was triturated in acetone (6 ml), filtered off, and washed two times with acetone. Finally, it was dried *in vacuo* at room temperature over P₂O₅ overnight to yield a white solid.

### Example 3:

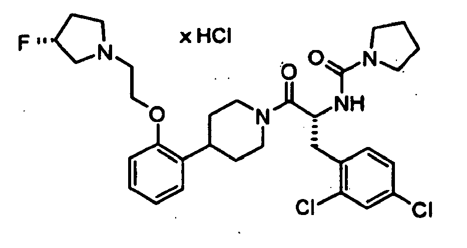

Intermediate 3c) (30 mg), B-C Moiety 1 (36 mg), and HOBt (19 mg) were dissolved in DCM (2.5 ml). NMM (26 µl) was added and the mixture stirred at room temperature for 30 min. EDCl (29 mg) was added, and the reaction stirred at room temperature for another 60 min. An additional amount of NMM (7 µl) was added and stirring, continued at room temperature overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated in *vacuo* to dryness. The residue was purified by flash chromatography. The purified product was dissolved in ethyl acetate (300 µl), and treated with 1 M HCl in Et₂O (26 µl) followed by hexane (3 ml). The precipitated salt was filtered off, washed with hexane (1 ml), and finally dried *in vacuo* at room temperature over P₂O₅ overnight.

### Synthesis of Example 13:

### Intermediate 13a):

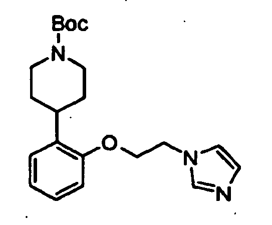

To a solution of 1-Boc-4-(2-hydroxy-phenyl)-piperidine (500 mg) in DMF (7.5 ml) was added 1-(2-chloroethyl) imidazole hydrochloride (680 mg) and Cs₂CO₃ (2060 mg). The reaction was stirred at room temperature for 18 h. An additional amount of 1-(2-chloroethyl) imidazole hydrochloride (300 mg) and Cs₂CO₃ (590 mg) were added and stirring at room temperature was continued for another 74 h. The reaction mixture was evaporated *in vacuo* to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated *in vacuo* to dryness. The residue was finally dried under high vacuum at room temperature overnight and purified by flash chromatography.

### Intermediate 13b):

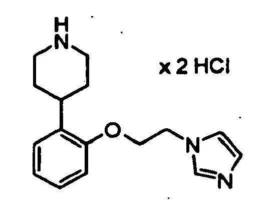

To Boc-protected intermediate 13a) (680 mg) in methanol (5 ml) was added hydrogen chloride, 4.0 M sol. in 1,4-dioxane (10 ml) and the solution was stirred at room temperature for 2 h. The solvent was removed under reduced pressure. The residue was triturated in acetone (30 ml), filtered off, and washed with acetone and diethyl ether. Finally, it was dried *in vacuo* at room temperature over P₂O₅ overnight to yield a white solid.

### Example 13:

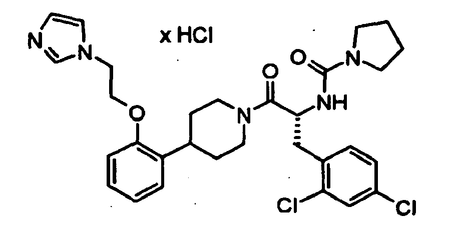

Intermediate 13b) (30 mg), B-C Moiety 1 (26 mg), and HOBt (14 mg) were dissolved in DCM (2 ml). NMM (19 µl) was added and the mixture stirred at room temperature for 30 min. EDCl (21 mg) was added, and the reaction stirred at room temperature for another 60 min. An additional amount of NMM (5 µl) was added and stirring continued at room temperature overnight. The reaction mixture was evaporated *in vacuo,* diluted with EtOAc, washed with sat. Na₂CO₃. water and brine. The aqueous layers were extracted with EtOAc. The combined organic layer was dried over Na₂SO₄, filtered and evaporated *in vacuo* to dryness. The residue was purified by flash chromatography. The purified product was dissolved in DCM, and treated with 1 M HCl in Et₂O (27 µl) and evaporated *in vacuo* to yield the hydrochloride as clear colorless oil.

### Synthesis of Example 14:

### Intermediate 14a):

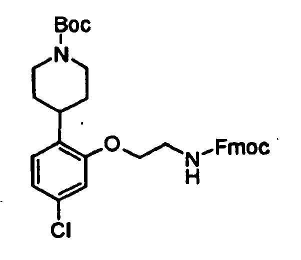

A solution of intermediate 27d) (100 mg) and N-(Fmoc)-ethanolamine (182 mg), and triphenylphosphine (168 mg) in anhydrous THF (4 ml) under argon, was cooled in ice/H₂O Then DEAD (ca. 40% in toluene, 294 µl) was added dropwise, at a rate to keep the temperature below 5°C (ca. 15 min). After stirring for another 10 min in ice/H₂O, the cooling bath was removed and the mixture was stirred at room temperature overnight. The reaction mixture was evaporated to dryness *in vacuo* at 40°C. The product was purified by column chromatography.

### Intermediate 14b):

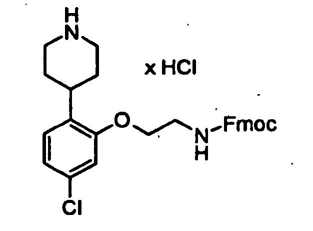

To Boc-protected intermediate 14a) (156 mg) in dioxane (0.5 ml) was added hydrogen chloride, 4.0 M sol. in 1,4-dioxane (3.0 ml) and the solution was stirred at room temperature for 2 h. The solvent was removed under reduced pressure. The residue was triturated in acetone and Et₂O, filtered off, and washed with acetone/Et₂O. Finally, it was dried *in vacuo* at room temperature over P₂O₅ overnight to yield a white solid.

### Intermediate 14c):

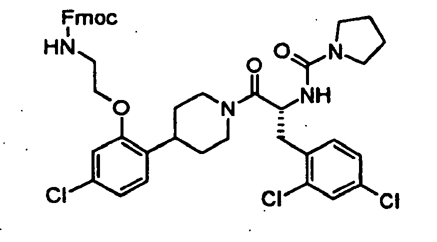

Intermediate 14b) (50 mg), B-C Moiety 1 (40 mg), and HOBt (22 mg) were dissolved in DCM (2 ml). NMM (19 µl) was added and the mixture stirred at room temperature for 30 min. EDCI (33 mg) was added, and the reaction stirred at room temperature for another 60 min. An additional amount of NMM (8 µl) was added and stirring continued at room temperature overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated in *vacuo* to dryness. The residue was purified by flash chromatography.

### Example 14:

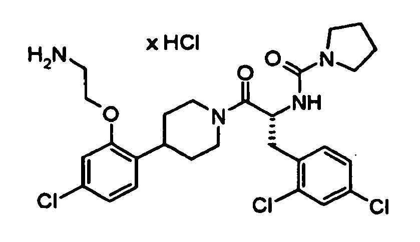

To a solution of intermediate 14c) (86 mg) in CH₂Cl₂ (2 ml) was added diethylamine (1 ml) and the reaction mixture was stirred at room temperature for 4 h. The reaction mixture was evaporated *in vacuo* and the residue was purified by flash chromatography. The purified product was dissolved in DCM and treated with 1 M HCl in Et₂O (97 µl) and evaporated in *vacuo.* The residue was dissolved in DCM and treated with diethyl ether. The precipitated salt was filtered off, washed with diethyl ether, and finally dried *in vacuo* at 40°C for 2 h.

### Synthesis of Example 20:

### Intermediate 20a):

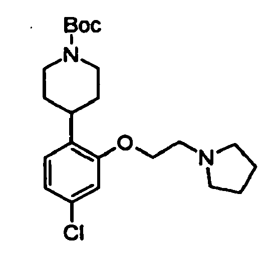

To a solution of intermediate 27d) (887 mg) in DMF (15 ml) was added 1-(3-chloroethyl)pyrrolidine hydrochloride (605 mg) and Cs₂CO₃ (3243 mg). The reaction was stirred at room temperature for 18 h. An additional amount of 1-(3-chloroethyl)pyrrolidine hydrochloride (483 mg) and Cs₂CO₃ (926 mg) were added and stirring at room temperature was continued for 6 h. The reaction mixture was evaporated at 40°C *in vacuo* to dryness and the residue was partitioned between EtOAc and water. The aqueous layer was extracted with EtOAc. The combined organic layer was washed with water and brine. The organic layer was dried over Na₂SO₄ and evaporated *in vacuo* to dryness. The crude product was purified using flash chromatography.

### Intermediate 20b):

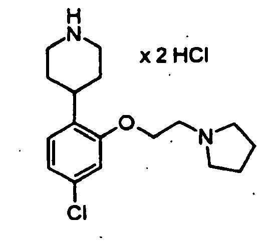

To Boc-protected intermediate 20a) (1170 mg) in dioxane (5 ml) was added hydrogen chloride, 4.0 M sol. in 1,4-dioxane (20 ml) and the solution was stirred at room temperature for 2 h. The solvent was removed under reduced pressure. The residue was triturated in acetone and Et₂O, filtered off, and washed with Et₂O. Finally, it was dried *in vacuo* at room temperature over P₂O₅ overnight to yield a white solid.

### Example 20:

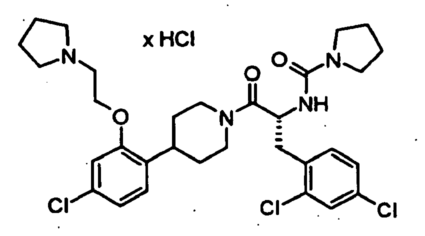

Intermediate 20b) (510 mg), B-C Moiety 1 (590 mg), and HOBt (308 mg) were dissolved in DCM (30 ml). NMM (417 µl) was added and the mixture stirred at room temperature for 30 min. EDCl (470 mg) was added, and the reaction stirred at room temperature for another 60 min. An additional amount of NMM (122 µl) was added and stirring continued at room temperature overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated *in vacuo* to dryness. The residue was purified by flash chromatography. The purified product was dissolved in DCM and treated with 1 M HCl in Et₂O (1.27 ml) and evaporated *in vacuo.* The residue was dissolved in DCM and treated with diethyl ether and hexane. The precipitated salt was filtered off, washed with hexane and diethyl ether, and finally dried *in vacuo* at 40°C for 2 h.

### Synthesis of Example 21:

### Intermediate 21a):

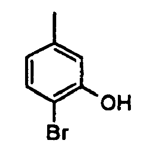

To a mixture of 48% aqueous HBr (89.5 ml) and water (90 ml) was added 6-amino-m-cresol (10.0 g). The mixture was kept under reflux for 10 min and then stirred at room temperature for 2 h. The suspension was cooled down to -15°C (ice/NaCl) and NaNO₂ (5.49 g) dissolved in water (90 ml) was added dropwise in a way to keep the temperature below -5°C. The mixture was stirred for 10 min and was added dropwise to an ice-cooled mixture of 48% aqueous HBr (53.7 ml), EtOAc (300 ml) and CuBr (22.8 g) during a period of 15 min. The resulting brown suspension was stirred at room temperature for 1 h and at 40°C for 3 h. The reaction mixture was diluted with EtOAc (300 ml), the organic phase was removed and the aqueous phase was extracted with diethyl ether (3 x 200ml). The combined organic layer was washed with 48% aqueous HBr (2 x 50 ml) followed by water (5 x 100 ml) and brine (70 ml), dried over Na₂SO₄ and evaporated to give a brown oil. The crude product was purified by distillation. All fractions which distilled of at normal pressure up to 60°C were discarded. Vacuum was applied and the fraction distilling off at 45°C was collected. This fraction was further purified by column chromatography.

### Intermediate 21b):

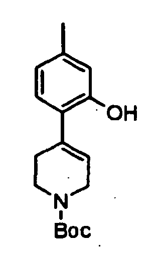

Intermediate 21a) (2.07 g), *N*-Boc-1,2,3,6-tetrahydropyridine-4-boronic acid pinacol ester (3.42 g) and potassium carbonate (4.59 g) were dissolved in DMF (58 ml). The solution was degassed by bubbling with argon for 1 h. Then [Pd(dppf)Cl₂] (542 mg) was added. The brown suspension was then heated under argon in an oil bath at 85°C for 3 d. Another load of catalyst was added (220 mg) and the reaction mixture was stirred at 85°C for 7 h. The reaction mixture was filtered through Celite and rinsed with ethyl acetate. The combined filtrates were evaporated and the residue was partitioned between ethyl acetate (150 ml) and water (150 ml). The mixture was filtered through Celite again and rinsed with ethyl acetate. The phases were separated and the aqueous layer was extracted with ethyl acetate (2 x 60 ml). The combined organic layer was washed with water (60 ml) and brine (60 ml), dried over Na₂SO₄, filtered and evaporated *in vacuo* to dryness. The residue was purified by flash chromatography.

### Intermediate 21c):

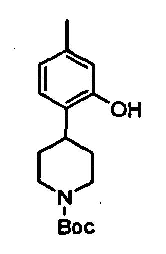

Intermediate 21b) (438 mg) was dissolved in dry ethanol (18 ml) and acetic acid (18 ml) and the solution was degassed by bubbling with argon. Platinum(IV) oxide (120 mg) was added and the reaction mixture was placed under a H₂ atmosphere using a balloon. The reaction mixture was then stirred at room temperatur for 2 h. The reaction mixture was filtered through Celite, rinsed with EtOAc and evaporated to dryness *in vacuo.* The residue was coevaporated with toluene (3x 40 ml) and finally dried under high vacuum overnight to give a yellow oil that started to crystallize on standing.

### Intermediate 21d):

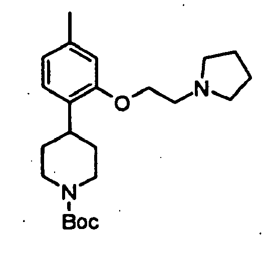

To a solution of intermediate 21c) (256 mg) in DMF (5.0 ml) was added 1-(2-chloroethyl)-pyrrolidine hydrochloride (179 mg) and cesium carbonate (958 mg). The reaction mixture was stirred at room temperature for 36 h. The reaction mixture was evaporated and the residue was partitioned between ethyl acetate (50 ml) and water (40 ml). The organic layer was washed with water (20 ml) and brine (20 ml), dried over Na₂SO₄ and evaporated *in vacuo* to dryness. The crude product was purified by flash chromatography.

### Intermediate 21e):

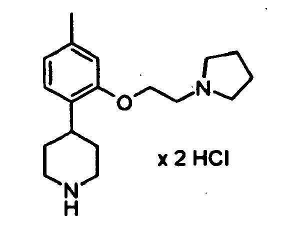

Intermediate 21d) (408 mg) was dissolved in dioxane (2.0 ml) and 4M HCl in dioxane (20 ml) was added at 0°C. The reaction mixture was stirred at room temperature for 90 min. The reaction mixture was evaporated to dryness *in vacuo* and the residue triturated in acetone (1 ml), ethyl acetate (1 ml) and Et₂O (1 ml). A beige sticky compound was obtained. A few drops of MeOH were added to get a beige powder that was filtered off, rinsed with Et₂O and dried.

### Example 21:

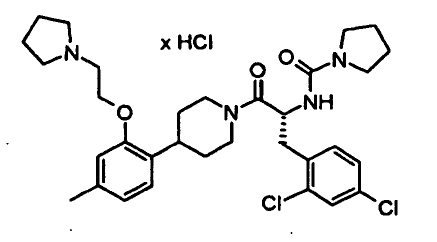

Intermediate 21e) (30 mg), B-C Moiety 1 (35 mg), and HOBt (19 mg) were dissolved in DCM (2.5 ml). NMM (27 µl) was added and the mixture stirred at room temperature for 30 min. EDCI (24 mg) was added, and the reaction stirred at room temperature for another 60 min. An additional amount of NMM (9 µl) was added and stirring continued at room temperature overnight. The reaction mixture was diluted with EtOAc, washed with water, sat. Na₂CO₃ and brine. The organic layer was dried over Na₂SO₄, filtered and evaporated *in vacuo* to dryness. The residue was purified by flash chromatography. The purified product was dissolved in ethyl acetate (200 µl), cooled to 0°C and treated with 1 M HCl in Et₂O (70 µl) and treated with diethyl ether (1 ml). The precipitate was filtered off and dried under vacuum over Sicapent. The product was obtained as an off-white solid.

### Synthesis of Example 23:

### Intermediate 23a):

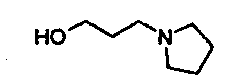

A suspension of pyrrolidine (8.35 ml), 3-bromo-1-propanol (8.67 ml), and potassium carbonate (17.28 g) in MeCN (100 ml) was heated under reflux overnight. The reaction mixture was filtered and evaporated *in vacuo.* The residue was partitioned between EtOAc (100 ml) and 1 M HCl (50 ml). The organic layer was separated and extracted with 1 M HCl (2 x 25 ml). The combined acidic extract was adjusted to pH 13 with solid KOH, while cooling in ice/H₂O. The resulting clear, slightly yellowish solution was extracted with DCM (5 x 50 ml). The combined organic extract was dried over Na₂SO₄ and evaporated *in vacuo.* The crude product was purified by vacuum distillation employing a 5 cm *Vigreux* column at a pressure of ca. 15 mbar (with an oil-bath temperature of ca. 120°C). The fraction distilling off at 89-90°C was collected. The product was obtained as a colorless oil.

### Intermediate 23b):

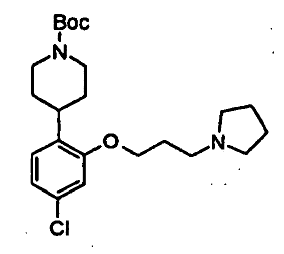

A solution of intermediate 27d) (468 mg), intermediate 23a) (388 mg), and triphenylphosphine (787 mg) in THF (15 ml) under argon, was cooled in ice/H₂O. DEAD (ca. 40% in toluene, 1375 µl) was added dropwise, at a rate to keep the temperature below 5°C (ca. 15 min). After stirring for another 10 min in ice/H₂O, the cooling bath was removed and the mixture was stirred at room temperature overnight. The reaction mixture was evaporated to dryness *in vacuo* at 40°C. The crude product was purified by flash chromatography to yield a clear yellowish oil.

### Intermediate 23c):

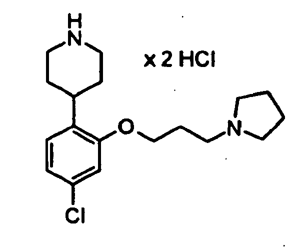

To Boc-protected intermediate 23b) (635 mg) in methanol (3 ml) was added hydrogen chloride, 4.0 M sol. in 1,4-dioxane (11 ml) and the solution was stirred at room temperature for 2 h. The solvent was removed under reduced pressure. The residue was triturated in acetone and diethyl ether, filtered off, and washed with diethyl ether. Finally, it was dried *in vacuo* at room temperature over P₂O₅ overnight to yield a beige solid.

### Example 23:

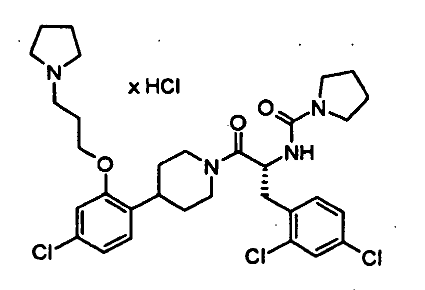

Intermediate 23c) (30 mg), B-C Moiety 1 (31 mg), and HOBt (17 mg) were dissolved in DCM (2.5 ml). NMM (23 µl) was added and the mixture stirred at room temperature for 30 min. EDCl (25 mg) was added, and the reaction stirred at room temperature for another 60 min. An additional amount of NMM (6 µl) was added and stirring continued at room temperature overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated *in vacuo* to dryness. The residue was purified by flash chromatography. The purified product was dissolved in DCM and treated with 1 M HCl in Et₂O (73 µl) and evaporated *in vacuo.* The residue was dissolved in DCM and the salt was precipitated by addition of Et₂O and hexane. The precipitate was filtered off, washed with hexane and Et₂O and dried *in vacuo* at 40°C for 2 hours. The product was obtained as a white solid.

### Synthesis of Example 26:

### Intermediate 26a):

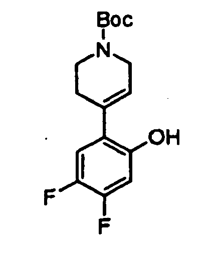

2-Bromo-4,5-difluorophenol (2857 µl), N-Boc-1,2,3,6-tetrahydropyridine-4-boronic acid pinacol ester (7.73 g), potassium carbonate (10.36 g) and dichloro(1,1'-bis(diphenyl-phosphino)-ferrocene)palladium(II) DCM adduct (1.22 g) were dissolved in DMF (150 ml) in a dry apparatus under argon and the mixture was degassed by bubbling with argon for 30 min. The orange suspension was then heated under argon in an oil bath at 85°C for 1 day to give a dark purple suspension. The reaction mixture was filtered through Celite and evaporated to dryness *in vacuo.* The crude product was purified by flash chromatography to yield pale green crystals.

### Intermediate 26b):

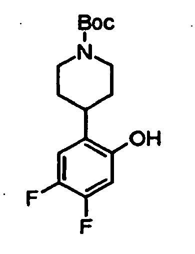

Intermediate 26a) (1404 mg) was dissolved in EtOH (50 ml) and AcOH (50 ml) and platinum(IV) oxide (102 mg) was added. The reaction mixture was evacuated three times and purged with hydrogen. The reaction mixture was then stirred at room temperature for 2 h. The reaction mixture was filtered and evaporated to dryness *in vacuo.* The residue was coevaporated with toluene (3 x 75 ml) and was finally dried under high vacuum at room temperature overnight to yield a beige solid.

### Intermediate 26c):

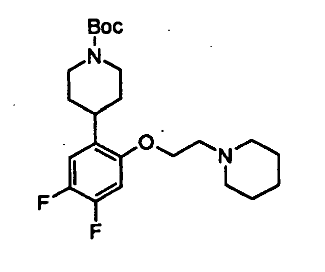

To a solution of intermediate 26b) (674 mg) in DMF (15 ml) was added 1-(2-chloroethyl)piperidine hydrochloride (605 mg) and Cs₂CO₃ (2453 mg). The reaction was stirred at room temperature for 2 days. An additional amount of 1-(2-chloroethyl)piperidine hydrochloride (199 mg) and Cs₂CO₃ (352 mg) was added and stirring at room temperature was continued for another 3 d. The reaction mixture was evaporated at 50°C *in vacuo* to dryness and the residue was partitioned, between Et₂O (75 ml) and water (25 ml). The aqueous layer was extracted, with Et₂O (25 ml). The combined organic layer was washed with water (10 ml) and brine (15 ml). The organic layer was dried over Na₂SO₄ and evaporated *in vacuo* to dryness. The residue was finally dried under high vacuum at room temperature overnight. The crude product was purified by flash chromatography.

### Intermediate 26d):

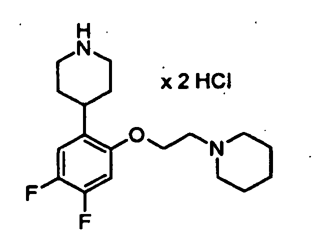

To Boc-protected intermediate 26c) (490 mg) in methanol (2 ml) and dioxane (10 ml) was added hydrogen chloride, 4.0 M sol. in 1,4-dioxane (10 ml) and the solution was stirred at room temperature for 30 min. The solvent was removed under reduced pressure. The residue was triturated in acetone and Et₂O, filtered off, and washed with Et₂O. Finally it was dried *in vacuo* at room temperature over P₂O₅ overnight to yield an off-white solid.

### Example 26:

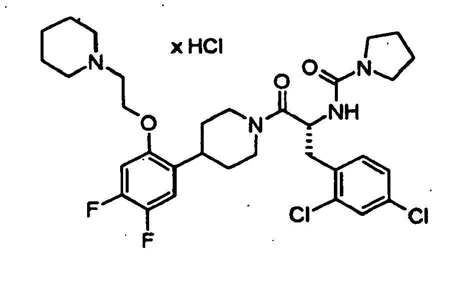

Intermediate 26d) (64 mg), B-C-Moiety 1 (58 mg), and HOBt (27 mg) were dissolved in DCM (2 ml). NMM (26 µl) was added and the mixture stirred at room temperature for 20 min. EDCl (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated *in vacuo.* The crude product was purified by flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried *in vacuo* at room temperature over P₂O₅ overnight. The product was obtained as white solid.

### Synthesis of Example 27:

### Intermediate 27a):

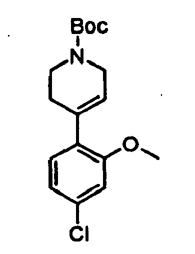

2-Bromo-5-chloroanisole (5.54 g), 1-(2(H)-pyridine-carboxylic acid-3,6-dihydro-4-(4,4,5,5-tetramethyl-1,3,2-dioxaborolan-2-yl)-tert.-butyl ester (7.73 g), dichloro(1,1'-bis(diphenyl-phosphino)-ferrocene)palladium(II) DCM adduct (1.22 g) and K₂CO₃ (10.36 g) were dissolved in degassed DMF in a dry apparatus under argon and the mixture was degassed again by evacuation followed by refilling with argon. The resulting suspension was heated in an oil bath at 85°C overnight. The mixture was cooled, filtered through Celite and evaporated to dryness. The crude product was purified by flash chromatography to yield a clear yellowish oil.

### Intermediate 27b):

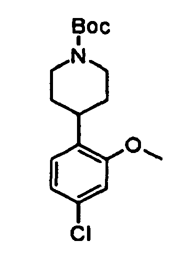

Intermediate 27a) (2.18 g) was dissolved in EtOH (80 ml) and AcOH (80 ml) under argon. Platinum(IV) oxide (0.23 g) was added and the reaction mixture was placed under an H₂ atmosphere using a balloon. The reaction mixture was then stirred at room temperature for 120 min. The reaction mixture was filtered through Celite and evaporated to dryness in *vacuo.* The residue was coevaporated with toluene (3 x 40 ml). The crude product was purified by flash chromatography to yield a clear colorless oil.

### Intermediate 27c):

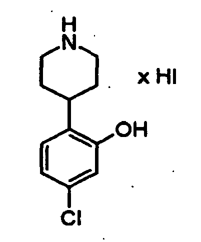

To a solution of intermediate 27b) (1.56 g) in AcOH (6.5 ml) was added hydroiodic acid (5.2 ml of a 57 wt.% aq. solution) and the mixture was heated under reflux (oil bath at 140°C) in an argon atmosphere for 2 h. The reaction mixture was cooled to room temperature and then evaporated to dryness *in vacuo.* The residue was coevaporated with toluene (3 x 30 ml). The crude product was triturated in Et₂O (40 ml), the insoluble compound was filtered off and washed with Et₂O (10 ml). Finally, the product was dried *in vacuo* over P₂O₅ at room temperature overnight to yield a white solid.

### Intermediate 27d):

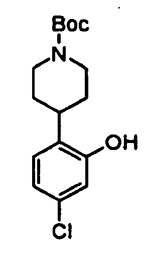

To a solution of intermediate 27c) (1.55 g) in DMF (10 ml) was added DIEA (0.88 ml) followed by di-tert.-butyl-dicarbonate (1.01 g). The reaction mixture was stirred at room temperature for 4 h. The mixture was evaporated *in vacuo* to dryness and partitioned between 0.5 M HCl (50 ml) and EtOAc (100 ml). The organic layer was washed with water (25 ml) and brine (30 ml). The organic layer was dried with MgSO₄ and evaporated *in vacuo* to dryness to yield a yellowish solid. The solid residue was triturated in EtOAc (1 ml) and Et₂O (10 ml), and left in the fridge overnight to complete crystallization of the product. The precipitate was then filtered off, washed with cold Et₂O (1 ml), and finally dried *in vacuo* at room temperature over P₂O₅ overnight. The product was obtained in form of a white solid.

### Intermediate 27e):

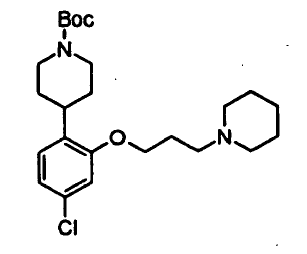

To a solution of intermediate 27d) (468 mg) in DMF (8 ml) was added 1-(3-chloropropyl)piperidine hydrochloride (373 mg) and Cs₂CO₃ (1710 mg). The reaction was stirred at room temperature for 18 h. An additional amount of 1-(3-chloropropyl)piperidine hydrochloride (297 mg) and Cs₂CO₃ (489 mg) were added and stirring at room temperature was continued for 3 d. The reaction mixture was evaporated at 40°C *in vacuo* to dryness and the residue was partitioned between EtOAc and water. The aqueous layer was extracted with EtOAc. The combined organic layer was washed with water and brine. The organic layer was dried over Na₂SO₄ and evaporated *in vacuo* to dryness. The crude product was purified using flash chromatography.

### Intermediate 27f):

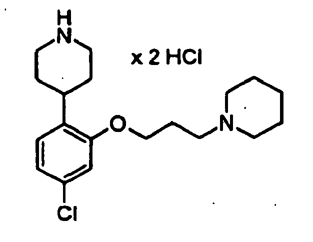

To Boc-protected intermediate 27e) (651 mg) in methanol (3 ml) was added hydrogen chloride, 4.0 M sol. in 1,4-dioxane (11 ml) and the solution was stirred at room temperature for 90 min. The solvent was removed under reduced pressure. The residue was triturated in acetone and Et₂O, filtered off, and washed with Et₂O. Finally, it was dried *in vacuo* at room temperature over P₂O₅ overnight to yield a white solid.

### Example 27:

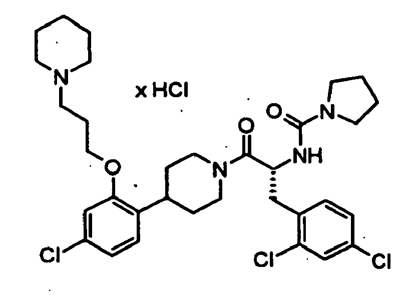

Intermediate 27f) (30 mg), B-C Moiety 1 (30 mg), and HOBt (17 mg) were dissolved in DCM (2 ml). NMM (22 µl) was added and the mixture stirred at room temperature for 30 min. EDCl (25 mg) was added, and the reaction stirred at room temperature for another 60 min. An additional amount of NMM (6 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated *in vacuo.* The crude product was purified by flash chromatography. The purified product was dissolved in DCM, treated with 1 M HCl in Et₂O (66 µl), and the resulting suspension was diluted with diethyl ether and hexane. The precipitate was filtered off, washed with hexane and diethyl ether, and dried *in vacuo* at room temperature over P₂O₅ overnight. The product was obtained as a white solid.

### Synthesis of Example 36:

### Intermediate 36a):

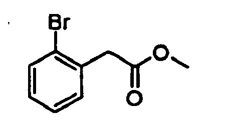

(2-Bromo-phenyl)-acetic acid (5.38 g) was dissolved in methanol (20.26 ml). Then concentrated sulfuric acid (0.27 ml) was added, and the reaction mixture was heated under reflux overnight (oil bath temperature 85°C) with exclusion of humidity by means of a drying tube (blue silica gel). The reaction mixture was evaporated *in vacuo* at 40°C and the colorless oily residue was poured into ice-water (50 ml). The resulting white emulsion was extracted with Et₂O (75 ml), and the organic phase was washed with sat. Na₂CO₃ (3 x 20 ml), H₂O (15 ml), and brine (15 ml). The organic phase was dried with MgSO₄ and evaporated *in vacuo* to yield a colorless clear oil.

### Intermediate 36b):

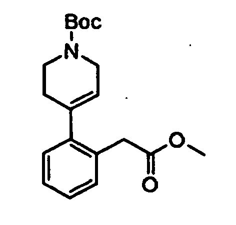

Intermediate 36a) (4.00 g), N-Boc-1,2,3,6-tetrahydropyridine-4-boronic acid pinacol ester (5.40 g), potassium carbonate (7.24 g), and dichloro(1,1'-bis(diphenyl-phosphino)-ferrocene)palladium(II) DCM adduct (860 mg) were dissolved in DMF (150 ml) in a dry apparatus under argon and the mixture was degassed by bubbling with argon for 30 min. The orange suspension was then heated under argon in an oil bath at 85°C overnight. The reaction mixture was filtered through Celite and evaporated to dryness *in vacuo.* The residue was triturated in DCM (50 ml) and the insoluble parts were filtered off. The filtrate was concentrated and subjected to flash chromatography. The product was obtained as clear yellow oil.

### Intermediate 36c):

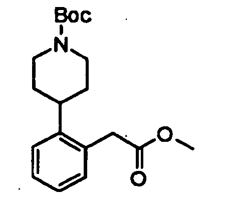

Intermediate 36b) (1.99 g) was dissolved in EtOH (30 ml) and AcOH (30 ml) and platinum(IV) oxide (400 mg) was added. The reaction mixture was evacuated three times and purged with hydrogen. The reaction mixture was then stirred at room temperature for 2 h. The reaction mixture was filtered and evaporated to dryness *in vacuo.* The residue was coevaporated with toluene (3 x 75 ml) and was finally dried under high vacuum at room temperature overnight.

### Intermediate 36d):

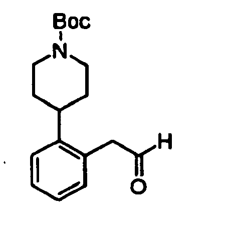

Intermediate 36c) (2.85 g) was dissolved in dry diethyl ether (30 ml) under inert atmosphere and cooled to -72°C. At this temperature diisobutylaluminum hydride, 1.0 M in hexane (12.8 ml) was added dropwise in the course of 30 min. The reaction mixture was stirred at -72°C for 2 h. Methanol (173 µl) was added and the mixture was warmed up to 0°C. Water (1.5 ml) was added and the mixture was filtered through a bed of sodium sulfate. After washing twice with diethyl ether (30 ml each) the combined organic filtrate was concentrated *in vacuo.* The crude product was purified by flash chromatography to yield a colorless oil.

### Intermediate 36e):

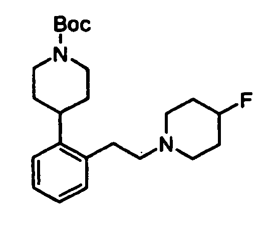

To a solution of the intermediate 36d) (121 mg) and 4-fluoropiperidine hydrochloride (56 mg) in dichloroethane (5 ml), DIEA (139 µl) was added followed by sodium triacetoxyborohydride (119 mg). The reaction mixture was then stirred for 4 h at room temperature. The mixture was diluted with EtOAc (70 ml) and washed two times with sat. NaHCO₃ (25 ml each), water and brine (25 ml each). The organic phase was dried over Na₂SO₄ and concentrated. The crude product was purified using flash chromatography.

### Intermediate 36f):

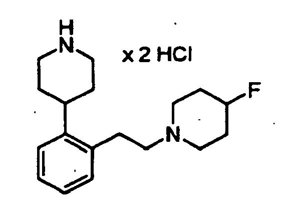

To intermediate 36e) (102 mg) in dioxane (5 ml) and methanol (1 ml) was added hydrogen chloride, 4.0 M sol. in 1,4-dioxane (5 ml) and the solution was stirred for 30 min at room temperature. The solvent was removed under reduced pressure, the residue was triturated with acetone (5 ml) and diethyl ether (25 ml) and the product was filtered off.

### Example 36:

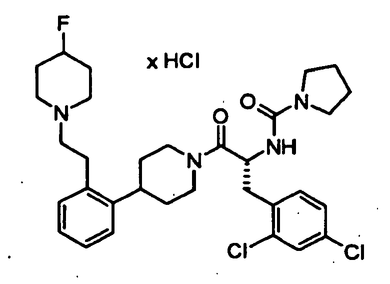

Intermediate 36f) (29 mg), B-C Moiety 1 (28 mg), and HOBt (14 mg) were dissolved in DCM (1 ml). NMM (13 µl) was added and the mixture stirred at room temperature for 20 min. EDCl (23 mg) was added, and the reaction stirred at room temperature for another 60 min. An additional amount of NMM (10 µl) was added and stirring continued at room temperature overnight. The reaction mixture was poured into water (20 ml), diluted with ethyl acetate and the organic phase was separated. The aqueous phase was extracted two times with ethyl acetate. The combined organic phase was washed three times with saturated sodium bicarbonate solution, dried over Na₂SO₄ and concentrated. The residue was purified by flash chromatography. The purified product was dissolved in ethyl acetate and treated with 1 M HCl in Et₂O (100 µl). The product was precipitated by addition of hexane (20 ml). The precipitate was filtered off and dried *in vacuo* over P₂O₅. The product was obtained as white solid.

### Synthesis of Example 37:

### Intermediate 37a):

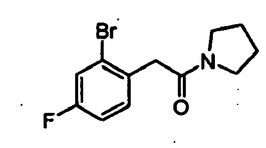

2-Bromo-4-fluorophenylacetic acid (2330 mg), EDCl (2109 mg) and DMAP (100 mg) were dissolved in DCM (100 ml). Pyrrolidine (918 µl) was added and the reaction mixture was stirred overnight. The reaction mixture was poured into water (100 ml) and the organic layer was separated. The aqueous layer was extracted twice with DCM. The combined organics were washed three times with 0.5 N HCl (30 ml each), three times with 1 M sodium hydroxide solution and brine, dried over Na₂SO₄ and the solvent was removed under reduced pressure. The crude product was purified by flash chromatography.

### Intermediate 37b):

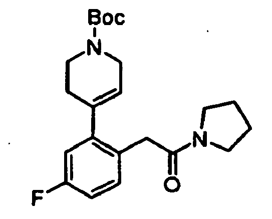

Intermediate 37a) (1692 mg), N-Boc-1,2,3,6-tetrahydropyridine-4-boronic acid pinacol ester (1920 mg), potassium carbonate (2450 mg), and dichloro(1,1'-bis(diphenyl-phosphino)-ferrocene)palladium(II) DCM adduct (286 mg) were dissolved in DMF (70 ml) in a dry apparatus under argon and the mixture was degassed by bubbling with argon for 30 min. The orange suspension was then heated under Argon in an oil bath at 85°C overnight. The reaction mixture was filtered through Celite and evaporated to dryness *in vacuo.* The residue was triturated in DCM (50 ml) and the insoluble parts were filtered off. The filtrate was concentrated and subjected to flash chromatography.

### Intermediate 37c):

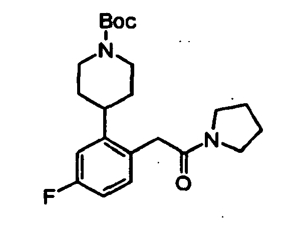

Intermediate 37b) (2266 mg) was dissolved in EtOH (50 ml) and AcOH (50 ml) and platinum(IV) oxide (132 mg) was added. The reaction mixture was evacuated three times and purged with hydrogen. The reaction mixture was then stirred at room temperature for 2 h. The reaction mixture was filtered and evaporated to dryness *in vacuo.* The residue was coevaporated with toluene (3 x 75 ml) and was finally dried under high vacuum at room temperature overnight. The crude product was purified by flash chromatography to yield a white solid.

### Intermediate 37d):

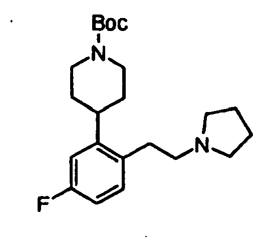

Intermediate 37c) (1392 mg) in diethyl ether (20 ml) was slowly added to a mixture of lithium aluminum hydride (203 mg) and diethyl ether (30 ml) at 0°C. After addition the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered; and the solvent was removed under reduced pressure. The product was purified by flash chromatography.

### Intermediate 37e):

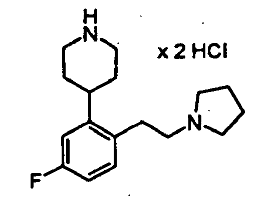

To intermediate 37d) (373 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (10 ml) and diethyl ether (50 ml) and the product was filtered off.

### Example 37:

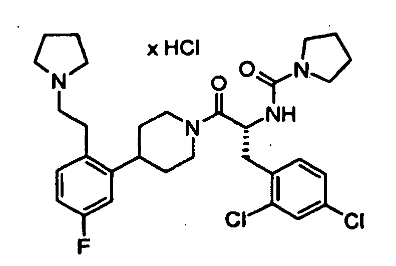

Intermediate 37e) (28 mg), B-C Moiety 1 (28 mg), and HOBt (14 mg) were dissolved in DCM (1 ml). NMM (13 µl) was added and the mixture stirred at room temperature for 20 min. EDCl (23 mg) was added, and the reaction stirred at room temperature for another 60 min. An additional amount of NMM (10 µl) was added and stirring continued at room temperature overnight. The reaction mixture was poured into water (20 ml), diluted with ethyl acetate and the organic phase was separated. The aqueous phase was extracted two times with ethyl acetate. The combined organic phase was washed three times with saturated sodium bicarbonate solution, dried over Na₂SO₄ and concentrated. The residue was purified by flash chromatography. The purified product was dissolved in ethyl acetate and treated with 1 M HCl in Et₂O (100 µl). The product was precipitated by addition of hexane (20 ml). The precipitate was filtered off and dried *in vacuo* over P₂O₅. The product was obtained as a white solid.

### Synthesis of Example 42:

### Intermediate 42a):

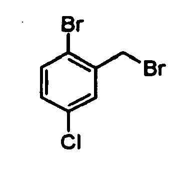

2-Bromo-5-chlorotoluene (8.26 ml) and N-bromosuccinimide (11.03 g) in carbon-tetrachloride (50 ml) were treated with a catalytic amount of benzoylperoxide (100 mg) and heated under reflux until the reaction had reached completion as monitored by TLC. The reaction mixture was then allowed to cool and filtered. The filtrate was washed twice with water and brine, dried over sodium sulfate and concentrated *in vacuo.*

### Intermediate 42b):

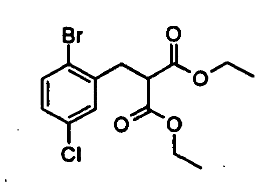

To a solution of sodium ethoxide (2.79 g) in ethanol (30 ml) was added diethyl malonate (6.54 ml) and the mixture was stirred for 1 h at room temperature. The mixture was cooled in an ice-bath and intermediate 42a) (11.67 g) was slowly added and the reaction mixture was kept under reflux overnight. The reaction mixture was evaporated *in vacuo* and the residue was partioned between diethyl ether and water and the aqueous layer extracted two times with diethyl ether. The combined organic layer was washed twice with water and brine. The combined organic layer was dried over Na₂SO₄ and evaporated *in vacuo* to dryness. The product was purified by Kugelrohr distillation. The fractions which distilled off between 160 and 230°C at 0.2-0.3 mbar were collected.

### Intermediate 42c):

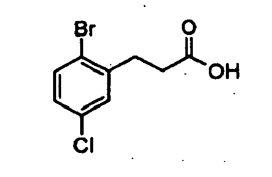

Intermediate 42b) (8.98 g) was heated under reflux in 1.8 M KOH in H₂O/EtOH (60 ml) for 5 h. After evaporation of the ethanol an additional amount of KOH (18 g) was added to the residue, and the reaction mixture was stirred for 2 h at 100°C. The reaction mixture was diluted with 100 ml of H₂O, extracted with Et₂O and the organic layer was washed with H₂O. The combined aqueous layer was cooled with ice/H₂O and acidified with 50 % H₂SO₄ to pH 1. The precipitate was extracted twice with Et₂O (100 ml each) and the organic layers were washed with water and brine. The combined organic layers were dried over Na₂SO₄ and evaporated *in vacuo* to dryness. The residue was triturated in hexane and less Et₂O, then filtered and washed with hexane and less Et₂O. The solid residue was decarboxylated by heating at 200°C. The development of CO₂ ceased after 20 min and the melt was cooled to room temperature. The residue was crushed with a glass rod to get a homogeneous beige solid.

### Intermediate 42d):

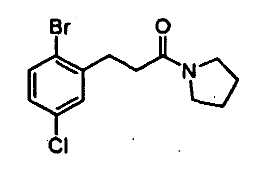

Intermediate 42c) (2320 mg), pyrrolidine (808 µl), EDCI (1856 mg) and DMAP (100 mg) were dissolved in DCM (100 ml) and stirred overnight. The reaction mixture was poured into water (100 ml) and the organic layer was separated. The aqueous layer was extracted twice with DCM. The combined organics were washed three times with with 0.5 N HCl (30 ml each), three times with 1 M sodium hydroxide solution and brine, dried over Na₂SO₄ and the solvent was removed under reduced pressure. The product was obtained as off-white solid after purification by flash chromatography.

### Intermediate 42e):

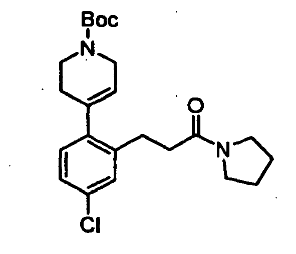

Intermediate 42d) (1901 mg), N-Boc-1,2,3,6-tetrahydropyridine-4-boronic acid pinacol ester (1948 mg), potassium carbonate (3317 mg) and dichloro(1,1'-bis(diphenyl-phosphino)-ferrocene)palladium(II) DCM adduct (294 mg) were dissolved in DMF (70 ml) in a dry apparatus under argon and the mixture was degassed by bubbling with argon for 30 min. The orange suspension was then heated under argon in an oil bath at 85°C for 3 days to give a dark purple suspension. The reaction mixture was filtered through Celite and evaporated to dryness *in vacuo.* The crude product was purified by column chromatography.

### Intermediate 42f):

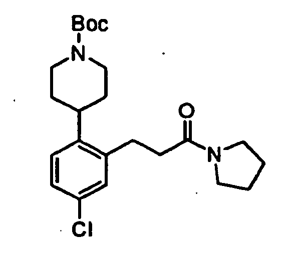

Intermediate 42e) (2372 mg) was dissolved in EtOH (50 ml) and AcOH (50 ml) and platinum(IV) oxide (129 mg) was added. The reaction mixture was evacuated three times and purged with hydrogen. The reaction mixture was then stirred at room temperature for 2 h. The reaction mixture was filtered and evaporated to dryness *in vacuo.* The residue was coevaporated with toluene (3 x 75 ml) and was finally dried under high vacuum at room temperature overnight.

### Intermediate 42g):

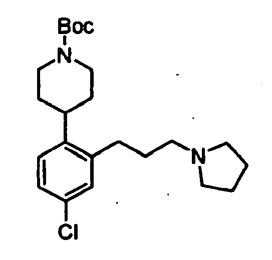

Intermediate 42f) (1687 mg) in diethyl ether (20 ml) was slowly added to a mixture of lithium aluminum hydride (228 mg) and diethyl ether (30 ml) at 0°C. After addition, the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a colorless oil.

### Intermediate 42h):

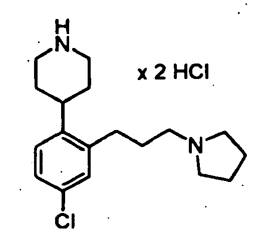

To intermediate 42g) (864 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off. The product was obtained as off-white solid.

### Example 42:

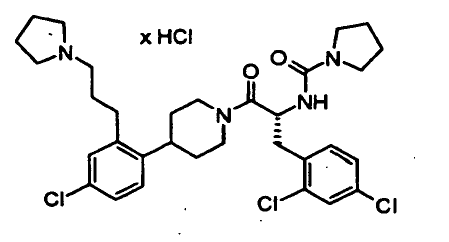

Intermediate 42h) (61 mg), B-C Moiety 1 (58 mg), and HOBt (27 mg) were dissolved in DCM (2 ml). NMM (26 µl) was added and the mixture stirred at room temperature for 20 min. EDCl (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated *in vacuo.* The crude product was purified by flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried *in vacuo* at room temperature over P₂O₅ overnight. The product was obtained as a white solid.

### Synthesis of Example 44:

### Intermediate 44a):

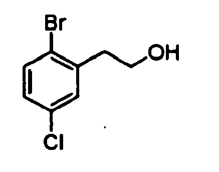

A solution of 2-bromo-5-chlorophenylacetic acid (20.0 g) in THF (200 ml) was added to a suspension of sodium borohydride (3.18 g) in THF (50 ml) over 30 min at room temperature. After being stirred for 15 min, boron trifluoride diethyl etherate (13.2 ml) was added over 30 min while the temperature was maintained at 25-35°C. The resulting slurry was stirred for 1 h at room temperature, then cooled to 0°C and carefully hydrolyzed by addition of sat. NH₄Cl (50 ml). The main part of the THF was removed *in vacuo,* the residue was diluted with sat. NH₄Cl and water (each with 50 ml), followed by extraction with diethyl ether (3 x 100 ml). The combined ether layer was washed with 1 M NaOH (2 x 100 ml) and water (100 ml). All aqueous phases were combined and extracted with diethyl ether (2 x 100 ml). All organic layers were combined, washed with brine (100 ml), and dried (Na₂SO₄). Evaporation of the solvent afforded the desired product as a yellowish oil.

### Intermediate 44b):

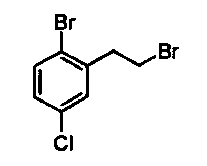

Phosphorous tribromide (3.71 ml) was dissolved in toluene (30 ml) and cooled to 0°C. Then pyridine (1.68 ml) was added: To the suspension thus obtained, a solution of intermediate 44a) (18.6 g) and pyridine (0.56 ml) in toluene (30 ml) was added over 15 min. The cooling bath was removed and stirring was continued at room temperature for 1 h. Then the reaction mixture was heated to 100°C for another hour. The reaction mixture was cooled to ambient temperature, diluted with EtOAc (300 ml) and washed with water (2 x 100 ml). The combined aqueous layer was extracted with EtOAc (100 ml), all organic extracts were merged and washed with brine (100 ml), dried (Na₂SO₄), and concentrated *in vacuo.* The crude product was purified by column chromatography to furnish the desired compound as a colorless oil.

### Intermediate 44c):

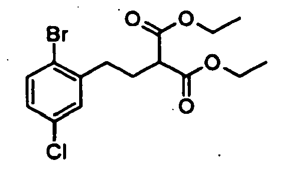

Diethyl malonate (9.59 ml) was added dropwise to a suspension of sodium hydride, 60% dispersion in mineral oil (2.42 g) in THF (50 ml) at 0°C. The cooling bath was removed and stirring was continued for 30 min at room temperature. Then a solution of intermediate 44b) (15.7 g) in THF (50 ml) was added and the reaction mixture was kept under reflux overnight. The suspension was concentrated *in vacuo.* The residue was diluted with water (300 ml) and extracted with diethyl ether (3 x 200 ml). The combined organic layer was washed with brine (200 ml), dried (Na₂SO₄), and concentrated *in vacuo.* The crude product was purified by column chromatography to furnish the desired compound as a colorless oil.

### Intermediate 44d):

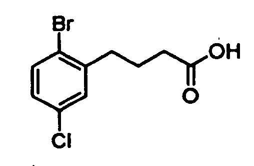

An emulsion of intermediate 44c) and potassium hydroxide (11.6 g) in a 1:1 mixture of EtOH and water (100 ml) was heated under reflux for 5 h. The main part of EtOH was evaporated, more potassium hydroxide (19.3 g) was added and the reaction mixture was heated to 100°C for 45 min. After dilution with water (150 ml), the solution was extracted with diethyl ether (2 x 50 ml). The combined ether layer was re-extracted with water (50 ml). The two water layers were combined and acidified with 50% H₂SO₄ to pH 1, extracted with diethyl ether (3 x 100 ml), re-extracted with water (100 ml). Following drying over Na₂SO₄ the solvent was evaporated to afford the corresponding malonic acid derivative in form of a white solid. Decarboxylation was achieved by heating the product at 200°C until evolution of carbon dioxide ceased to provide the desired compound as a slightly brownish solid.

### Intermediate 44e):

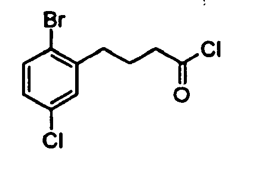

A solution of intermediate 44d) (5.03 g) in DCM (130 ml) was cooled to 0°C. To this solution a solution of oxalyl chloride (1.69 ml) in DCM (20 ml) was added dropwise. DMF (5 drops) was added and the reaction mixture was stirred at 0°C for 1 h and then at room temperature until the gas formation ceased. Concentration *in vacuo* furnished the desired product in form of a yellowish oil.

### Intermediate 44):

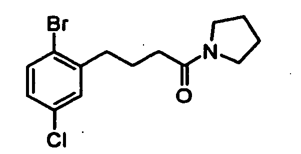

A solution of intermediate 44e) (5.31 g) in DCM (70 ml) was cooled to 0°C. Pyrrolidine (4.49 ml) was added dropwise and stirring was continued at 0°C for 45 min. The ice bath was removed and stirring was continued at room temperature. After being stirred for 2 h in total the reaction mixture was diluted with DCM (100 ml) and washed with 1 M HCl, 1 M NaOH (each with 3 x 50 ml), water and brine (each with 50 ml). The organic phase was dried (Na₂SO₄) and evaporated to afford the desired product as a yellow oil.

### Intermediate 44g):

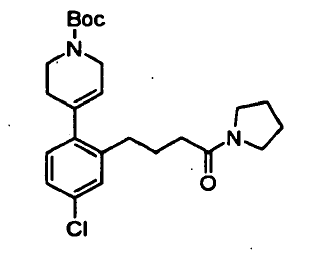

A mixture of intermediate 44f) (5.50 g), *N*-Boc-1,2,3,6-tetrahydropyridine-4-boronic acid pinacol ester (5.40 g), potassium carbonate (6.90 g), and dichloro(1,1'-bis(diphenyl-phosphino)-ferrocene)palladium(II) DCM adduct (0.82 g) in degassed DMF (100 ml) was heated under argon at 85°C overnight. The reaction mixture was evaporated. The residue was partitioned between EtOAc and water (each with 100 ml) and filtered through Celite. The filtrate was extracted with EtOAc (2 x 100 ml) and the combined organic layer re-extracted with water and brine (100 ml each), dried (Na₂SO₄) and concentrated *in vacuo.* The crude product was purified by column chromatography to furnish the desired compound as a brownish resin.

### Intermediate 44h):

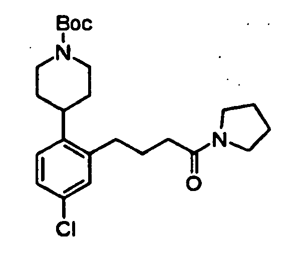

To a solution of intermediate 44g) (6.06 g) in EtOH and AcOH (100 ml each) platinum(IV) oxide (0.32 g) was added. The flask was purged with H₂ at atmospheric pressure. The reaction mixture was then vigorously stirred for 3 h at room temperature. Filtration through Celite, evaporation of the solvent and purification of the residue by column chromatography afforded the desired product in form of a colorless oil.

### Intermediate 44i):

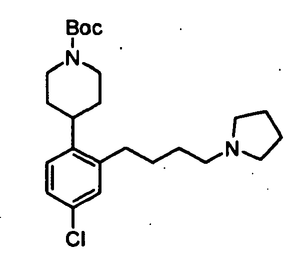

Under vigorous stirring a solution of intermediate 44h) (2.16 g) in diethyl ether (20 ml) was added to a suspension of lithium aluminum hydride (0.28 g) in diethyl ether (30 ml) at 0°C under argon. The reaction mixture was stirred for 1 h at this temperature and was then hydrolyzed by addition of water (0.5 ml). The inorganic precipitate thus obtained was filtered off and washed with diethyl ether (3 x 40 ml). The filtrate was dried (Na₂SO₄) and concentrated *in vacuo.* Purification of the crude product by column chromatography furnished the desired compound as a colorless resin.

### Intermediate 44j):

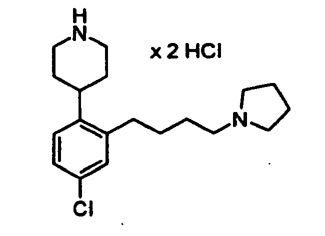

To Boc-protected intermediate 44i) (670 mg) in MeOH (5 ml) was added hydrogen chloride, 4.0 M sol. in 1,4-dioxane (5 ml) and the solution was stirred at room temperature for 30 min. The solvent was completely removed *in vacuo* to afford the desired compound as a white solid.

### Example 44

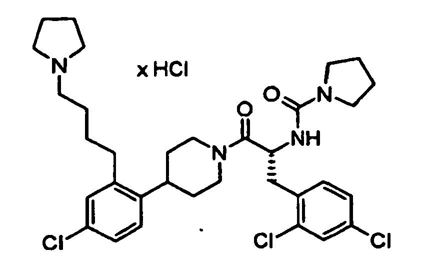

Intermediate 44j) (65 mg) and B-C Moiety 1 (71 mg), 1-hydroxy-benzotriazole hydrate (38 mg) and N-methylmorpholine (51 µl) were dissolved in DMF (5 ml). After being stirred for 30 min at room temperature, *N*-(3-dimethylaminopropyl)-*N'*-ethylcarbodiimide hydochloride (57 mg) was added and stirring was continued for another hour. An additional amount of N-methylmorpholine (14 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed in *vacuo.* Purification of the crude product by column chromatography furnished the corresponding amine in form of a yellowish oil. This was dissolved in EtOAc (1 ml) and treated with hydrogen chloride solution, 1.0 M in diethyl ether (235 µl). The resulting suspension was diluted with ether and hexane (3 ml each) in order to obtain a complete precipitation of the corresponding hydrochloride. The solid was filtered off, washed with hexane, and dried *in vacuo* over P₂O₅ overnight to provide the desired compound in form of an off-white solid.

### Synthesis of Examples 49 and 50:

### Intermediate 49-50a):

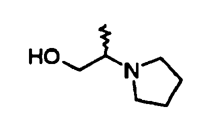

To a solution of DL-2-amino-1-propanol (5.34 ml) and 1,4-dibromobutane (7.91 ml) in acetonitrile (67 ml) was added potassium carbonate (18.52 g) and the resulting solution was stirred at reflux temperature for 20 h. The reaction mixture was evaporated *in vacuo* and the residue was partioned between EtOAc and water. The organic layer was washed with water and brine. The aqueous layers were re-extracted with EtOAc. The combined organic layer was dried over Na₂SO₄ and evaporated *in vacuo* to dryness. The crude product was purified by Kugelrohr distillation (20 mbar, 103-112°C) to yield a clear colorless oil.

### Intermediate 49-50b):

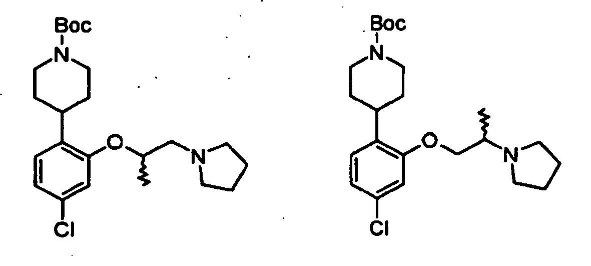

A solution of intermediate 27d) (1.21 g), intermediate 49-50a) (1.00 g), and triphenylphosphine (2.03 g) in THF (30 ml) under argon, was cooled in ice/H₂O. DEAD (ca. 40% in toluene, 1.42 ml) was added dropwise, at a rate to keep the temperature below 5°C (ca. 15 min). After stirring for another 10 min in ice/H₂O, the cooling bath was removed and the mixture was stirred at room temperature overnight. The reaction mixture was evaporated to dryness *in vacuo* at 40°C. The crude product was purified by flash chromatography.

### Intermediate 49-50c):

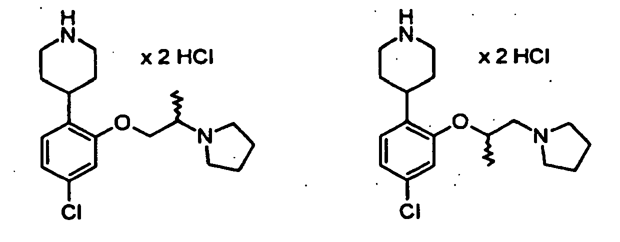

To Boc-protected intermediate 49-50b) (1.25 g) in dioxane (5 ml) was added hydrogen chloride, 4.0 M sol. in 1,4-dioxane (20 ml) and the solution was stirred at room temperature for 60 min. The solvent was removed under reduced pressure. The residue was triturated in acetone and Et₂O, filtered off, and washed with Et₂O. Finally, it was dried *in vacuo* at room temperature over P₂O₅ overnight to yield a white solid.

### Examples 49 and 50:

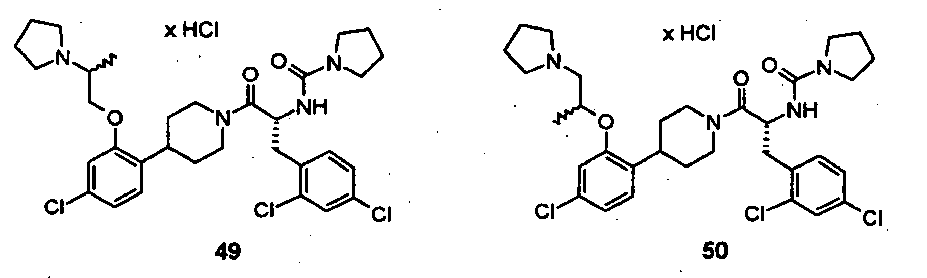

Intermediate 49-50c) (100 mg), B-C Moiety 1 (105 mg), and HOBt (58 mg) were dissolved in DCM (7 ml). NMM (77 µl) was added and the mixture stirred at room temperature for 30 min. EDCl (85 mg) was added, and the reaction stirred at room temperature for another 60 min. An additional amount of NMM (21 µl) was added and stirring continued at room temperature overnight. The reaction mixture was evaporated *in vacuo,* diluted with EtOAc and washed with sat. Na₂CO₃ water and brine. The organic layer was dried over Na₂SO₄ and evaporated *in vacuo.* The two products were separated by flash chromatography. The free base of example 49 was dissolved in DCM, treated with 1 M HCl in Et₂O (172 µl), and evaporated *in vacuo.* The residue was dissolved in DCM and the salt precipitated by addition of diethyl ether and hexane. The precipitate was filtered off, washed with hexane and diethyl ether, and dried *in vacuo* at room temperature for 2 h. The product was obtained as a white solid.

The free base of example 50 was dissolved in DCM, treated with 1 M HCl in Et₂O (44 µl), and evaporated *in vacuo.* The residue was dissolved in DCM and the salt precipitated by addition of diethyl ether and hexane. The precipitate was filtered off, washed with hexane and diethyl ether, and dried *in vacuo* at room temperature for 2 h. The product was obtained as a white solid.

### Synthesis of Example 57:

### Intermediate 57a):

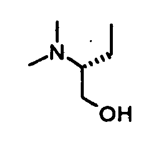

A mixture of (*R*)-(-)-2-amino-1-butanol (4.24 ml), formaldehyde solution (36.5% in H₂O, 10.87 ml) and formic acid (6.79 ml) in water (34.06 ml) was heated under reflux for 24 h. The reaction mixture was concentrated *in vacuo,* the residue was diluted with water (80 ml) and made alkaline by addition of NaOH 1 N (pH 14). The aqueous solution was extracted with CH₂Cl₂ (3 x 60 ml) and the organic layers were washed with water (25 ml) and brine (25 ml). The combined organic layer was dried over Na₂SO₄ and evaporated *in vacuo* at 40°C. The crude product was purified by Kugelrohr distillation (atmospheric pressure / 155 - 250°C) to yield a clear colorless oil.

### Intermediate 57b):

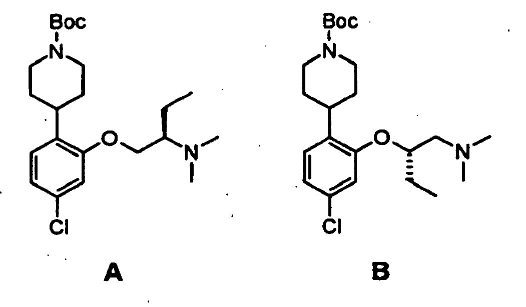

A solution of intermediate 27d) (1.00 g), intermediate 57a) (0.75 g), and triphenylphosphine (1.68 g) in THF (30 ml) under argon, was cooled in ice/H₂O. DEAD (ca. 40% in toluene, 2.94 ml) was added dropwise, at a rate to keep the temperature below 5°C (ca. 15 min). After stirring for another 10 min in ice/H₂O, the cooling bath was removed and the mixture was stirred at room temperature overnight. The reaction mixture was evaporated to dryness *in vacuo* at 40°C. The two regioisomeric products A and B were separated by flash chromatography.

### Intermediate 57c):

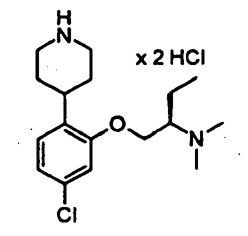

To Boc-protected intermediate 57b) (product A) (825 mg) in dioxane (2.5 ml) was added hydrogen chloride, 4.0 M sol. in 1,4-dioxane (11 ml) and the solution was stirred at room temperature for 120 min. The solvent was removed under reduced pressure. The residue was triturated in a mixture of acetone, methanol and Et₂O, filtered off, and washed with Et₂O. Finally it was dried *in vacuo* at room temperature over P₂O₅ overnight to yield a white solid.

### Example 57:

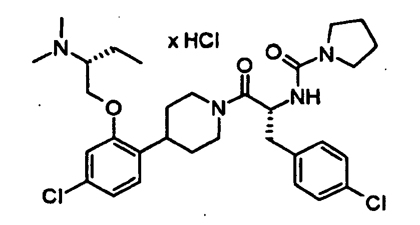

Intermediate 57c) (40 mg), B-C Moiety 1 (43 mg), and HOBt (24 mg) were dissolved in DCM (3 ml). NMM (31 µl) was added and the mixture stirred at room temperature for 30 min. EDCl (55 mg) was added, and the reaction stirred at room temperature for another 60 min. An additional amount of NMM (9 µl) was added and stirring continued at room temperature overnight. The reaction mixture was evaporated *in vacuo,* diluted with EtOAc and washed with sat. Na₂CO₃, water and brine. The organic layer was dried over Na₂SO₄ and evaporated *in vacuo.* The crude product was purifed by flash chromatography. The purified product was dissolved in DCM, treated with 1 M HCl in Et₂O (106 µl), and the resulting suspension was diluted with diethyl ether and hexane. The precipitate was filtered off, washed with hexane and diethyl ether, and dried *in vacuo* at 40°C for 2 h. The product was obtained as a white solid.

### Synthesis of Example 58:

### Intermediate 58a):

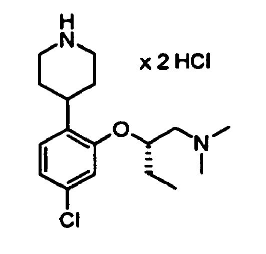

To Boc-protected intermediate 57b) (product B) (251 mg) in dioxane (0.75 ml) was added hydrogen chloride, 4.0 M sol. in 1,4-dioxane (3.5 ml) and the solution was stirred at room temperature for 120 min. The reaction mixture was diluted with Et₂O, the precipitated salt was filtered off, and washed with Et₂O. Finally it was dried *in vacuo* at room temperature over P₂O₅ overnight to yield a white solid.

### Example 58:

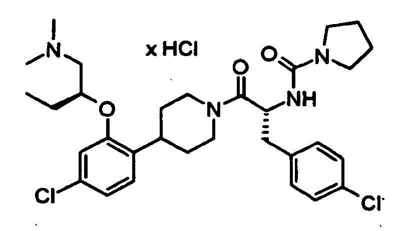

Intermediate 58a) (40 mg), B-C Moiety 1 (43 mg), and HOBt (24 mg) were dissolved in DCM (3 ml). NMM (31 µl) was added and the mixture stirred at room temperature for 30 min. EDCl (55 mg) was added, and the reaction stirred at room temperature for another 60 min. An additional amount of NMM (9 µl) was added and stirring continued at room temperature overnight. The reaction mixture was evaporated *in vacuo,* diluted with EtOAc and washed with sat. Na₂CO₃, water and brine. The organic layer was dried over Na₂SO₄ and evaporated *in vacuo.* The crude product was purified by flash chromatography. The purified product was dissolved in DCM, treated with 1 M HCl in Et₂O (96 µl), and the resulting suspension was diluted with diethyl ether and hexane. The precipitate was filtered off, washed with hexane and diethyl ether, and dried *in vacuo* at 40°C for 2 h. The product was obtained as a white solid.

### Synthesis of Example 59:

### Intermediate 59a):

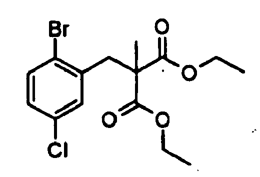

To a solution of sodium ethoxide (1.26 g) in ethanol (30 ml) was added diethyl methylmalonate (3.31 ml) followed by intermediate 42a) (5.26 g) and the reaction mixture was kept under reflux overnight. The reaction mixture was evaporated *in vacuo* and the residue was partitioned between diethyl ether and water. The aqueous layer was extracted two times with diethyl ether. The combined organic layer was washed twice with water and brine. The organic layer was dried over Na₂SO₄ and evaporated *in vacuo* to dryness. The product was purified by distillation. The fractions which distilled off between 140 and 220°C at 0.2 mbar were collected.

### Intermediate 59b):

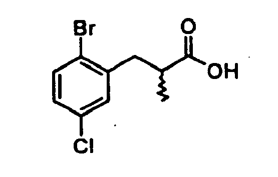

Intermediate 59a) (5.89 g) was heated under reflux in 1.8 M KOH in H₂O/EtOH (60 ml) for 5 h. After evaporation of the ethanol, an additional amount of KOH (18 g) was added to the residue, and the reaction mixture was stirred at 100°C for 2 h. The reaction mixture was diluted with 100 ml of H₂O, extracted with Et₂O, and the organic layer was washed with H₂O. The combined aqueous layer was cooled in ice/H₂O and acidified with 50% H₂SO₄ to pH 1. The resulting suspension was extracted twice with Et₂O (100 ml each) and the organic layers were washed with water and brine. The combined organic layer was dried over Na₂SO₄ and evaporated *in vacuo* to dryness. The residue was triturated in hexane and less Et₂O, then filtered and washed with hexane and less Et₂O. The solid residue was decarboxylated by heating at 200°C. The evolution of CO₂ ceased after 20 min and the product was left to cool to room temperature to yield a brown oil.

### Intermediate 59c):

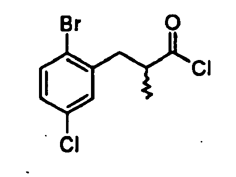

Intermediate 59b) (3.08 g mmol) was dissolved in dry DCM (85 ml) and cooled to 0°C in an ice-water bath. Oxalyl chloride (1.03 ml) in DCM (15 ml) was added dropwise followed by the addition of 1-2 drops of DMF. This mixture was stirred at 0°C for 1.5 h and at room temperature for 2 h (no more gas evolution, clear solution obtained) and then concentrated. The product was dried under reduced pressure.

### Intermediate 59d):

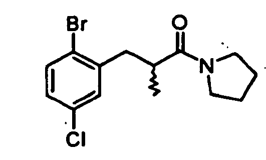

To a solution of intermediate 59c) (3.24 g) in DCM (70 ml) at 0°C was added pyrrolidine (2.73 ml) dropwise and the reaction mixture was stirred at 0°C for 2 h. The reaction mixture was diluted with DCM (100 ml) and washed twice each with 1 M HCl, 1 M NaOH and once with brine. The organic phase was dried over sodium sulfate and the solvent was distilled off.

### Intermediate 59e):

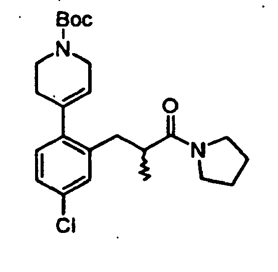

Intermediate 59d) (3226 mg), N-Boc-1,2,3,6-tetrahydropyridine-4-boronic acid pinacol ester (3170 mg), potassium carbonate (4047 mg) and dichloro(1,1'-bis(diphenyl-phosphino)-ferrocene)palladium(II) DCM adduct (482 mg) were dissolved in DMF (100 ml) in a dry apparatus under argon and the mixture was degassed by bubbling with argon for 30 min. The orange suspension was then heated under argon in an oil bath at 85°C overnight to give a dark purple suspension. The reaction mixture was filtered through Celite and evaporated to dryness *in vacuo.* The crude product was purified by column chromatography.

### Intermediate 59f):

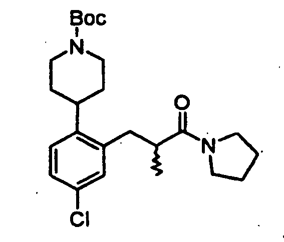

Intermediate 59e) (2793 mg) was dissolved in EtOH (50 ml) and AcOH (50 ml) and platinum(IV) oxide (148 mg) was added. The reaction mixture was evacuated three times and purged with hydrogen. The reaction mixture was then stirred at room temperature under hydrogen for 2 h. The reaction mixture was filtered and evaporated to dryness *in vacuo.* The residue was coevaporated with toluene (3 x 75 ml) and was finally dried under high vacuum at room temperature overnight. The crude product was purified by column chromatography.

### Intermediate 59g):

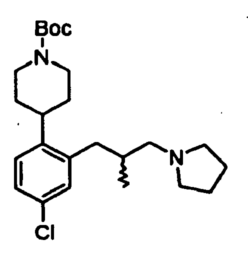

Intermediate 59f) (2092 mg) in diethyl ether (20 ml) was slowly added to a mixture of lithium aluminum hydride (274 mg) and diethyl ether (30 ml) at 0°C. After addition, the reaction mixture was stirred at 0°C for 2 h. The reaction mixture was hydrolyzed with a minimum amount of water. The inorganic precipitate was filtered off and washed twice with diethyl ether. The combined filtrate was dried over sodium sulfate, filtered again, and the solvent was removed under reduced pressure. The crude product was purified by flash chromatography to yield a colorless oil.

### Intermediate 59h):

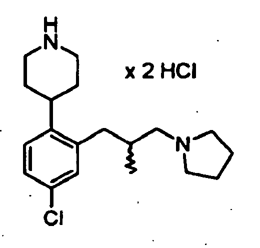

To intermediate 59g) (901 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml), and the product was filtered off. The product was obtained as a white solid.

### Example 59:

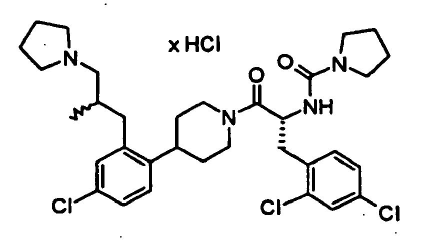

Intermediate 59h) (63 mg), B-C Moiety 1 (58 mg), and HOBt (27 mg) were dissolved in DMF (2 ml). NMM (26 µl) was added and the mixture stirred at room temperature for 20 min. EDCI (34 mg) was added, and the reaction stirred at room temperature for another 60 min. An additional amount of NMM (20 µl) was added and stirring continued at room temperature overnight. The reaction mixture was poured into brine and extracted with EtOAc and the phases were separated. The aqueous phase was extracted twice with ethyl acteate. The combined organic layer was washed twice with sat. Na₂CO₃, twice with water and brine. The organic layer was dried over Na₂SO₄ and evaporated *in vacuo.* The crude product was purified with flash chromatography. The free base was dissolved in ethyl acetate (2 ml) and 1 M HCl in diethyl ether (200 µl) was added. The product was precipitated by addition of hexane (20 ml). The precipitate was filtered off and dried *in vacuo* over P₂O₅.

### Synthesis of Example 63:

### Intermediate 63a):

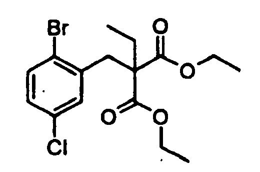

To a solution of sodium ethoxide (2.72 g) in ethanol (60 ml) was added diethyl methylmalonate (7.87 ml) followed by intermediate 42a) (11.38 g) and the reaction mixture was kept under reflux overnight. The reaction mixture was evaporated *in vacuo* and the residue was partitioned between diethyl ether and water. The aqueous layer was extracted two times with diethyl ether. The combined organic layer was washed twice with water and brine. The organic layer was dried over Na₂SO₄ and evaporated *in vacuo* to dryness. The product was purified by distillation. The fractions which distilled off between 140 and 220°C at 0.2 mbar were collected.

### Intermediate 63b):

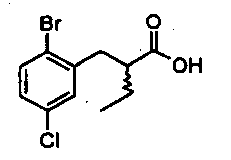

Intermediate 63a) (12.11 g) was heated under reflux in 1.8 M KOH in H₂O/EtOH (150 ml) for 5 h. After evaporation of the ethanol, an additional amount of KOH (54 g) was added to the residue, and the reaction mixture was stirred at 100°C for 2 h. The reaction mixture was diluted with H₂O (200 ml), extracted with Et₂O, and the organic layer was washed with H₂O. The combined aqueous layer was cooled in ice/H₂O and acidified with 50% H₂SO₄ to pH 1. The resulting suspension was extracted twice with Et₂O (200 ml each) and the organic layers were washed with water and brine. The combined organic layer was dried over Na₂SO₄ and evaporated *in vacuo* to dryness. The residue was triturated in hexane and less Et₂O, then filtered and washed with hexane and less Et₂O. The solid residue was decarboxylated by heating at 200°C. The evolution of CO₂ ceased after 20 min and the product was left to cool to room temperature to yield a brown oil.

### Intermediate 63c):

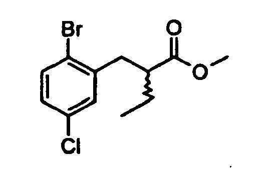

Intermediate 63b) (5.43 g) was dissolved in methanol (11.93 ml). Then sulfuric acid (365 µl) was added, and the reaction mixture was heated under reflux overnight (oil bath temperature 85°C) with exclusion of humidity by means of a drying tube (blue silica gel). The reaction mixture was evaporated *in vacuo* at 40°C and the colorless oily residue was poured into ice-water (100 ml). The resulting white emulsion was extracted with Et₂O (100 ml), and the organic phase was washed with sat. Na₂CO₃ (3 x 30 ml), H₂O (20 ml), and brine (20 ml). The organic phase was then dried over MgSO₄ and evaporated in *vacuo.*

### Intermediate 63d):

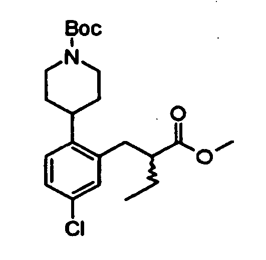

Zinc activation. Celite (174 mg) was added into a flame dried 50 ml Schlenk flask and dried by heating *in vacuo.* Then zinc dust (883 mg) and dry DMA (1.5 ml) were added under argon. The mixture was stirred at room temperature while a 7:5 v/v mixture of TMSCl/1,2-dibromoethane (153 µl TMSCI, 109 µl 1,2-dibromoethane, solution in 0.7 ml of DMA) was added at a rate to maintain the temperature below 65°C. The resulting slurry was aged for 15 min.

Zink insertion. A solution of Boc-4-iodopiperidine (3364 mg) in dry DMA (6.8 ml) was slowly added under argon atmosphere to the mixture described above at a rate to maintain the temperature below 65°C. The reaction mixture was then aged for 30 min at room temperature.

Coupling. A 50 ml three-necked flask was charged with dichloro-1,1'-bis(diphenylphosphino)-ferrocene-palladium(II) DCM adduct (188 mg), copper iodide (88 mg) and intermediate 63c) (2.36 g) in DMA (11 ml) The resulting mixture was degassed three times and the filtrate of the zinc insertion reaction was then added. The reaction mixture was degassed two times, then heated to 80°C and stirred overnight. The reaction mixture was concentrated under high vacuum at 60°C and the remainig black oil was taken up in a mixture of ethyl acetate and water. The mixture was filtered through Celite and the phases were separated. The aqueous phase was extracted twice with ethyl acetate The combined organic layer was washed with water and brine, dried over sodium sulfate and the solvent was removed under reduced pressure. The product was purified using flash chromatography.

### Intermediate 63e):

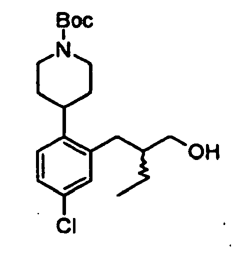

Intermediate 63d) (571 mg) in diethyl ether (20 ml) was slowly added to a mixture of lithium aluminum hydride (79 mg) and diethyl ether (30 ml) at 0°C. After addition the reaction mixture was stirred at 0°C for 2 h. The reaction mixture was hydrolyzed with a minimum amount of water. The inorganic precipitate was filtered off and washed twice with diethyl ether. The combined filtrate was dried over sodium sulfate, filtered, and the solvent was removed under reduced pressure.

### Intermediate 63f):

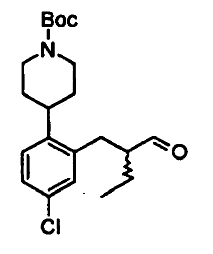

Intermediate 63e) (450 mg) was dissolved in dry DMSO (6 ml) and triethylamine (1151 µl). A solution of sulfurtrioxide-pyridine complex (563 mg) in dry DMSO (6 ml) was slowly added while the reaction mixture was maintained at 25°C. The reaction mixture was stirred for 4 h. After acidification of the reaction mixture to pH 4.5-5 with 1N HCl, it was poured into water. The resulting emulsion was extracted three times with diethyl ether. The combined organic layer was washed with water and brine, dried over sodium sulfate, and the solvent was removed under reduced pressure. The crude product was purified by flash chromatography.

### Intermediate 63g):

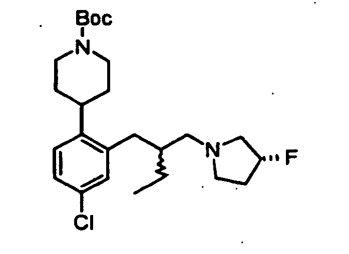

To a solution of intermediate 63f) (97 mg) and (R)-3-fluoropyrrolidine hydrochloride (33 mg) in 1,2-dichloroethane (3 ml), DIEA (67 µl) was added followed by sodium triacetoxyborohydride (74 mg). The reaction mixture was then stirred at room temperature overnight. The mixture was diluted with EtOAc (70 ml) and washed two times with sat. NaHCO₃ (25 ml), water and brine (25 ml each). The organic phase was dried over Na₂SO₄ and concentrated. The product was purified with flash chromatography.

### Intermediate 63h):

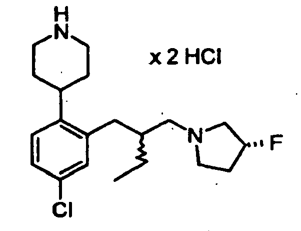

To intermediate 63g) (101 mg) in dioxane (5 ml) and methanol (1 ml) was added hydrogen chloride, 4.0 M sol. in 1,4-dioxane (5 ml) and the solution was stirred for 60 min at room temperature. Then the solvent was removed under reduced pressure to yield the product as a colorless glassy solid.

### Example 63:

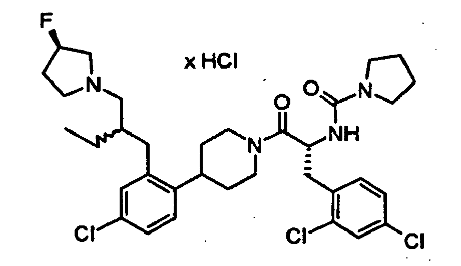

Intermediate 63h) (47 mg), B-C Moiety 1 (39 mg), and HOBt (27 mg) were dissolved in DMF (1 ml). NMM (18 µl) was added and the mixture stirred at room temperature for 20 min. EDCI (23 mg) was added, and the reaction stirred at room temperature for another 60 min. An additional amount of NMM (10 µl) was added and stirring continued at room temperature overnight. The reaction mixture was poured into brine and extracted with EtOAc and the phases were separated. The aqueous phase was extracted twice with ethyl acteate. The combined organic layer was washed twice with sat. Na₂CO₃, twice with water and brine. The organic layer was dried over Na₂SO₄ and evaporated *in vacuo.* The crude product was purified with flash chromatography. The free base was dissolved in ethyl acetate (2 ml) and 1 M HCl in diethyl ether (200 µl) was added. The product was precipitated by addition of hexane (20 ml).- The precipitate was filtered off and dried in *vacuo* over P₂O₅.

### Synthesis of Example 65:

### Intermediate 65a):

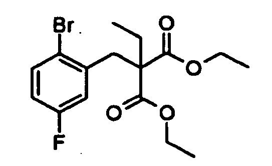

To a solution of sodium ethoxide (1.26 g) in ethanol (30 ml) was added diethyl ethylmalonate (3.64 ml) followed by 2-bromo-5-fluorobenzylbromide (4.96 g) and the reaction mixture was kept under reflux overnight. The reaction mixture was evaporated in *vacuo* and the residue was partitioned between diethyl ether and water and the aqueous layer was extracted two times with diethyl ether. The combined organic layer was washed twice with water and brine. The organic layer was dried over Na₂SO₄ and evaporated in *vacuo* to dryness. The product was purified using flash chromatography.

### Intermediate 65b):

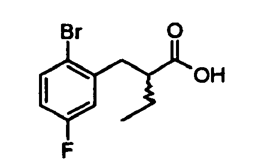

Intermediate 65a) (5.27 g) was heated under reflux in 1.8 M KOH in H₂O/EtOH (60 ml) for 5 h. After evaporation of the ethanol an additional amount of KOH (18 g) was added to the residue, and the reaction mixture was stirred at 100°C for 2 h. The reaction mixture was diluted with H₂O (100 ml), extracted with Et₂O and the organic layer was washed with H₂O. The combined aqueous layer was cooled in ice/H₂O and acidified with 50% H₂SO₄ to pH 1. The resulting suspension was extracted twice with Et₂O (100 ml each) and the organic layers were washed with water and brine. The combined organic layer was dried over Na₂SO₄ and evaporated *in vacuo* to dryness. The residue was triturated in hexane and less Et₂O, then filtered and washed with hexane and less Et₂O. The solid residue was decarboxylated by heating at 200°C. The evolution of CO₂ ceased after 20 min and the melt was left to cool to room temperature to yield beige needles.

### Intermediate 65c):

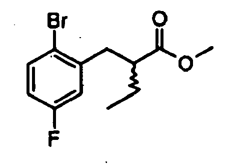

Intermediate 65b) (2.71 g) was dissolved in methanol (7.53 ml). Then sulfuric acid (100 µl) was added, and the reaction mixture was heated under reflux overnight (oil bath temperature 85°C) with exclusion of humidity by means of a drying tube (blue silica gel). The reaction mixture was evaporated *in vacuo* at 40°C and the colorless oily residue was poured into ice-water (50 ml). The resulting white emulsion was extracted with Et₂O (75 ml), and the organic phase was washed with sat. Na₂CO₃ (3 x 20 ml), H₂O (15 ml), and brine (15 ml). The organic phase was then dried over MgSO₄ and evaporated *in vacuo.*

### Intermediate 65d):

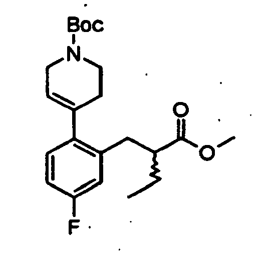

Intermediate 65c) (2520 mg), N-Boc-1,2,3,6-tetrahydropyridine-4-boronic acid pinacol ester (2829 mg), potassium carbonate (3611 mg) and dichloro(1,1'-bis(diphenyl-phosphino)-ferrocene)palladium(II) DCM adduct (425 mg) were dissolved in DMF (100 ml) in a dry apparatus under argon and the mixture was degassed by bubbling with argon for 30 min. The orange suspension was then heated under argon in an oil bath at 85°C overnight to give a dark purple suspension. The reaction mixture was filtered through Celite and evaporated to dryness *in vacuo.* The crude product was purified by column chromatography.

### Intermediate 65e):

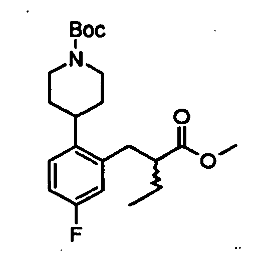

Intermediate 65d) (1453 mg) was dissolved in ethanol (50 ml) and 10% palladium on activated carbon (150 mg) was added. The reaction mixture was purged three times with hydrogen (5 bar) and stirred under a hydrogen atmosphere (25 bar) overnight. The crude mixture was filtered through Celite and the solvent was removed under reduced pressure to yield a beige oil.

### Intermediate 65f):

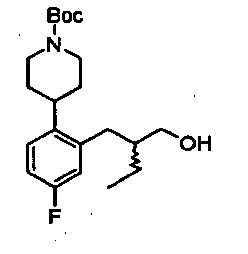

Intermediate 65e) (1288 mg) in diethyl ether (20 ml) was slowly added to a mixture of lithium aluminum hydride (186 mg) and diethyl ether (30 ml) at 0°C. After addition, the reaction mixture was stirred at 0°C for 2 h. The reaction mixture was hydrolyzed with a minimum amount of water. The inorganic precipitate was filtered off and washed twice with diethyl ether. The combined filtrate was dried over sodium sulfate, filtered again, and the solvent was removed under reduced pressure.

### Intermediate 65g):

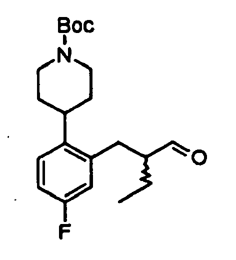

To a solution of intermediate 65f) (1001 mg) in DCM (20 ml) was slowly added Dess-Martin periodinane (1510 mg). After addition, the reaction mixture was stirred at room temperature for 3 h. TLC indicated that the reaction was not complete. A second batch of Dess-Martin periodinane (755 mg) was added and the reaction was stirred overnight. The reaction mixture was diluted with DCM and washed three times with saturated sodium bicarbonate solution and brine. The organic phase was dried over sodium sulfate, filtered, and the solvent was removed under reduced pressure. The product was purified by flash chromatography.

### Intermediate 65h):

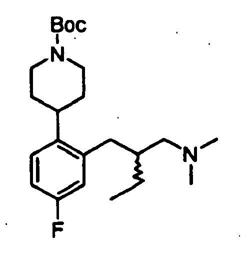

To a solution of intermediate 65g) (91 mg) and dimethylamine, 2.0 M solution in THF (250 µl) in 1,2-dichloroethane (3 ml), sodium triacetoxyborohydride (74 mg) was added. The reaction mixture was then stirred at room temperature overnight. The mixture was diluted with EtOAc (70 ml) and washed two times with sat. NaHCO₃ (25 ml), water and brine (25 ml each). The organic phase was dried over Na₂SO₄ and concentrated. The product was purified by flash chromatography.

### Intermediate 65i):

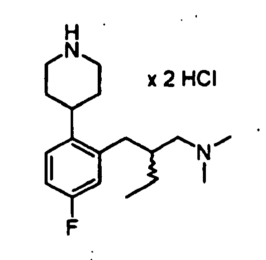

To intermediate 65h) (74 mg) in dioxane (5 ml) and methanol (1 ml) was added hydrogen chloride, 4.0 M sol. in 1,4-dioxane (5 ml) and the solution was stirred for 60 min at room temperature. The solvent was removed under reduced pressure to yield the product as a colorless glassy solid.

### Example 65:

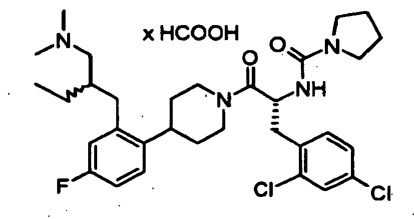

Intermediate 65i) (35 mg), B-C Moiety 1 (34 mg), and HOBt (16 mg) were dissolved in DMF (1 ml). NMM (26 µl) was added and the mixture stirred at room temperature for 20 min. EDCI (20 mg) was added, and the reaction stirred at room temperature for another 60 min. An additional amount of NMM (10 µl) was added and stirring continued at room temperature overnight. The reaction mixture was poured into brine and extracted with EtOAc and the phases were separated. The aqueous phase was extracted twice with ethyl acteate. The combined organic layer was washed twice with sat. Na₂CO₃, twice with water and brine. The organic layer was dried over Na₂SO₄ and evaporated *in vacuo.* The crude product was purified with preparative HPLC-MS.

### Synthesis of Example 69:

### Intermediate 69a):

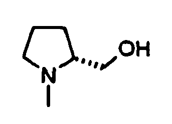

A mixture of D-prolinol (2.50 ml), formaldehyde solution (3.23 ml, 36.5% in H₂O) and formic acid (1.93 ml) in water (17 ml) was kept under reflux for 24 h. The reaction mixture was concentrated *in vacuo,* the residue was diluted with water and made alkaline by addition of 1 N NaOH (pH 10-11). The aqueous solution was extracted twice with Et₂O and the organic layers were washed with water and brine. The combined organic layer was dried over Na₂SO₄ and evaporated *in vacuo* to dryness. The combined aqueous layer was saturated with NaCl and the pH was adjusted to 14 by adding 1 N NaOH. The aqueous solution was extracted twice with CH₂Cl₂. The combined organic layer was dried over Na₂SO₄ and evaporated *in vacuo* to dryness. Extract 1 and extract 2 were combined and purified by Kugelrohr-distillation (20 mbar / 90-110°C).

### Intermediate 69b):

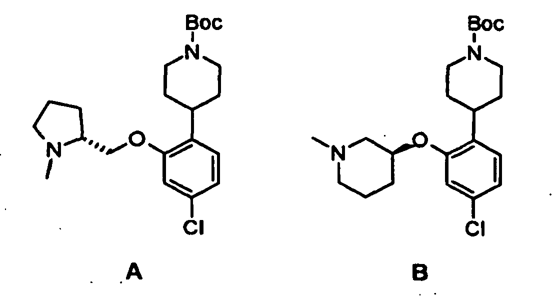

A solution of intermediate 27d) (0.50 g), intermediate 69a) (0.37 g), and triphenylphosphine (0.84 g) in THF (15 ml) under argon, was cooled in ice/H₂O. DEAD (ca. 40% in toluene, 1.47 ml) was added dropwise, at a rate to keep the temperature below 5°C (ca. 15 min). After stirring for another 10 min in ice/H₂O, the cooling bath was removed and the mixture was stirred at room temperature overnight. The reaction mixture was evaporated to dryness *in vacuo* at 40°C. The two regioisomeric products were separated by flash chromatography.

### Intermediate 69c):

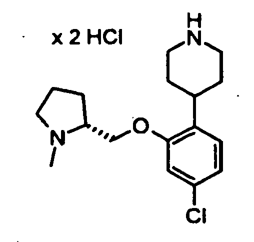

To Boc-protected intermediate 69b) (product A) (358 mg) in dioxane (2 ml) was added hydrogen chloride, 4.0 M sol. in 1,4-dioxane (5 ml) and the solution was stirred at room temperature for 1 h. The solvent was removed under reduced pressure. The residue was dissolved in iso-propanol and the salt precipitated after 5 min. The suspension was diluted with acetone and Et₂O, the solid was filtered off, washed with acetone and Et₂O and finally dried *in vacuo* over Sicapent overnight.

### Example 69:

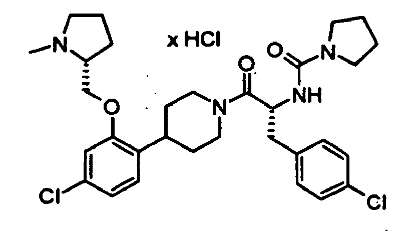

Intermediate 69c) (50 mg), B-C Moiety 1 (40 mg), and HOBt (30 mg) were dissolved in DCM (3 ml). NMM (40 µl) was added and the mixture stirred at room temperature for 30 min. EDCI (44 mg) was added, and the reaction stirred at room temperature for another 60 min. An additional amount of NMM (11 µl) was added and stirring continued at room temperature overnight. The reaction mixture was evaporated *in vacuo,* diluted with EtOAc and washed with sat. Na₂CO₃, water and brine. The organic layer was dried over Na₂SO₄ and evaporated *in vacuo.* The crude product was purified by flash chromatography. The purified product was dissolved in DCM, treated with 1 M HCl in Et₂O (108 µl), and the resulting suspension was diluted with diethyl ether and hexane. The precipitate was filtered off, washed with hexane and diethylether, and dried *in vacuo* at 40°C for 2 h. The product was obtained as an off-white solid.

### Synthesis of Example 70:

### Intermediate 70a):

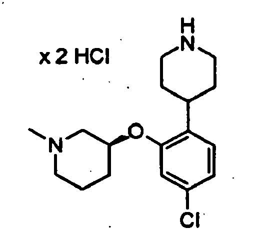

To Boc-protected intermediate 69b) (product B) (84 mg) in dioxane (0.5 ml) was added hydrogen chloride, 4.0 M sol. in 1,4-dioxane (1.5 ml) and the solution was stirred at room temperature for 1 h. The solvent was removed under reduced pressure. The residue was dissolved in iso-propanol and the salt precipitated after 5 min. The suspension was diluted with acetone and Et₂O, the solid was filtered off, washed with acetone and Et₂O and finally dried *in vacuo* over Sicapent overnight.

### Example 70:

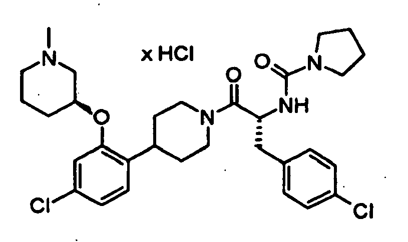

Intermediate 70a) (24 mg), B-C Moiety 1 (26 mg), and HOBt (15 mg) were dissolved in DCM (2 ml). NMM (19 µl) was added and the mixture stirred at room temperature for 30 min. EDCI (21 mg) was added, and the reaction stirred at room temperature for another 60 min. An additional amount of NMM (5 µl) was added and stirring continued at room temperature overnight. The reaction mixture was evaporated *in vacuo,* diluted with EtOAc and washed with sat. Na₂CO₃, water and brine. The organic layer was dried over Na₂SO₄ and evaporated *in vacuo.* The crude product was purified by flash chromatography. The purified product was dissolved in DCM, treated with 1 M HCI in Et₂O (60 µl), and the resulting suspension was diluted with diethyl ether and hexane. The precipitate was filtered off, washed with hexane and diethyl ether, and dried *in vacuo* at 40°C for 2 h. The product was obtained as a white solid.

### Synthesis of Example 71:

### Intermediate 71a):

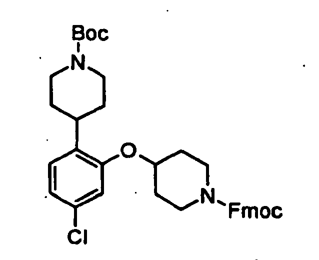

A solution of intermediate 27d) (250 mg) and N-(Fmoc)-4-piperidinol (519 mg), and triphenylphosphine (521 mg) in anhydrous THF (8 ml) under argon, was cooled in ice/H₂O. Then DEAD (ca. 40% in toluene, 735 µl) was added dropwise, at a rate to keep the temperature below 5°C (ca. 15 min). After stirring for another 10 min in ice/H₂O, the cooling bath was removed and the mixture was stirred at room temperature overnight and at 45°C for 3 h. The reaction mixture was evaporated to dryness *in vacuo,* diluted with EtOAc, washed with 0.5 N HCl, sat. Na₂CO₃, H₂O and brine. The aqueous layers were extracted with EtOAc. The combined organic layer was dried over Na₂SO₄. filtered and evaporated *in vacuo* to dryness. The residue was triturated in CH₂Cl₂ and EtOAc, the insoluble solid was filtered off and washed CH₂Cl₂ and EtOAc. The filtrate was evaporated *in vacuo* to dryness and the residue was purified by flash column chromatography.

### Intermediate 71b):

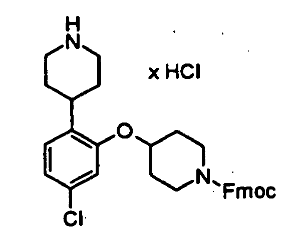

To Boc-protected intermediate 71a) (40 mg) in dioxane (0.5 ml) was added hydrogen chloride, 4.0 M solution in 1,4-dioxane (2.0 ml) and the solution was stirred at room temperature for 1.5 h. The solvent was removed under reduced pressure. The residue was dissolved in acetone and the product was precipitated by addition Et₂O and hexane. The product was filtered off, and washed with hexane and Et₂O.

### Intermediate 71c):

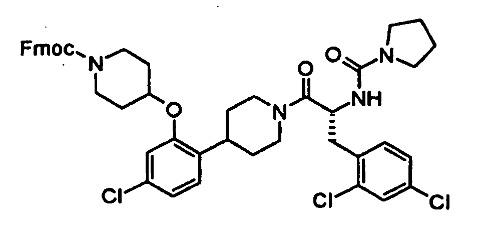

B-C Moiety 1 (14 mg) and HOAt (6 mg) were dissolved in CH₂CL₂ (2 ml), then EDCI (11 mg) was added and the reaction mixture was stirred at room temperature for 30 min. Then intermediate 71b) (21 mg) was added, followed by NMM (10 µl), and the reaction mixture was stirred at room temperature overnight. The reaction mixture was diluted with EtOAc, washed with HCl 0.5 N, sat. Na₂CO₃, H₂O and brine. The aqueous layers were extracted with EtOAc. The combined organic layer was dried over Na₂SO₄, filtered and evaporated *in vacuo* to dryness.

### Example 71:

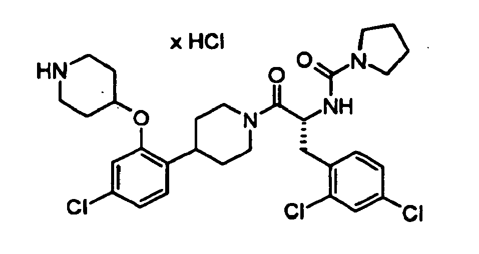

Diethylamine (0.5 ml) was added to a solution of intermediate 71c) (31 mg) in CH₂Cl₂ (1 ml) and the reaction mixture was stirred at room temperature overnight. The reaction mixture was evaporated *in vacuo* and the residue purified by flash chromatography.
The free base was dissolved in CH₂Cl₂, acidified with HCl in Et₂O 1 M (25 µl) and evaporated *in vacuo.* The residue was dissolved in CH₂Cl₂ and the salt was precipitated by addition of Et₂O and hexane. The precipitate was filtered off, washed with hexane and Et₂O and dried *in vacuo* at 40°C for 2 hours. The product was obtained as a white solid

### Synthesis of Example 80:

### Intermediate 80a):

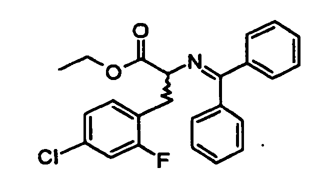

N-(Diphenylmethylene) glycine ethyl ester (9.29 g), 1-(bromomethyl)-4-chloro-2-fluorobenzene (8.63 g) and benzyltriethylammonium chloride (TEBAC) (7.91 g) were dissolved in DCM (100 ml) and 10% aqueous KOH (91 ml) was added. The resulting two-phase mixture was stirred at room temperature for 24 hours. Then the organic layer was separated and concentrated. The residue was taken up in diethyl ether (200 ml) and washed with water (150 ml) followed by brine (100 ml) and the organic layer was dried over Na₂SO₄. The solvent was removed under reduced pressure. The product was purified by flash chromatography.

### Intermediate 80b):

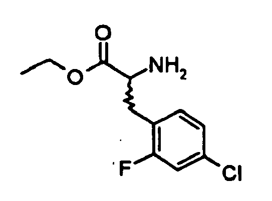

5% aq. HCl in H₂O (9 ml) was added portionwise to a solution of intermediate 80a) (1 g) in THF (3.4 ml) at 0°C. A precipitate appeared after the addition. The mixture was stirred at room temperature for 1 h. THF was evaporated under reduced pressure. Water (50 ml) was added to the residue. The aqueous solution was washed with diethyl ether (3 x 75 ml) and with DCM (75 ml). The aqueous layer was then basified with 5 N aqueous NaOH (2 ml) and 1 N aqueous NaOH (12 ml) to get pH7/8. The compound was extracted with DCM (5 x 100 ml). The combined organic layer was dried over Na₂SO₄. filtered and evaporated under reduced pressure. The crude compound was purified by column chromatography.

### Intermediate 80c):

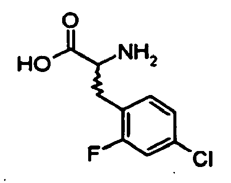

1 N Aqueous sodium hydroxide (5.5 ml) was added portionwise to a solution of intermediate 80b) (446 mg) in THF (5 ml) at 0°C. The reaction mixture was stirred at room temperature overnight. The solvent was evaporated under reduced pressure and water (60 ml) was added to the residue. The aqueous phase was washed with diethyl ether (2 x 90 ml; 3 x 30 ml) and DCM (2 x 100 ml). The aqueous phase was neutralized with 5 N aqueous HCl (1 ml). The impurities were extracted with diethyl ether (2 x 100 ml). The aqueous phase was evaporated to dryness and water (40 ml) was added. The suspension was put in the fridge overnight, filtered, and the product, a white solid, was rinsed with water and diethyl ether. The filtrate was evaporated again to dryness and water (10 ml) was added. The suspension was put in the fridge overnight, filtered and the second batch of product was rinsed with water and diethyl ether. The solids from the two batches were combined and dried in vacuo.

### Intermediate 80d):

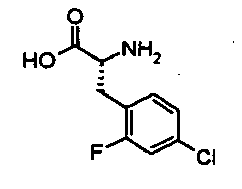

Racemic intermediate 80c) (313 mg) was dissolved in Tris-maleate buffer (26 ml, pH 7.8) containing 0.1 M KCl. To this solution was added L-amino acid oxidase (Sigma Type 1, activity 0.33 units/mg; 10 mg) and catalase (1 mg). After 84 h, the reaction mixture was brought to pH 7 with 0.5 N HCl and purified by ion-exchange chromatography over Dowex 50, eluting the amino acid with 1 N ammonia. The solvent was removed under reduced pressure and the product was dried *in vacuo* at room temperature over P₂O₅ overnight.

### Intermediate 80e):

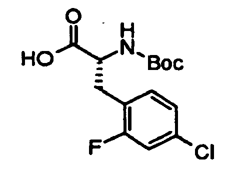

Intermediate 80d) (360 mg) was dissolved in 2 M sodium hydroxide (0.7 ml) and cooled to 0°C. Di-tert-butyl dicarbonate (188 mg) in dioxane (1 ml) was slowly added. After half an hour, the reaction mixture was warmed to room temperature and allowed to stir overnight. A second batch of di-tert-butyl-dicarbonate (79 mg) was added and stirring was continued for another 4 h. The reaction mixture was evaporated to dryness and water (20 ml) was added. The aqueous phase was washed with diethyl ether (5 x 40 ml) and DCM (3 x 30 ml). The aqueous phase was acidified to pH 2 using 1 N, aqueous hydrochloric acid and extracted with ethyl acetate (3 x 40 ml). The combined organic layer was then dried over Na₂SO₄. filtered and concentrated *in vacuo.*

### Intermediate 80f):

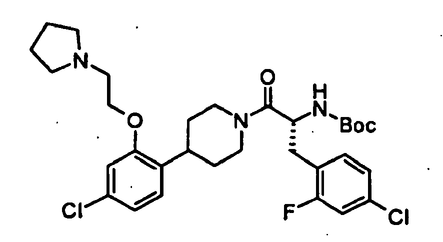

To a solution of intermediate 80e) (60 mg) in dry DMF (3.6 ml) was added HOBt (32 mg), EDCI (40 mg) and NMM (83 µl). The reaction mixture was stirred for 5 min and then intermediate 20b) (79 mg) was added as a solid at 0°C. The reaction stirred at room temperature overnight. The solvent was evaporated and the residue was diluted with ethyl acetate (35 ml) and successively washed with water (20 ml), followed by saturated NaHCO₃ (20 ml) and brine (35 ml). The aqueous phases were extracted again with ethyl acetate (20 ml). The combined organic layer was dried over Na₂SO₄, filtered and evaporated. The crude compound was purified by column chromatography.

### Intermediate 80g)

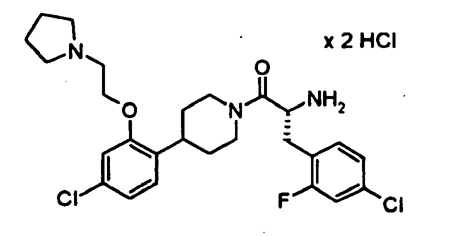

Intermediate 80f) (95 mg) was dissolved in dioxane (1.6 ml) and 4M HCl in dioxane (1.13ml) was added at 0°C. The reaction mixture was stirred at room temperature for 90 min. The reaction mixture was evaporated to dryness *in vacuo.* The residue was dissolved in MeOH and triturated with Et₂O. A beige solid compound was obtained. It was filtered off, rinsed with Et₂O and dried under high vacuum.

### Example 80:

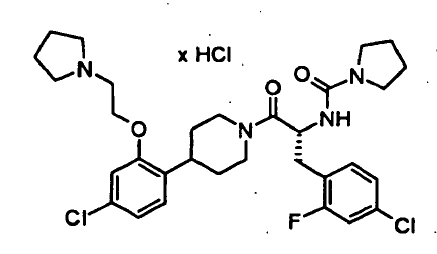

Intermediate 80g) (58 mg) was dissolved in 1,2-dichloroethane (3 ml) and triethylamine (45 µl) was added followed by intermediate 104i) (37 mg) and the reaction mixture was stirred in an oil bath at 60°C overnight. The reaction mixture was diluted with ethyl acetate (25 ml) and washed with sat. NaHCO₃ (2 x 10 ml), water (2 x 10 ml) and brine (10 ml). The organic layer was dried over Na₂SO₄, filtered and evaporated *in vacuo* to give a yellow oil. The crude product was purified by flash chromatography.
The free base was dissolved in ethyl acetate (100 µl), treated with 1N HCl in Et₂O (53 µl), and the resulting suspension was diluted with diethyl ether (0.5 ml). The precipitate was filtered off, washed with diethyl ether, and dried *in vacuo* over Sicapent.

### Synthesis of Example 96:

### Intermediate 96a):

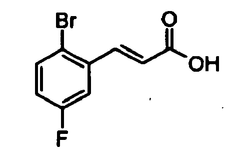

A mixture of 2-bromo-5-fluorobenzaldehyde (10.15 g), malonic acid (5.72 g) and pyridine (1.5 ml) in ethanol (25 ml) was kept under reflux for 7.5 h. After cooling in an ice bath the crystal mass was filtered off. The crystals were washed with cold ethanol (10 ml) and then washed twice with diethyl ether (10 ml each). The residue was suspended in ethanol (60 ml) and kept under reflux for 2-3 h. The mixture was cooled and filtered and the solid was dried under reduced pressure. The product was obtained in form of colorless needles.

### Intermediate 96b):

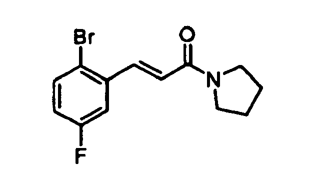

Intermediate 96a) (2451 mg), pyrrolidine (918 µl), EDCI (2109 mg) and DMAP (100 mg) were dissolved in DCM (100 ml) and stirred overnight. The reaction mixture was poured into water (100 ml) and the organic layer was separated. The aqueous layer was extracted twice with DCM: The combined organics were washed three times with with 0.5 N HCl (30 ml each), three times with 1 M sodium hydroxide solution and brine, dried over Na₂SO₄ and the solvent was removed under reduced pressure. The crude product was purified by flash chromatography to yield a white solid.

### Intermediate 96c):

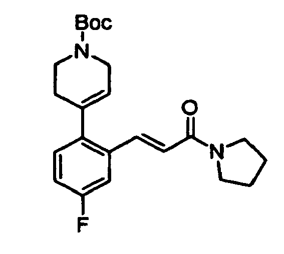

Intermediate 96b) (1130 mg), N-Boc-1,2,3,6-tetrahydropyridine-4-boronic acid pinacol ester (1231 mg), potassium carbonate (1571 mg) and dichloro(1,1'-bis(diphenyl-phosphino)-ferrocene)palladium(II) DCM adduct (188 mg) were dissolved in DMF (50 ml) in a dry apparatus under argon and the mixture was degassed by bubbling with argon for 30 min. The orange suspension was then heated under argon in an oil bath at 85°C for.1 day. The reaction mixture was filtered through Celite and evaporated to dryness *in vacuo.* The crude product was purified by flash chromatography to yield a beige solid.

### Intermediate 96d):

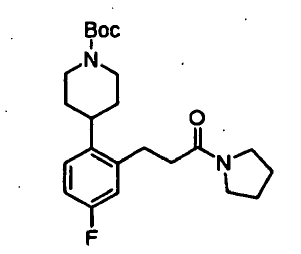

Intermediate 96c) (1459 mg) was dissolved in ethanol (50 ml) and 10% palladium on activated carbon (150 mg) was added. The reaction mixture was purged three times with hydrogen (5 bar) and stirred under hydrogen atmosphere (10 bar) for 3 days. The crude mixture was filtered through Celite and the solvent was removed under reduced pressures to yield a yellow-grey oil.

### Intermediate 96e):

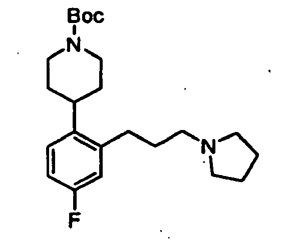

Intermediate 96d) (1472 mg) in diethyl ether (20 ml) was slowly added to a mixture of lithium aluminum hydride (207 mg) and diethyl ether (30 ml) at 0°C. After addition the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a pale yellow oil.

### Intermediate 96f):

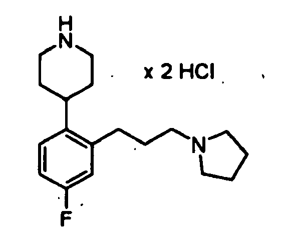

To intermediate 96e) (654 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off to yield an off-white solid.

### Example 96:

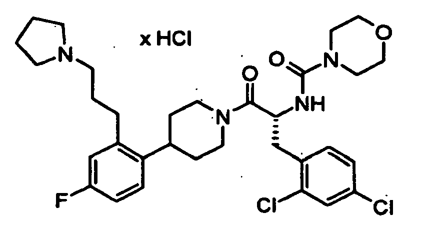

Intermediate 96f) (58 mg), B-C Moiety 2 (61 mg), and HOBt (27 mg) were dissolved in DCM (2 ml). NMM (26 µl) was added and the mixture stirred at room temperature for 20 min. EDCI (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated *in vacuo.* The crude product was purified with flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried *in vacuo* at room temperature over P₂O₅ overnight. The product was obtained as a white solid.

### Synthesis of Example 104:

### Intermediate 104a):

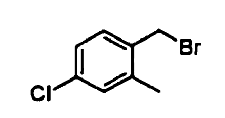

Phosphorous tribromide (735 µl) was added to a stirred solution of 4-chloro-2-methylbenzyl alcohol (3.5 g) in toluene (30 ml) at 40°C. The solution was heated to 100°C for 30 min, and the reaction was cooled to ambient temperature. The liquid was decanted and washed with water (2 x 50 ml) and brine (50 ml). The combined aqueous layer was extracted with diethyl ether (70 ml) and the combined organic layer was evaporated to yield a semisolid residue. The residue was dissolved in diethyl ether (350 ml) and washed with water (2 x 100 ml) and brine (100 ml). The organic phase was dried over Na₂SO₄, filtered and evaporated to yield a light yellow oil.

### Intermediate 104b):

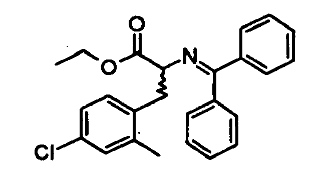

N-(Diphenylmethylene) glycine ethyl ester (5.27 g), intermediate 104a) (4.81 g) and benzyltriethylammonium chloride (TEBAC) (4.49 g) were dissolved in DCM (52 ml) and 10% aqueous KOH (52 ml) was added. The resulting two-phase mixture was stirred at room temperature for 24 h. The organic layer was separated and concentrated. The residue was taken up with diethyl ether (125 ml) and washed with water (100 ml) followed by brine (100 ml) and dried over Na₂SO₄. The solvent was removed to give the crude product as a yellow oil. The crude product was purified by flash column chromatography.

### Intermediate 104c):

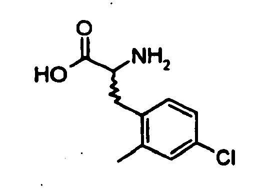

5% Aqueous HCl (50 ml) was added portionwise to a solution of intermediate 104b) (5.6 g) in THF (20 ml) at 0°C. The mixture was then stirred at room temperature for 2 h. The solvent was evaporated under reduced pressure and water (200 ml) was added to the residue. The aqueous phase was washed with diethyl ether (3 x 250 ml) and DCM (250 ml). The aqueous phase was then basified with 5N aqueous NaOH (11 ml) to get pH 7/8 and extracted with DCM (8 x 400 ml). The aqueous phase was concentrated to a volume of 150 ml and extracted with DCM (11 x 150 ml). The combined organic layer was dried over Na₂SO₄, filtered and the solvent was removed under reduced pressure to give a yellow oil. The crude product was dissolved in THF (20 ml) and 1 N aqueous sodium hydroxide (12.1 ml) was added portionwise at 0°C. The reaction mixture was stirred at room temperature overnight. The solvent was removed under reduced pressure and water (50 ml) was added to the residue. The aqueous phase was washed with diethyl ether (2 x 100 ml) and DCM (2 x 100 ml) and then neutralized to pH 7 with 5N aqueous HCl (2 ml). The aqueous phase was evaporated under reduced pressure to yield the product as a white solid.

### Intermediate 104d):

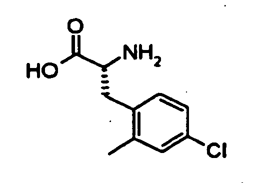

Intermediate 104c) (2.96 g) was dissolved in Tris-maleate buffer (125 mL) containing 0.1 M KCl. To the turbid solution was added L-amino acid oxidase (sigma Type 1, 85 mg) and catalase (8.5 mg). After 84 h of vigorous stirring at 35°C, the reaction was brought to pH 7 with 0.5N HCl (4.5 ml). The aqueous solution was reduced to a volume of 10 ml and then purified by ion exchange using Dowex 50 (60 ml). The product was eluted with water (500 ml), then 1 N ammonia (800 ml). The combined eluants were evaporated under reduced pressure to yield the title compound.

### Intermediate 104e):

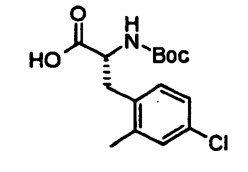

Intermediate 104d) (88 mg) was dissolved in 2N aqueous sodium hydroxide (0.58 ml) and cooled to 0°C. Di-tert-butyl dicarbonate (101 mg) was slowly added followed by dioxane (0.5 ml). After half an hour, the reaction mixture was warmed to room temperature and allowed to stir for 12 h. Di-tert-butyl-dicarbonate (101 mg) and 1 N aqueous NaOH (0.29 ml) were added. The reaction was stirred at room temperature for another 20 h. The reaction mixture was evaporated to dryness and water (2 ml) was added. The reaction mixture was acidified to pH 2 using 1N aqueous hydrochloric acid (1.3 ml) and extracted with DCM (3 x 20 ml). The combined organic layer was dried over Na₂SO₄, filtered and concentrated *in vacuo* to give a colorless wax. The crude product was purified by column chromatography.

### Intermediate 104f):

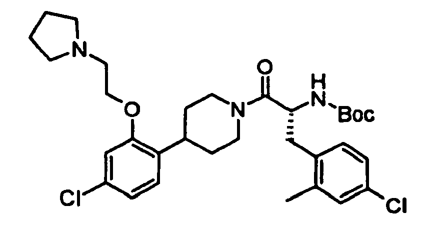

To a solution of intermediate 104e) (35 mg) in dry DMF (2 ml) was added HOBt (19 mg), EDCl (23 mg) and NMM (49 µl). The reaction mixture was stirred for 5 min and then intermediate 20b) (47 mg) was added as a solid at 0°C. The reaction was stirred at room temperature overnight. The solvent was evaporated and the residue was diluted with ethyl acetate (25 ml) and successively washed with water (15 ml), followed by saturated NaHCO₃ (15 ml) and brine (25 ml). The aqueous phases were extracted again with ethyl acetate (15 ml). The combined organic layer was dried over Na₂SO₄, filtered and evaporated. The crude compound was purified by column chromatography.

### Intermediate 104g)

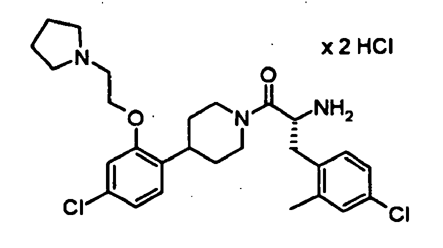

Intermediate 104f) (23 mg) was dissolved in dioxane (0.4 ml) and 4M HCl in dioxane (0.28 ml) was added at 0°C. The reaction mixture was stirred at room temperature for 90 min. The reaction mixture was evaporated to dryness *in vacuo.* The residue was dissolved in MeOH (0.1 ml) and triturated with Et₂O. A beige solid compound was obtained. It was filtered off, rinsed with Et₂O and dried under high vacuum.

### Intermediate 104h)

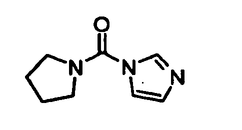

Pyrrolidine (2295 µl) and 1,1'-carbonyldiimidazole (4865 mg), were dissolved in dry THF (10 ml) and the reaction mixture was heated under reflux overnight. The reaction mixture was evaporated *in vacuo* to dryness. The residue was dissolved in CH₂Cl₂ (100 ml), and washed with H₂O (2x100 ml). The organic phase was dried with MgSO₄ and evaporated in *vacuo.* The product was obtained as a colorless crystalline solid.

### Intermediate 104i)

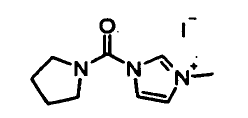

Intermediate 104h) (3725 mg) was dissolved in dry MeCN (35 ml). Methyl iodide (9200 µl) was added and the reaction mixture was stirred at room temperature under exclusion of light for 24 h. The reaction mixture was evaporated *in vacuo* to dryness. The residue was dried under high vacuum for 4 h at room temperature. The crude product was triturated in hot acetone (25 ml), left to cool down to room temperature, and was then stirred vigorously overnight. The insoluble crystalline compound was filtered off, washed with acetone (3x6 ml), and finally dried *in vacuo* over P₂O₅ overnight to yield a colorless crystalline solid.

### Example 104:

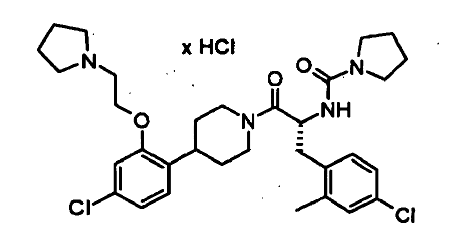

Intermediate 104g) (17 mg) was dissolved in 1,2-dichloroethane (2 ml) and triethylamine (14 µl) was added followed by intermediate 104i) (11 mg) and the reaction mixture was stirred in an oil bath at 60°C overnight. The reaction mixture was diluted with ethyl acetate (20 ml) and washed with sat. NaHCO₃ (2 x 10 ml), water (2 x 10 ml) and brine (10 ml). The organic layer was dried over Na₂SO₄, filtered and evaporated *in vacuo* to give a yellow oil. The crude product was purified by flash chromatography.
The free base was dissolved in ethyl acetate (25 µl), treated with 1 N HCl in Et₂O (20 µl), and the resulting suspension was diluted with diethyl ether (0.5 ml). The precipitate was filtered off, washed with diethyl ether, and dried *in vacuo* over Sicapent.

### Synthesis of Example 113:

### Intermediate 113a):

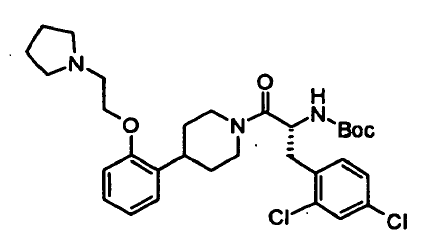

To Boc-D-2,4-dichlorophenylalanine (685 mg) in DCM (20 ml) was added the amine hydrochloride from 2b) (347 mg), N-methylmorpholine (311 µl), HOBt (230 mg) and the mixture was stirred for 30 min. EDCl (351 mg) was added and stirring was continued for 1 h. An additional amount of N-methylmorpholine (91 µl) was added and stirred overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated *in vacuo* to dryness. Purification by flash chromatography yielded the title compound as a white foam.

### Intermediate 113b):

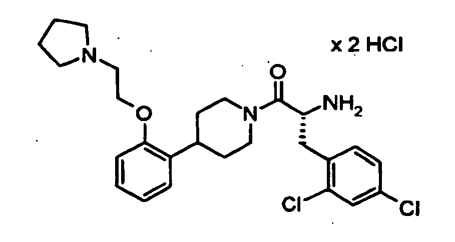

To Boc-protected intermediate 113a) (560 mg) in methanol (5 ml) was added hydrogen chloride, 4.0 M sol. in 1,4-dioxane (9 ml) and the solution was stirred at room temperature for 90 min. The reaction mixture was evaporated *in vacuo* to dryness. The residue was triturated in Et₂O, filtered off and washed with Et₂O to yield a beige solid.

### Intermediate 113c):

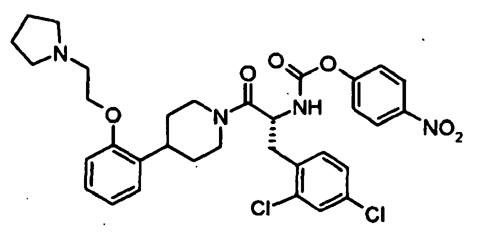

Intermediate 113b) was suspended in DCM (15 ml) and NMM (171 µl) was added. The mixture was cooled in an ice bath with stirring. Then 4-nitrophenyl chloroformate (134 mg) was added, and the reaction mixture was stirred at 0°C for 60 min. The ice bath was then removed and stirring was continued at room temperature overnight. The reaction mixture was diluted with EtOAc and washed with sat. Na₂CO₃, water, and brine. The aqueous layers were extracted with EtOAc. The combined organic layer was dried over Na₂SO₄ and evaporated *in vacuo.* The residue was purified by flash chromatography to yield a yellow oil.

### Example 113:

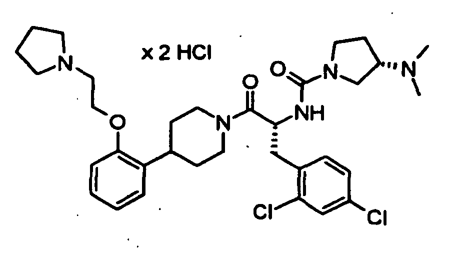

To a solution of intermediate 113c) (25 mg) in THF (1 ml) was added (3S)-3-dimethylaminopyrrolidine (22 mg) and the reaction mixture was stirred at room temperature overnight. The reaction mixture was evaporated *in vacuo.* The residue was redissolved in EtOAc and the organic layer was washed with sat. Na₂CO₃, water and brine. The aqueous layers were extracted with EtOAc. The combined organic layer was dried over Na₂SO₄ and evaporated *in vacuo* to dryness. The crude product was purified by flash chromatography. The purified product was dissolved in DCM, treated with 1 M HCl in Et₂O (296 µl), and evaporated *in vacuo.* The residue was dissolved in DCM and the salt was precipitated by addition of hexane and diethyl ether. The precipitate was filtered off, washed with hexane and dried *in vacuo.* The product was obtained as a white solid.

### Synthesis of Example 119:

### Example 119:

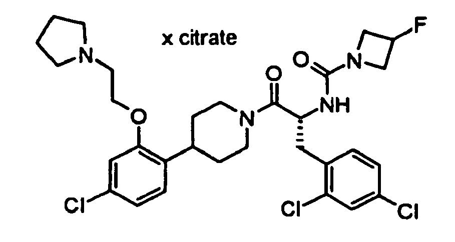

Intermediate 20b) (31 mg), B-C Moiety 3 (37 mg), and HOBt (19 mg) were dissolved in DCM (2.5 ml). NMM (26 µl) was added and the mixture stirred at room temperature for 30 min. EDCl (29 mg) was added, and the reaction stirred at room temperature for another 60 min. An additional amount of NMM (7 µl) was added and stirring continued at room temperature overnight. The reaction mixture was evaporated *in vacuo,* diluted with EtOAc, washed with sat. Na₂CO₃, water and brine. The aqueous layers were extracted with EtOAc. The combined organic layer was dried over Na₂SO₄, filtered and evaporated in *vacuo* to dryness. The residue was purified by flash chromatography. The purified product was dissolved in EtOAc (400 µl), treated with 0.1 M citric acid in EtOH (721 µl), and hexane (8.0 ml). The precipitate was filtered off, washed with hexane (1.0 ml) and dried *in vacuo* over P₂O₅ at room temperature overnight.

### Synthesis of Example 121:

### Intermediate 121a):

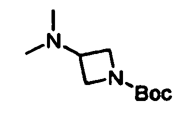

3-Amino-1 N-Boc-azetidine (500 mg) and formaldehyde solution (≥ 36.5% in H₂O, 876 µl) were dissolved in of 1,2-dichloroethane (1 ml) and sodium triacetoxyborohydride (5.46 g) was added at room temperature in one portion and the reaction mixture was cooled down to room temperature with a ice/water bath. The mixture was stirred at room temperature for 90 min. Then the reaction mixture was quenched by adding aqueous sat. NaHCO₃ and the product was extracted with EtOAc. The organic layer was washed with H₂O and brine. The aqueous layers were extracted with EtOAc. The combined organic layer was dried over Na₂SO₄ and evaporated *in vacuo* to dryness.
The crude product was dissolved in CH₂Cl₂ (7 ml), Fmoc-OSu (911 mg) was added and the reaction mixture was stirred at room temperature for 2 h. The reaction mixture was concentrated *in vacuo,* redissolved in EtOAc and washed with HCl 0.1 N, water and brine, The combined aqueous layers were basified with sat. Na₂CO₃ and extracted twice with EtOAc. The organic layers were washed with water and brine, then combined and evaporated *in vacuo* to dryness. The residue was purified by flash chromatography.

### Intermediate 121b):

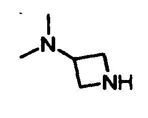

Intermediate 121a) (280 mg) was dissolved in CH₂Cl₂ (5 ml), and TFA (1.3 ml) was added at room temperature. The reaction mixture was stirred at 0°C for 2 h. The reaction mixture was basified by adding aqueous sat. Na₂CO₃ and the mixture was extracted with three times with CH₂Cl₂. The aqueous layer was saturated with NaCl and extracted twice with THF. The combined THF-layer was dried over Na₂SO₄ and concentrated *in vacuo.* The product was obtained as solution in water and THF (~2 ml).

### Intermediate 121c):

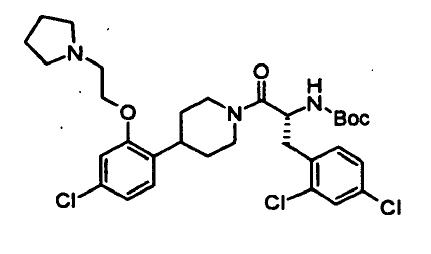

To Boc-D-2,4-dichlorophenylalanine (1336 mg) in DCM (20 ml) was added the amine hydrochloride from 20b) (1145 mg), N-methylmorpholine (935 µl), HOBt (689 mg) and the mixture was stirred for 30 min. EDCl (1052 mg) was added and stirring was continued for 1 h. An additional amount of N-methylmorpholine (275 µl) was added and stirred overnight. The reaction mixture was diluted with EtOAc (180 ml), washed with sat. Na₂CO₃ (3x30 ml), water (2x30 ml) and brine (25 ml). The organic layer was dried over Na₂SO₄, filtered and evaporated *in vacuo* to dryness. Purification by flash chromatography yielded the title compound as a white foam.

### Intermediate 121d):

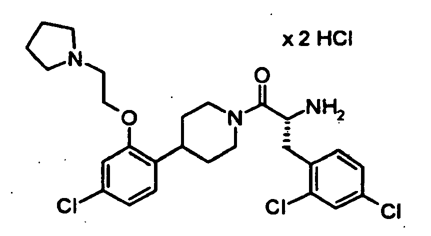

To Boc-protected intermediate 121c) (1756 mg) in dioxane (5 ml) was added hydrogen chloride, 4.0 M sol. in 1,4-dioxane (30 ml) and the solution was stirred at room temperature for 2 h. The reaction mixture was evaporated *in vacuo* to dryness. The residue was triturated in acetone (30 ml), filtered off and washed with acetone (3 ml) and Et₂O (2x5 ml) to yield a white solid which was dried *in vacuo* over P₂O₅ at room temperature overnight.

### Intermediate 121e):

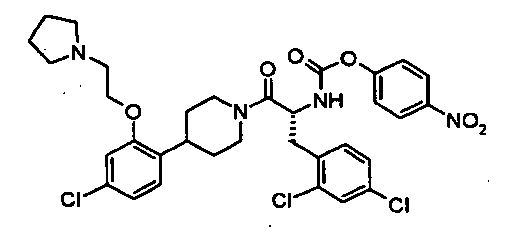

Intermediate 121d) (149 mg) was suspended in DCM (7.5 ml) and NMM (96 µl) was added. The mixture was cooled in an ice bath with stirring. Then 4-nitrophenyl chloroformate (76 mg) was added, and the reaction mixture was stirred at 0°C for 60 min. The ice bath was then removed and stirring was continued at room temperature overnight. The reaction mixture was diluted with EtOAc and washed with sat. Na₂CO₃ (3x25 ml), water (20 ml), and brine (15 ml). The organic layer was dried over Na₂SO₄ and evaporated *in vacuo.*

### Example 121:

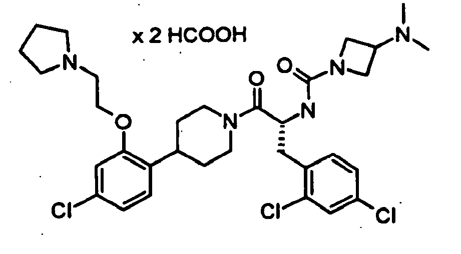

To a solution of intermediate 121e) (50 mg) in THF (4 ml) was added intermediate 121b) (~104 mg in 2 ml water/THF) and the reaction mixture was stirred at room temperature overnight. The reaction mixture was evaporated *in vacuo.* The residue was redissolved in EtOAc and the organic layer was washed with sat. Na₂CO₃. water and brine. The aqueous layers were extracted with EtOAc. The combined organic layer was dried over Na₂SO₄ and evaporated *in vacuo* to dryness. The crude product was purified by flash chromatography following purification with preparative HPLC MS.

Further examples are exemplified below.

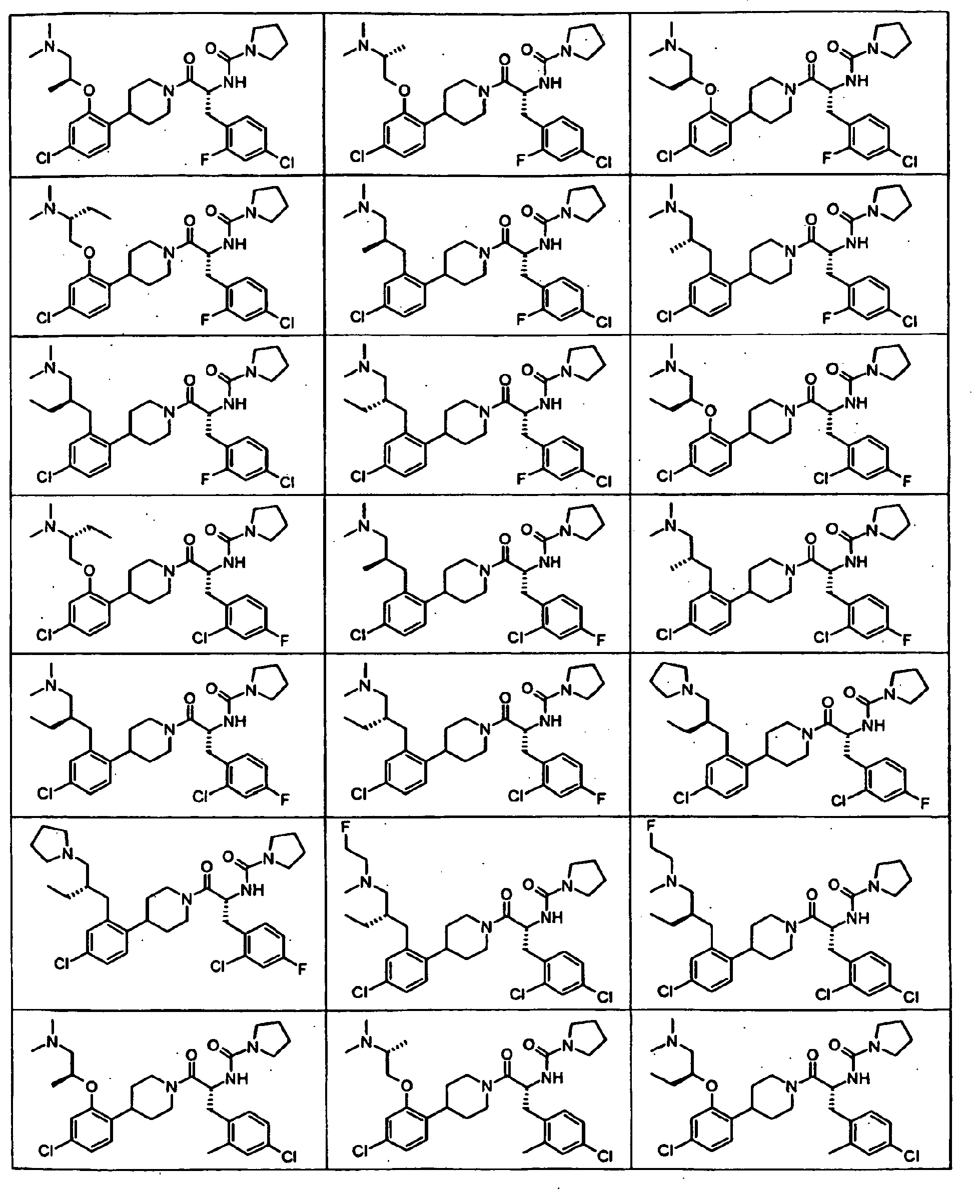

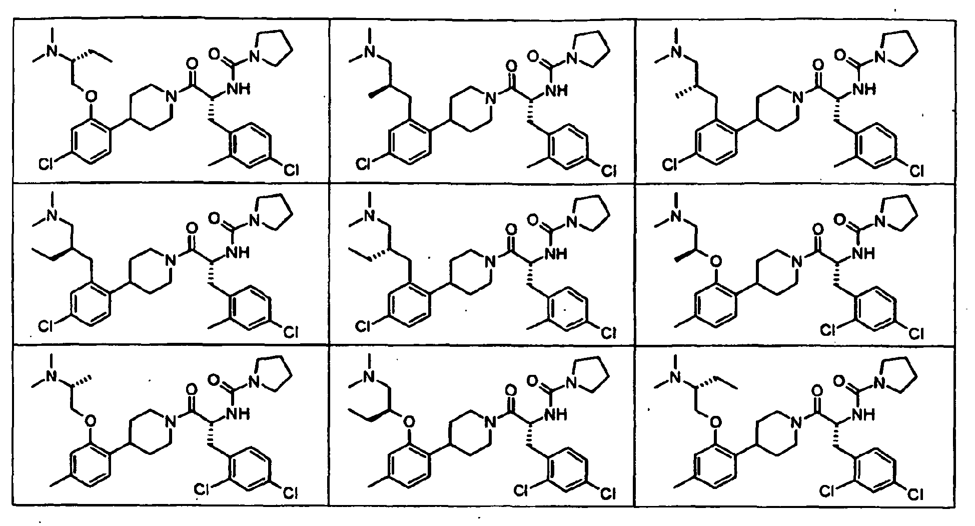

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM.

**Table 16: Biological data for selected examples of the invention**

| Example | hMC-4R binding assay IC₅₀/nM | hMC-4R functional assay EC₅₀/nM | % activation functional assay |
|---|---|---|---|
| SHU-9119 | 1.9 | | 7 |
| NDP-α-MSH | 1.1 | 3.4 | 100 |
| 2 | 3.7 | | 0 |
| 36 | 9.6 | | 0 |
| 95 | 5.3 | | 0 |
| 110 | 4.4 | | 0 |
| 113 | 3.0 | | 0 |

### C. In Vivo Food Intake Models

### 1. Spontaneous Feeding Paradigm

Food intake in rats is measured after i.p. or p.o. administration of the test compound (see e.g. A.S. Chen et al. Transgenic Res 2000 Apr; 9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61(4):1432-1438).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in K.E. McKenna et al, A Model For The Study of Sexual Function In Anesthetized Male And Female Rats, Am. J. Physiol. (Regulatory Integrative Comp. Physiol 30): R1276-R1285, 1991; K.E. McKenna et al, Modulation By Peripheral Serotonin of The Threshold For Sexual Reflexes In Female Rats, Pharm. Bioch. Behav., 40:151-156, 1991; and L.K. Takahashi et al, Dual Estradiol Action In The Diencephalon And The Regulation of Sociosexual Behavior In Female Golden Hamsters, Brain Res., 359: 194-207, 1985.

### Examples of a Pharmaceutical Composition

As a specific embodiment of an oral composition of a compound of the present invention, 30 mg of Example 2 is formulated with sufficient finely divided lactose to provide a total amount of 580 to 590 mg to fill a size 0 hard gelatin capsule.

As another specific embodiment of an oral composition of a compound of the present invention, 25 mg of Example 20 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 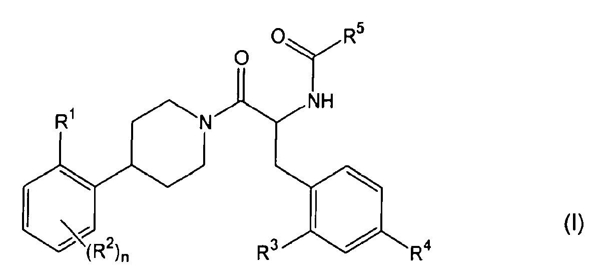 and the enantiomers, diastereomers, tautomers, solvates and pharmaceutically acceptable salts thereof,
wherein
R¹ is -(C(R⁶)₂)ₗ-T, or
-O-(C(R⁶)₂)ₘ-T;
R⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸,
morpholine, 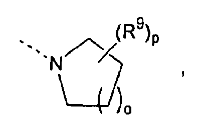 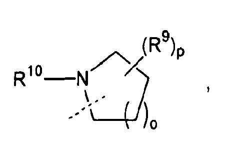 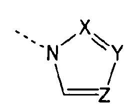 or 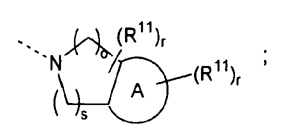
R⁷ and R⁸ are independently from each other selected from
H,
C₁₋₆-alkyl,
C₂₋₆-alkenyl
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH, and
C₁₋₆-alkylene-O-C₁₋₆-alkyl optionally substituted with 1 to 3 substituents selected from halogen, CN and OH;
R¹⁰ is H, or
C₁-C₆-alkyl;
R¹¹ is independently selected from
halogen,
CN, OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X is CH or N;
Y is CH or N;
Z is CH or N;
A is a 3-7-membered saturated, unsaturated or aromatic ring containing 0-2 nitrogen atoms;
R² is independently selected from
F,
Cl,
CH₃, and
CF₃;
R³ is H,
Cl,
F, or
CH₃;
R⁴ is Cl or F;
R⁵ is 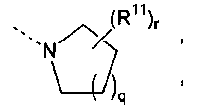 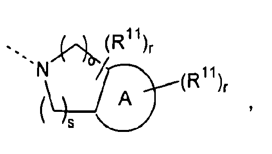 morpholine, optionally substituted by 1 to 3, same or different substituents R¹⁴, or NR¹²R¹³;
R¹² and R¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
l is 1, 2, 3, or 4;
m is 0, 1, 2, 3, or 4;
n is 0, 1, 2, 3, or 4;
o is 0, 1, or 2;
p is 0, 1, 2, 3, or 4;
q is 0, 1, 2, or 3;
r is 0, 1, 2, 3, or 4 and
s is 1, or 2.

2. The compound of claim 1 according to formula (I') 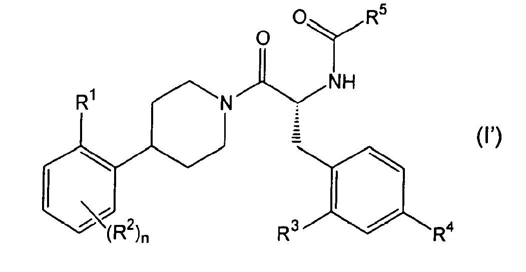 wherein R¹, R², R³, R⁴, R⁵ and n are as defined in claim 1.

3. The compound of claim 1 or 2, wherein
R¹ is -(CH₂)ₗ-T,
-O-(CH₂)ₘ-T;
T is NR⁷R⁸,
morpholine, 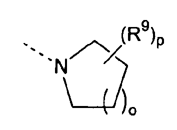 or 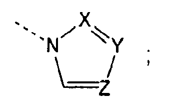
R⁷ and R⁸ are independently from each other selected from
C₁₋₆-alkyl,
C₂₋₆-alkenyl
C₂₋₆-alkinyl, and
C₂₋₆-alkylene-O-C₁₋₆-alkyl;
R⁹ is independently selected from
halogen,
CN,
OH,
C₁₋₆-alkyl optionally substituted with 1 to 3 substituents selected from halogen,
CN and OH, and
O-C₁₋₆-alkyl optionally substituted with 1 to 3 substituents selected from halogen,
CN and OH;
X is CH or N;
Y is CH or N;
Z is CH or N;
R² is independently selected from
Cl,
CH₃, and
CF₃;
R³ is H,
Cl, or
CH₃;
R⁴ is Cl;
R⁵ is 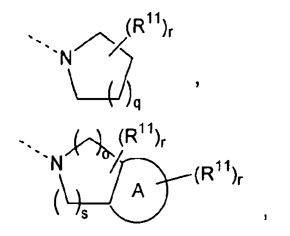 morpholine, optionally substituted by 1 to 3, same or different substituents R¹⁴ or NR¹²R¹³;
R¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
R¹² and R¹³ are independently from each other selected from
C₁₋₆-alkyl,
C₂₋₆-alkenyl,
C₂₋₆-alkinyl,
C₂₋₆-alkylene-O-C₁₋₆-alkyl;
R¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)2;
A is a 3-7-membered saturated, unsaturated or aromatic ring containing 0-2 nitrogen atoms;
l is 1, 2, 3, or 4;
m is 2, 3, or 4,
n is 0, 1, 2, 3, or 4;
o is 0, 1, or 2;
p is 0, 1, 2, 3, or 4;
q is 0, 1, 2, or 3;
r is 0, 1, 2, 3, or 4; and
s is 1, or 2.

4. The compound of any of claims 1 to 3, wherein at least one of R⁷ and R⁸ is selected from
C₂₋₆-alkenyl,
C₂₋₆-alkinyl, and
C₂₋₆-alkylene-O-C₁₋₆-alkyl.

5. The compound of any of claims 1 to 4, wherein
R² is F or Cl, and
R³ is Cl.

6. The compound of any of claims 1 to 5 wherein
l is 2 or 3, and
m is 2 or 3.

7. The compound of any of claims 1 to 6 as medicament.

8. Use of the compound of any of claims 1 to 6 for the preparation of a medicament for the treatment or prophylaxis of disorders, diseases or conditions responsive to the inactivation or activation of the melanocortin-4 receptor in a mammal.

9. Use according to claim 8 for the preparation of a medicament for the treatment or prophylaxis of cancer cachexia.

10. Use according to claim 8 for the preparation of a medicament for the treatment or prophylaxis of muscle wasting.

11. Use according to claim 8 for the preparation of a medicament for the treatment or prophylaxis of anorexia.

12. Use according to claim 8 for the preparation of a medicament for the treatment or prophylaxis of anxiety and/or depression.

13. Use according to claim 8 for the preparation of a medicament for the treatment or prophylaxis of obesity.

14. Use according to claim 8 for the preparation of a medicament for the treatment or prophylaxis of diabetes mellitus.

15. Use according to claim 8 for the preparation of a medicament for the treatment or prophylaxis of male or female sexual dysfunction.

16. Use according to claim 8 for the preparation of a medicament for the treatment or prophylaxis of erectile dysfunction.

17. A pharmaceutical composition comprising a compound of any of claims 1 to 6 and a pharmaceutically acceptable carrier.

18. The compound of any of claims 1 to 6 for use in the treatment or prophylaxis of cancer cachexia, muscle wasting, anorexia, anxiety and/or depression, obesity, diabetes mellitus, male or female sexual dysfunction and/or erectile dysfunction.

## Patentansprüche

1. Verbindung gemäß Formel I 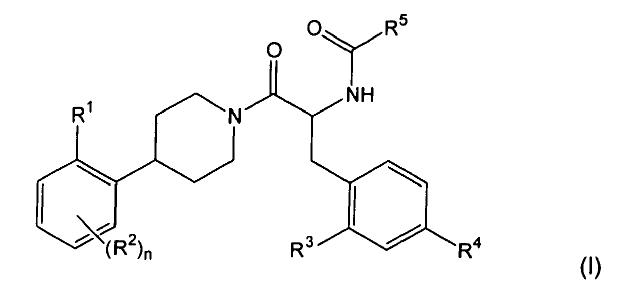 und die Enantiomeren, Diastereomeren, Tautomeren, Solvate und pharmazeutisch akzeptablen Salze davon,
worin
R¹ ist -(C(R⁶)₂)ₗ-T, oder
-O-(C(R⁶)₂)ₘ-T;
R⁶ ist unabhängig ausgewählt aus
H,
F,
OH,
OCH₃,
C₁₋₆-Alkyl, optional substituiert mit 1 bis 3 Substituenten ausgewählt aus Halogen, CN, OH und OCH₃, und
C₃₋₆-Cycloalkyl, optional substituiert mit 1 bis 3 Substituenten ausgewählt aus Ha- logen, CN, OH und OCH₃;
T ist NR⁷R⁸,
Morpholin, 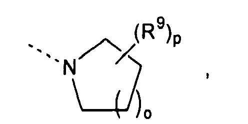 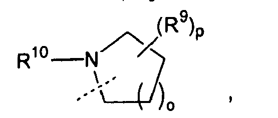 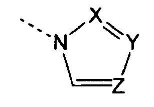 oder 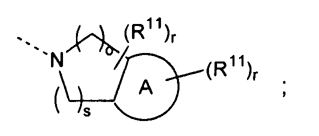
R⁷ und R⁸ sind unabhängig von einander ausgewählt aus
H,
C₁₋₆-Alkyl,
C₂₋₆-Alkenyl
C₂₋₆-Alkinyl, und
C₂₋₆-Alkylen-O-C₁₋₆-Alkyl,
worin jedes Alkyl, Alkenyl und Alkinyl optional mit einem oder mehreren Halo- genatomen, CN oder OH substituiert ist;
R⁹ ist unabhängig ausgewählt aus
Halogen,
CN,
OH,
C₁₋₆-Alkyl, optional substituiert mit 1 bis 3 Substituenten ausgewählt aus Halo- gen, CN and OH,
O-C₁₋₆-Alkyl, optional substituiert mit 1 bis 3 Substituenten ausgewählt aus Halo- gen, CN und OH, und
C₁₋₆-Alkylen-O-C₁₋₆-Alkyl, optional substituiert mit 1 bis 3 Substituenten ausge- wählt aus Halogen, CN und OH;
R¹⁰ ist H oder
C₁-C₆-Alkyl;
R¹¹ ist unabhängig ausgewählt aus
Halogen,
CN,
OH,
C₁₋₆-Alkyl, optional substituiert mit 1 bis 3 Substituenten ausgewählt aus Halo- gen, CN und OH,
O-C₁₋₆-Alkyl, optional substituiert mit 1 bis 3 Substituenten ausgewählt aus Halo- gen, CN und OH,
C₁₋₆-Alkylen-O-C₁₋₆-Alkyl, optional substituiert mit 1 bis 3 Substituenten ausge- wählt aus Halogen, CN und OH,
-NH₂,
-NH(C₁₋₆-Alkyl), und
-N(C₁₋₆-Alkyl)₂;
X ist CH oder N;
Y ist CH oder N;
Z ist CH oder N;
A ist ein 3-7-gliedriger gesättigter, ungesättigter oder aromatischer Ring enthaltend 0-2 Stickstoffatome;
R² ist unabhängig ausgewählt aus
F,
Cl,
CH₃, und
CF₃;
R³ ist H,
Cl,
F oder
CH₃;
R⁴ ist Cl oder F;
R⁵ ist 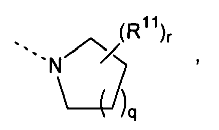 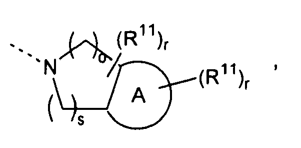 Morpholin, optional substituiert mit 1 bis 3, gleichen oder verschiedenen Substi- tuenten R¹⁴, oder
NR¹²R¹³;
R¹² und R¹³ sind unabhängig von einander ausgewählt aus
C₁₋₆-Alkyl,
C₂₋₆-Alkenyl,
C₂₋₆-Alkinyl,
C₂₋₆-Alkylen-O-C₁₋₆-Alkyl, und
C₂₋₆-Alkylen-N-(C₁₋₆-Alkyl)₂;
R¹⁴ ist C₁₋₆-Alkyl,
C₁₋₆-Alkylen-O-C₁₋₆-Alkyl,
C₁₋₆-Alkylen-OH,
C₁₋₆-Alkylen-NH₂,
C₁₋₆-Alkylen-NH-C₁₋₆-Alkyl, oder
C₁₋₆-Alkylen-N(C₁₋₆-alkyl)₂;
I ist 1, 2, 3 oder 4;
m ist 0, 1, 2, 3 oder 4;
n ist 0, 1, 2, 3 oder 4;
o ist 0, 1 oder 2;
p ist 0, 1, 2, 3 oder 4;
q ist 0, 1, 2 oder 3;
r ist 0, 1, 2, 3 oder 4 und
s ist 1 oder 2.

2. Die Verbindung gemäß Anspruch 1 gemäß der Formel (I') 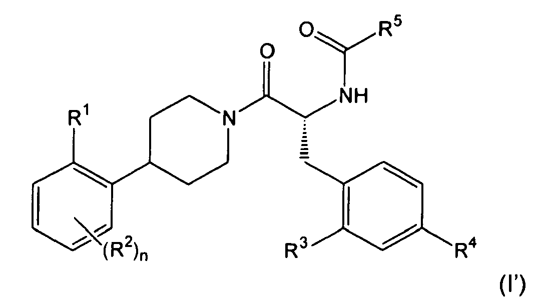 worin R¹, R², R³, R⁴, R⁵ und n wie in Anspruch 1 definiert sind.

3. Die Verbindung gemäß Anspruch 1 oder 2, worin
R¹ ist -(CH₂)ₗ-T,
-O-(CH₂)ₘ-T;
T ist NR⁷R⁸,
Morpholin, 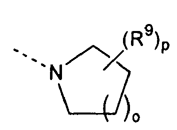 oder 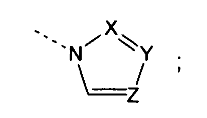
R⁷ und R⁸ sind unabhängig von einander ausgewählt aus
C₁₋₆-Alkyl,
C₂₋₆-Alkenyl
C₂₋₆-Alkinyl, und
C₂₋₆-Alkylen-O-C₁₋₆-Alkyl;
R⁹ ist unabhängig ausgewählt aus
Halogen,
CN,
OH,
C₁₋₆-Alkyl, optional substituiert mit 1 bis 3 Substituenten ausgewählt aus Halo- gen, CN and OH, und
O-C₁₋₆-Alkyl, optional substituiert mit 1 bis 3 Substituenten ausgewählt aus Halo- gen, CN und OH;
X ist CH oder N;
Y ist CH oder N;
Z ist CH oder N;
R² ist unabhängig ausgewählt aus
F,
Cl,
CH₃, und
CF₃;
R³ ist H,
Cl, oder
CH₃;
R⁴ ist Cl;
R⁵ ist 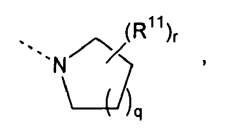 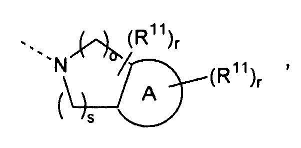 Morpholin, optional substituiert mit 1 bis 3, gleichen oder verschiedenen Substi- tuenten R¹⁴, oder
NR¹²R¹³;
R¹¹ ist unabhängig ausgewählt aus
Halogen,
CN,
OH,
C₁₋₆-Alkyl, optional substituiert mit 1 bis 3 Substituenten ausgewählt aus Halo- gen, CN und OH,
O-C₁₋₆-Alkyl, optional substituiert mit 1 bis 3 Substituenten ausgewählt aus Halo- gen, CN und OH,
C₁₋₆-Alkylen-O-C₁₋₆-Alkyl, optional substituiert mit 1 bis 3 Substituenten ausge- wählt aus Halogen, CN und OH,
-NH₂,
-NH(C₁₋₆-Alkyl), und
-N(C₁₋₆-Alkyl)₂;
R¹² und R¹³ sind unabhängig voneinander ausgewählt aus
C₁₋₆-Alkyl,
C₂₋₆-Alkenyl,
C₂₋₆-Alkinyl,
C₂₋₆-Alkylen-O-C₁₋₆-alkyl;
R¹⁴ ist C₁₋₆-Alkyl,
C₁₋₆-Alkylen-O-C₁₋₆-alkyl,
C₁₋₆-Alkylen-OH,
C₁₋₆-alkylene-NH₂,
C₁₋₆-Alkylen-NH-C₁-₆-Alkyl, oder
C₁₋₆-Alkylen-N(C₁₋₆-Alkyl)₂;
A ist ein 3-7-gliedriger gesättigter, ungesättigter oder aromatischer Ring enthaltend 0-2 Stickstoffatome;
I ist 1, 2, 3 oder 4;
m ist 2, 3 oder 4,
n ist 0, 1, 2, 3 oder 4;
o ist 0, 1 oder 2;
p ist 0, 1, 2, 3 oder 4;
q ist 0, 1, 2 oder 3;
r ist 0, 1, 2, 3 oder 4; und
s ist 1 oder 2.

4. Die Verbindung gemäß einem der Ansprüche 1 bis 3, worin mindestens eines von R⁷ und R⁸ ausgewählt ist aus
C₂₋₆-Alkenyl,
C₂₋₆-Alkinyl und
C₂₋₆-Alkylen-O-C₁₋₆-Alkyl.

5. Die Verbindung gemäß einem der Ansprüche 1 bis 4, worin
R² ist F oder Cl, und
R³ ist Cl.

6. Die Verbindung gemäß einem der Ansprüche 1 bis 5, worin
l 2 oder 3 ist, und
m 2 oder 3 ist.

7. Die Verbindung gemäß einem der Ansprüche 1 bis 6 als Arzneimittel.

8. Verwendung der Verbindung gemäß einem der Ansprüche 1 bis 6 für die Herstellung eines Arzneimittels für die Behandlung oder Prophylaxe von Störungen, Krankheiten oder Zustände, welche auf die Inaktivierung oder Aktivierung des Melanocortin-4-Rezeptors in einem Säuger ansprechen.

9. Verwendung gemäß Anspruch 8, für die Herstellung eines Arzneimittels für die Behandlung oder Prophylaxe von Krebskachexie.

10. Verwendung gemäß Anspruch 8, für die Herstellung eines Arzneimittels für die Behandlung oder Prophylaxe von Muskelschwund.

11. Verwendung gemäß Anspruch 8 für die Herstellung eines Arzneimittels für die Behandlung oder Prophylaxe von Anorexie.

12. Verwendung gemäß Anspruch 8 für die Herstellung eines Arzneimittels für die Behandlung oder Prophylaxe von Angstzuständen und/oder Depression.

13. Verwendung gemäß Anspruch 8 für die Herstellung eines Arzneimittels für die Behandlung oder Prophylaxe von Fettleibigkeit.

14. Verwendung gemäß Anspruch 8 für die Herstellung eines Arzneimittels für die Behandlung oder Prophylaxe von Diabetes mellitus.

15. Verwendung gemäß Anspruch 8 für die Herstellung eines Arzneimittels für die Behandlung oder Prophylaxe von männlichen oder weiblichen sexuellen Dysfunktionen.

16. Verwendung gemäß Anspruch 8 für die Herstellung eines Arzneimittels für die Behandlung oder Prophylaxe von Erektionsstörungen.

17. Pharmazeutische Zusammensetzung, umfassend eine Verbindung gemäß einem der Ansprüche 1 bis 6 und einen pharmazeutisch akzeptablen Träger.

18. Die Verbindung gemäß einem der Ansprüche 1 bis 6 zur Verwendung in der Behandlung oder Prophylaxe von Krebskachexie, Muskelschwund, Anorexie, Angstzuständen und/oder Depression, Fettleibigkeit, Diabetes mellitus, männlichen oder weiblichen sexuellen Dysfunktionen und/oder Erektionsstörungen.

## Revendications

1. Composé répondant à la formule (I) 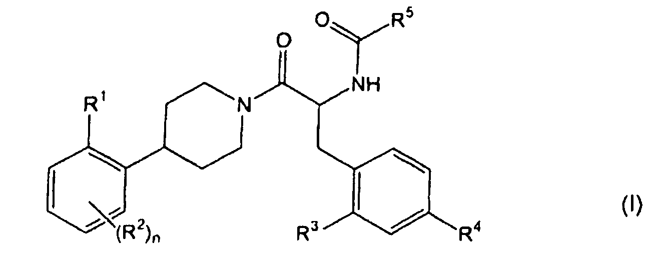 et les énantiomères, diastéréoisomères, tautomères, solvates, et sels pharmaceutiquement acceptables de celui-ci,
où
R¹ représente
-(C(R⁶)₂)ₗ-T, ou
-O-(C(R⁶)₂)ₘ-T;
R⁶ est indépendamment choisi parmi
H,
F,
OH,
OCH₃,
un groupe alkyle en C₁₋₆, éventuellement substitué par 1 à 3 substituants choisis parmi un halogène, CN, OH et OCH₃, et
un groupe cycloalkyle en C₃₋₆, éventuellement substitué par 1 à 3 substituants choisis parmi un halogène, CN, OH et OCH₃ ;
T représente
NR⁷R⁸,
une fraction morpholine, 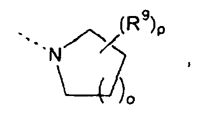 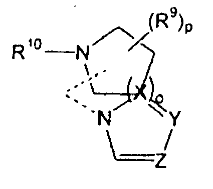 ou 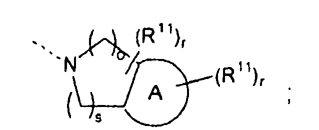
R⁷ et R⁸ sont indépendamment choisis parmi
H,
un groupe alkyle en C₁₋₆,
un groupe alcényle en C₂₋₆,
un groupe alcynyle en C₂₋₆, et
un groupe (alkylène en C₂₋₆)-O-(alkyle en C₁₋₆),
où chaque groupe alkyle, alcényle et alcynyle est éventuellement substitué par un ou plusieurs atomes d'halogène, CN ou OH ;
R⁹ est indépendamment choisi parmi
un halogène,
CN,
OH,
un groupe alkyle en C₁₋₆ éventuellement substitué par 1 à 3 substituants choisis parmi un halogène, CN et OH, et
un groupe O-alkyle en C₁₋₆ éventuellement substitué par 1 à 3 substituants choisis parmi un halogène, CN et OH,
un groupe (alkylène en C₁₋₆)-O-(alkyle en C₁₋₆) éventuellement substitué par 1 à 3 substituants choisis parmi un halogène, CN et OH ;
R¹⁰ représente
H, ou
un groupe alkyle en C₁₋₆ ;
R¹¹ est indépendamment choisi parmi
un halogène,
CN,
OH,
un groupe alkyle en C₁₋₆ éventuellement substitué par 1 à 3 substituants choisis parmi un halogène, CN et OH,
un groupe O-alkyle en C₁₋₆ éventuellement substitué par 1 à 3 substituants choisis parmi un halogène, CN et OH,
un groupe (alkylène en C₁₋₆)-O-(alkyle en C₁₋₆) éventuellement substitué par 1 à 3 substituants choisis parmi un halogène, CN et OH,
-NH₂,
un groupe -NH(alkyle en C₁₋₆), et un groupe -N(alkyle en C₁₋₆)₂ ;
X représente
CH ou N;
Y représente
CH ou N ;
Z représente
CH ou N ;
A représente
un cycle de 3 à 7 chaînons saturé, insaturé ou aromatique, contenant 0-2 atomes d'azote ;
R² est indépendamment choisi parmi
F,
Cl,
CH₃, et
CF₃ ;
R³ représente
H,
Cl,
F, ou
CH₃ ;
R⁴ représente
Cl ou F;
R⁵ représente 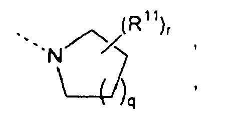 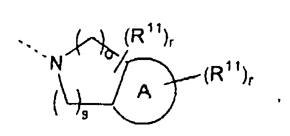 une fraction morpholine, éventuellement substituée par 1 à 3 substituants R¹⁴ identiques ou différents, ou
NR¹²R¹³;
R¹² et R¹³ sont indépendamment choisis parmi
un groupe alkyle en C₁₋₆,
un groupe alcényle en C₂₋₆,
un groupe alcynyle en C₂₋₆,
un groupe (alkylène en C₂₋₆)-O-(alkyle en C₁₋₆), et un groupe (alkylène en C₂₋₆)-N-(alkyle en C₁₋₆)₂ ;
R¹⁴ représente
un groupe alkyle en C₁₋₆,
un groupe (alkylène en C₁₋₆)-O-(alkyle en C₁₋₆), un groupe (alkylène en C₁₋₆)-OH,
un groupe (alkylène en C₁₋₆)-NH₂,
un groupe (alkylène en C₁₋₆)-NH-(alkyle en C₁₋₆), ou un groupe (alkylène en C₁₋₆)-N(alkyle en C₁₋₆)₂ ;
I vaut 1, 2, 3, ou 4 ;
m vaut 0, 1, 2, 3, ou 4 ;
n vaut 0, 1, 2, 3, ou 4 ;
o vaut 0, 1, ou 2 ;
p vaut 0, 1, 2, 3, ou 4 ;
q vaut 0, 1, 2, ou 3 ;
r vaut 0, 1, 2, 3, ou 4 et
s vaut 1, ou 2.

2. Composé selon la revendication 1 répondant à la formule (I') 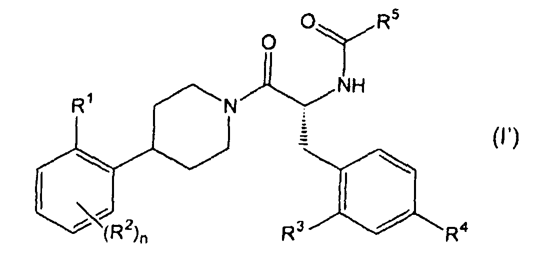 dans laquelle R¹, R², R³, R⁴, R⁵ et n sont tels que définis dans la revendication 1.

3. Composé selon la revendication 1 ou 2, dans lequel
R¹ représente
-(CH₂)ₗ-T,
-O-(CH₂)ₘ-T ;
T représente
NR⁷R⁸,
une fraction morpholine, 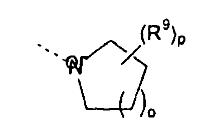 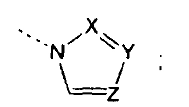
R⁷ et R⁸ sont indépendamment choisis parmi
un groupe alkyle en C₁₋₆,
un groupe alcényle en C₂₋₆,
un groupe alcynyle en C₂₋₆, et
un groupe (alkylène en C₂₋₆)-O-(alkyle en C₁₋₆) ;
R⁹ est indépendamment choisi parmi
un halogène,
CN,
OH,
un groupe alkyle en C₁₋₆ éventuellement substitué par 1 à 3 substituants choisis parmi un halogène, CN et OH, et
un groupe O-alkyle en C₁₋₆ éventuellement substitué par 1 à 3 substituants choisis parmi un halogène, CN et OH ;
X représente
CH ou N ;
Y représente
CH ou N ;
Z représente
CH ou N ;
R² est indépendamment choisi parmi
F,
Cl,
CH₃, et
CF₃ ;
R³ représente
H,
Cl, ou
CH₃ ;
R⁴ représente
CI ;
R⁵ représente 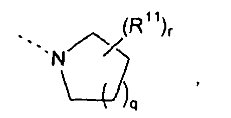 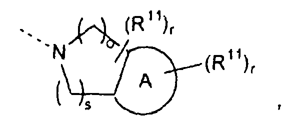 une fraction morpholine, éventuellement substituée par 1 à 3 substituants R¹⁴ identiques ou différents, ou
NR¹²R¹³;
R¹¹ est indépendamment choisi parmi
un halogène,
CN,
OH,
un groupe alkyle en C₁₋₆ éventuellement substitué par 1 à 3 substituants choisis parmi un halogène, CN et OH,
un groupe O-alkyle en C₁₋₆ éventuellement substitué par 1 à 3 substituants choisis parmi un halogène, CN et OH,
un groupe (alkylène en C₁₋₆)-O-(alkyle en C₁₋₆) éventuellement substitué par 1 à 3 substituants choisis parmi un halogène, CN et OH,
-NH₂,
un groupe -NH(alkyle en C₁₋₆), et
un groupe -N(alkyle en C₁₋₆)₂ ;
R¹² et R¹³ sont indépendamment choisis parmi
un groupe alkyle en C₁₋₆,
un groupe alcényle en C₂₋₆,
un groupe alcynyle en C₂₋₆,
un groupe (alkylène en C₂₋₆)-O-(alkyle en C₁₋₆) ;
R¹⁴ représente
un groupe alkyle en C₁₋₆,
un groupe (alkylène en C₁₋₆)-O-(alkyle en C₁₋₆), un groupe (alkylène en C₁₋₆)-OH,
un groupe (alkylène en C₁₋₆)-NH₂,
un groupe (alkylène en C₁₋₆)-NH-(alkyle en C₁₋₆), ou
un groupe (alkylène en C₁₋₆)-N(alkyle en C₁₋₆)₂;
A représente
un cycle de 3 à 7 chaînons saturé, insaturé ou aromatique, contenant 0-2 atomes d'azote ;
I vaut 1, 2, 3, ou 4 ;
m vaut 2, 3, ou 4 ;
n vaut 0, 1, 2, 3, ou 4 ;
o vaut 0, 1, ou 2 ;
p vaut 0, 1, 2, 3, ou 4 ;
q vaut 0, 1, 2, ou 3 ;
r vaut 0, 1, 2, 3, ou 4 ; et
s vaut 1, ou 2 ;

4. Composé selon l'une quelconque des revendications 1 à 3, dans lequel au moins un des R⁷ et R⁸ est choisi parmi
un groupe alcényle en C₂₋₆,
un groupe alcynyle en C₂₋₆, et
un groupe (alkylène en C₂₋₆)-O-(alkyle en C₁₋₆).

5. Composé selon l'une quelconque des revendications 1 à 4, dans lequel
R² représente
F ou Cl, et
R³ représente
Cl.

6. Composé selon l'une quelconque des revendications 1 à 5 dans lequel
I vaut 2 ou 3, et
m vaut 2 ou 3.

7. Composé selon l'une quelconque des revendications 1 à 6 en tant que médicament.

8. Utilisation du composé selon l'une quelconque des revendications 1 à 6 pour la préparation d'un médicament destiné au traitement ou à la prophylaxie de troubles, maladies ou affections sensibles à l'inactivation ou à l'activation du récepteur de la mélanocortine de type 4 chez un mammifère.

9. Utilisation selon la revendication 8 pour la préparation d'un médicament destiné au traitement ou à la prophylaxie de la cachexie d'origine cancéreuse.

10. Utilisation selon la revendication 8 pour la préparation d'un médicament destiné au traitement ou à la prophylaxie de la fonte musculaire.

11. Utilisation selon la revendication 8 pour la préparation d'un médicament destiné au traitement ou à la prophylaxie de l'anorexie.

12. Utilisation selon la revendication 8 pour la préparation d'un médicament destiné au traitement ou à la prophylaxie de l'anxiété et/ou de la dépression.

13. Utilisation selon la revendication 8 pour la préparation d'un médicament destiné au traitement ou à la prophylaxie de l'obésité.

14. Utilisation selon la revendication 8 pour la préparation d'un médicament destiné au traitement ou à la prophylaxie du diabète sucré.

15. Utilisation selon la revendication 8 pour la préparation d'un médicament destiné au traitement ou à la prophylaxie de la dysfonction sexuelle chez le mâle ou la femelle.

16. Utilisation selon la revendication 8 pour la préparation d'un médicament destiné au traitement ou à la prophylaxie de la dysfonction érectile.

17. Composition pharmaceutique comprenant un composé selon l'une quelconque des revendications 1 à 6 et un véhicule pharmaceutiquement acceptable.

18. Composé selon l'une quelconque des revendications 1 à 6 destiné à être utilisé dans le traitement ou la prophylaxie de la cachexie d'origine cancéreuse, de la fonte musculaire, de l'anorexie, de l'anxiété et/ou de la dépression, de l'obésité, du diabète sucré, de la dysfonction sexuelle chez le mâle ou la femelle et/ou de la dysfonction érectile.
